# EUROPEAN PATENT SPECIFICATION

(11) **EP 4 460 519 B1**
(45) Date of publication and mention of the grant of the patent: **11.03.2026**
(21) Application number: 23700455.1
(22) Date of filing: 05.01.2023
(51) Int. Cl.: C07K 16/28, C07K 14/725, A61K 39/00

(54) **ANTI-CD84 ANTIBODIES AND CHIMERIC ANTIGEN RECEPTORS**
ANTI-CD84-ANTIKÖRPER AMD CHIMÄRE ANTIGENREZEPTOREN
ANTICORPS ANTI-CD84 ET RÉCEPTEURS D'ANTIGÈNE CHIMÉRIQUE

(30) Priority: 05.01.2022 EP 22382003; 03.11.2022 EP 22205399
(43) Date of publication of application: 13.11.2024
(60) Divisional application: 26155524.7
(73) Proprietor: Gyala Therapeutics Sociedad Limitada, 08007 Barcelona (ES); Fundació de Recerca Clínic Barcelona-Institut d'Investigacions Biomèdiques August Pi I Sunyer, 08036 Barcelona (ES); Hospital Clínic De Barcelona (HCB), 08036 Barcelona (ES)
(72) Inventor: KLEIN GONZÁLEZ, Nela, 08017 Barcelona (ES); JUAN OTERO, Manuel, 08940 Cornellà del Llobregat (ES); VILELLA PUIG, Ramon, 08004 Barcelona (ES); PÉREZ AMILL, Lorena, 08238 Sant Quirze del Vallès (ES); RIBEIRO DOS SANTOS, Claudio Joao, 37001 Salamanca (ES)
(74) Representative: D Young & Co LLP
(86) International application number: PCT/EP2023/050194
(87) International publication number: WO 2023/131657

(56) References cited:
- WO-A1-2015/118538
- WO-A1-2021/001404
- CUENCA MARTA ET AL: "CD84 cell surface signaling molecule: An emerging biomarker and target for cancer and autoimmune disorders", CLINICAL IMMUNOLOGY, ELSEVIER, AMSTERDAM, NL, vol. 204, 26 October 2018 (2018-10-26), pages 43 - 49, XP085800151, ISSN: 1521-6616, [retrieved on 20181026], DOI: 10.1016/J.CLIM.2018.10.017
- DE LA FUENTE M A ET AL: "CD84 leukocyte antigen is a new member of the Ig superfamily", BLOOD, AMERICAN SOCIETY OF HEMATOLOGY, US, vol. 90, no. 6, 15 September 1997 (1997-09-15), pages 2398 - 2405, XP002124601, ISSN: 0006-4971

## Description

### FIELD OF THE INVENTION

The present invention relates to anti-CD84 chimeric antigen receptors (CARs), and their use in therapy, in particular cancer therapy.

### BACKGROUND TO THE INVENTION

The human CD84 protein is a "cluster of differentiation" first identified in 1993. Sequencing of CD84 has identified it as a member of the Slam *(Signaling Lymphocyte Activation Molecule)* family.

The extracellular part of the CD84 receptor contains a non-canonical IgV distal domain and an IgC2g proximal domain, a structure common to all members of the SLAM family and similar to CD2. CD84 functions as an homophilic adhesion molecule; the receptor:ligand interaction involves the IgV domain and is independent of the cytoplastic domain and there are specific differences in the homophilic interfaces that prevent the binding of CD84 to other molecules of the SLAM family.

Human CD84 is expressed in various types of immune cells. Expression levels are heterogeneous depending on the cell type and its state of differentiation or activation. CD84 appears very early during haematopoietic differentiation in bone marrow progenitor cells and is expressed in most T and B cells, but with higher expression in memory T and B lymphocytes, follicular *helper* T cells and germinal centre B cells. Myeloid antigen-presenting cells, such as monocytes and monocyte-derived dendritic cells (DCs) are also positive for CD84. Granulocytes also express significant levels of CD84 with the highest expression on basophils and mast cells. Among all haematopoietic cells, platelets express the highest levels of CD84, which was initially proposed as an aggregation-induced signalling receptor implicated in stabilizing platelet-platelet interactions. However, studies with CD84-deficient mice indicate that it does not play a significant role in haemostatic and thrombotic function. CD84 expression is low in natural killer cells (NK) and absent in red blood cells.

CD84 signalling can activate or inhibit leukocyte function depending on the cell type and its stage of activation or differentiation. CD84-mediated signalling regulates diverse immunological processes, including T-cell cytokine secretion, natural killer-cell cytotoxicity, monocyte activation, autophagy, cognate T:B interactions and B-cell tolerance at the level of germinal centres.

Alterations in CD84 have been related to autoimmune disorders. For example, specific allelic variations in CD84 are associated with autoimmune diseases such as systemic lupus erythematosus and rheumatoid arthritis (RA). Typical treatment for such autoimmune diseases often involves disease-modifying anti-rheumatic drugs (DMARDS), which are not curative but stop or slow down symptoms and/or disease progression.

CD84 is expressed in certain cancers, in particular haematological malignancies, such as chronic lymphocytic leukaemia (CLL).

WO 2015/118538 relates to anti-CD84 antibodies, compositions comprising the same and uses thereof. WO 2021/001404 relates to a CD84 inhibitor for use in treating or preventing an ischemic disease and/or ischemic tissue damage. Cuenca et al. (2018) Clinical Immunology 204: 43-49 relates to reviewing the structure and function of CD84. De L Fuente et al. (1997) Blood 90: 2398-2405 relates to analysis of CD84 as a member of the Ig superfamily.

Haematological malignancies are often treated by chemotherapy or radiotherapy, which have known adverse side effects. Accordingly, there remains a significant need for improved treatments for haematological malignancies.

### SUMMARY OF THE INVENTION

The inventors have determined that CD84 is overexpressed in a range of cancer cell lines derived from malignant haematological diseases, including Burkitt's lymphoma, acute myeloid leukaemia (AML), chronic myeloid leukaemia (CML), B-cell acute lymphoblastic leukaemia (B-ALL), T-cell acute lymphoblastic leukaemia (T-ALL) and histiocytic lymphoma.

The inventors have studied and developed a range of antibodies and chimeric antigen receptors (CARs) that bind CD84.

In particular, the inventors have characterised the cellular expression of the CARs and functionally characterised CAR-T cells, for example through determination of their proliferation, cytokine production and their cytotoxicity against a range of target cancer cell lines. Moreover, the inventors have demonstrated that chimeric antigen receptors of the present invention can induce cytotoxicity in cancer cells expressing CD84.

Accordingly, the present invention provides a chimeric antigen receptor (CAR) that binds to CD84, wherein the CAR comprises an antigen-binding domain comprising:
a) a heavy chain variable region (VH) having complementarity determining regions (CDRs) with the sequences: CDR1 - NYWIN (SEQ ID NO: 1); CDR2-DIYPVSGTTNYNEKFKR (SEQ ID NO: 2); and CDR3-GTGRFAY (SEQ ID NO: 3); and
   a light chain variable region (VL) having CDRs with the sequences: CDR1 - RASQSVSTSSYSYMH (SEQ ID NO: 4); CDR2 - FASNLES (SEQ ID NO: 5); and CDR3 - QHSWEIPYT (SEQ ID NO: 6);
b) a heavy chain variable region (VH) having complementarity determining regions (CDRs) with the sequences: CDR1 - NYWLG (SEQ ID NO: 7); CDR2 - DIYPGGGYTNYIEKFKG (SEQ ID NO: 8); and CDR3 - YEGGYYGNYDAMDY (SEQ ID NO: 9); and
   a light chain variable region (VL) having CDRs with the sequences: CDR1 - RASESVDNYGISFMN (SEQ ID NO: 10); CDR2 - AASNQGS (SEQ ID NO: 11); and CDR3 - QQSKAVPRT (SEQ ID NO: 12);
c) a heavy chain variable region (VH) having complementarity determining regions (CDRs) with the sequences: CDR1 - GFTFSSYP (SEQ ID NO: 19); CDR2 - ISYHGRNK (SEQ ID NO: 20); and CDR3 - ARDRDATPGGTGVGNHGMAV (SEQ ID NO: 21); and
   a light chain variable region (VL) having CDRs with the sequences: CDR1 - QSLLHSSGYNY (SEQ ID NO: 22); CDR2 - MGS (SEQ ID NO: 23); and CDR3 - MQGLQTPPT (SEQ ID NO: 24); or
d) a heavy chain variable region (VH) having complementarity determining regions (CDRs) with the sequences: CDR1 - GFTFSDNA (SEQ ID NO: 25); CDR2 - ISGTGRTT (SEQ ID NO: 26); and CDR3 - AKWDCSDGRCYWAY (SEQ ID NO: 27); and

a light chain variable region (VL) having CDRs with the sequences: CDR1 - QSLVYSDGDTY (SEQ ID NO: 28); CDR2 - KVS (SEQ ID NO: 29); and CDR3 - MQGTHWPPNT (SEQ ID NO: 30).In some embodiments, the antigen-binding domain comprises:
   a) a VH domain having the sequence of SEQ ID NO: 61; and a VL domain having the sequence of SEQ ID NO: 62 or 108;
   b) a VH domain having the sequence of SEQ ID NO: 63; and a VL domain having the sequence of SEQ ID NO: 64 or 109;
   c) a VH domain having the sequence of SEQ ID NO: 68; and a VL domain having the sequence of SEQ ID NO: 69; or
   d) a VH domain having the sequence of SEQ ID NO: 71; and a VL domain having the sequence of SEQ ID NO: 72;
or variants thereof each having at least 90%, at least 95%, at least 96%, at least 97%, at least 98% or at least 99%, preferably at least 90%, sequence identity thereto.

Suitably, the antigen-binding domain is a CD84-binding domain.

In some embodiments, the antigen-binding domain is an scFv.

In some embodiments, the CAR is an scFv CAR.

In some embodiments, the CAR comprises a CD8a or a CD28 transmembrane domain. In preferred embodiments, the CAR comprises a CD8a transmembrane domain.

The antigen-binding domain and the transmembrane domain may be connected by a spacer. In some embodiments, the spacer comprises a CD8a hinge or a CD28 hinge. In preferred embodiments, the CAR comprises a CD8a hinge.

The CAR may comprise one or more, for example two or three, intracellular signalling domains. In preferred embodiments, the CAR comprises a CD3-zeta signalling domain.

The CAR may comprise one or more, for example two or three, co-stimulatory domains. In some embodiments, the CAR comprises one or more co-stimulatory domains selected from the group consisting of a 4-1BB co-stimulatory domain, a CD28 co-stimulatory domain, a OX40 co-stimulatory domain and an ICOS co-stimulatory domain.

In preferred embodiments, the CAR comprises a 4-1BB co-stimulatory domain. In some embodiments, the CAR comprises a CD28 co-stimulatory domain. In some embodiments, the CAR comprises an OX40 co-stimulatory domain. In some embodiments, the CAR comprises an ICOS co-stimulatory domain.

In preferred embodiments, the CAR comprises a 4-1BB co-stimulatory domain and a CD3-zeta signalling domain.

In preferred embodiments, the CAR comprises the antigen-binding domain of the invention, a CD8a transmembrane domain, a 4-1BB co-stimulatory domain and a CD3-zeta signalling domain.

In another aspect, the invention provides a polynucleotide comprising one or more nucleotide sequences encoding the CAR of the invention.

In another aspect, the invention provides a vector comprising the polynucleotide of the invention.

In some embodiments, the vector is a viral vector. In some embodiments, the vector is a retroviral vector or lentiviral vector, preferably a lentiviral vector.

In another aspect, the invention provides a cell comprising the polynucleotide or the vector of the invention.

In another aspect, the invention provides a cell comprising the CAR of the invention.

The cell may comprise a further (second) CAR.

In another aspect, the invention provides a cell comprising a first CAR and a second CAR, wherein the first CAR is the CAR of the invention.

In another aspect, the invention provides a composition comprising a first cell and a second cell, wherein the first cell comprises a first CAR and the second cell comprises a second CAR, and wherein the first CAR is the CAR of the invention.

In some embodiments, the first CAR and the second CAR are different. In some embodiments, the first CAR and the second CAR bind to different antigens.

In another aspect, the invention provides a cell comprising a tandem CAR, wherein the tandem CAR comprises a first antigen-binding domain (e.g. scFv) and a second antigen-binding domain (e.g. scFv), and wherein the first antigen-binding domain (e.g. scFv) is the antigen-binding domain (e.g. scFv) as defined in claim 1.

In some embodiments, the first antigen-binding domain (e.g. scFv) and the second antigen-binding domain (e.g. scFv) are different. In some embodiments, the first antigen-binding domain (e.g. scFv) and the second antigen-binding domain (e.g. scFv) bind to different antigens.

In some embodiments, the second CAR is an anti-CD19 CAR, an anti-CD20 CAR or an anti-CD22 CAR (e.g. for the treatment of B-cell malignancies); an anti-CD33 CAR or an anti-CD123 CAR (e.g. for the treatment of AML); or an anti-CD7 CAR (e.g. for the treatment of T-ALL).

In some embodiments, the second antigen-binding domain (e.g. scFv) is an anti-CD19 antigen-binding domain (e.g. scFv), an anti-CD20 antigen-binding domain (e.g. scFv) or an anti-CD22 antigen-binding domain (e.g. scFv) (e.g. for the treatment of B-cell malignancies); an anti-CD33 antigen-binding domain (e.g. scFv) or an anti-CD123 antigen-binding domain (e.g. scFv) (e.g. for the treatment of AML); or an anti-CD7 antigen-binding domain (e.g. scFv) (e.g. for the treatment of T-ALL).

In some embodiments, the cell is a T cell or NK cell, preferably a T cell. In some embodiments, the T cell is an autologous or allogeneic T cell. In some embodiments, the NK cell is an autologous or allogeneic NK cell.

NK cells may, for example, be sourced from any suitable tissue sample, cell line or stem cell source. In some embodiments, the NK cell (preferably allogeneic NK cell) is from cord blood (CB), induced pluripotent stem cells (iPSCs), bone marrow (BM), human embryonic stem cells (hESCs), a cell line (e.g. NK92 or YT), or peripheral blood (PB) (e.g. autologous or allogeneic). Preferably, the NK cell is from CB.

In another aspect, the invention provides a pharmaceutical composition comprising the CAR, the polynucleotide, the vector or the cell of the invention.

In another aspect, the invention provides the CAR, the polynucleotide, the vector, the cell or the pharmaceutical composition of the invention for use in therapy.

In some embodiments, the therapy is treatment of cancer.

In preferred embodiments, the cancer is a haematological malignancy.

In preferred embodiments, the cancer cells express CD84, for example the haematological malignancy may be a CD84-expressing haematological malignancy.

In some embodiments, the cancer is selected from the group consisting of chronic lymphocytic leukaemia (CLL), B-cell lymphoma, diffuse large B-cell lymphoma (DLBCL), Burkitt's lymphoma, follicular lymphoma, mantle cell lymphoma, B-cell acute lymphoblastic leukaemia (B-ALL), acute myeloid leukaemia (AML), myelodysplastic syndrome, T-cell acute lymphoblastic leukaemia/lymphoma (T-ALL), chronic myeloproliferative syndrome, chronic myeloid leukaemia (CML), chronic myelomonocytic leukaemia, dendritic-cell neoplasm and histiocytic sarcoma.

In some embodiments, the cancer is selected from the group consisting of chronic lymphocytic leukaemia (CLL), diffuse large B-cell lymphoma (DLBCL), Burkitt's lymphoma, follicular lymphoma, mantle cell lymphoma, B-cell acute lymphoblastic leukaemia (B-ALL), T-cell acute lymphoblastic leukaemia/lymphoma (T-ALL), acute myeloid leukemia (AML) and histiocytic sarcoma.

In preferred embodiments, the cancer is selected from the group consisting of CLL, B-cell lymphoma, B-ALL, T-ALL and AML.

In another aspect, the invention provides the CAR, the polynucleotide, the vector, the cell or the pharmaceutical composition of the invention for use in treating AML.

In another aspect, the invention provides the CAR, the polynucleotide, the vector, the cell or the pharmaceutical composition of the invention for use in treating T-ALL.

In another aspect, the invention provides the CAR, the polynucleotide, the vector, the cell or the pharmaceutical composition of the invention for use in treating B-cell lymphoma.

In another aspect, the invention provides the CAR, the polynucleotide, the vector, the cell or the pharmaceutical composition of the invention for use in treating Burkitt's lymphoma.

### DESCRIPTION OF THE DRAWINGS

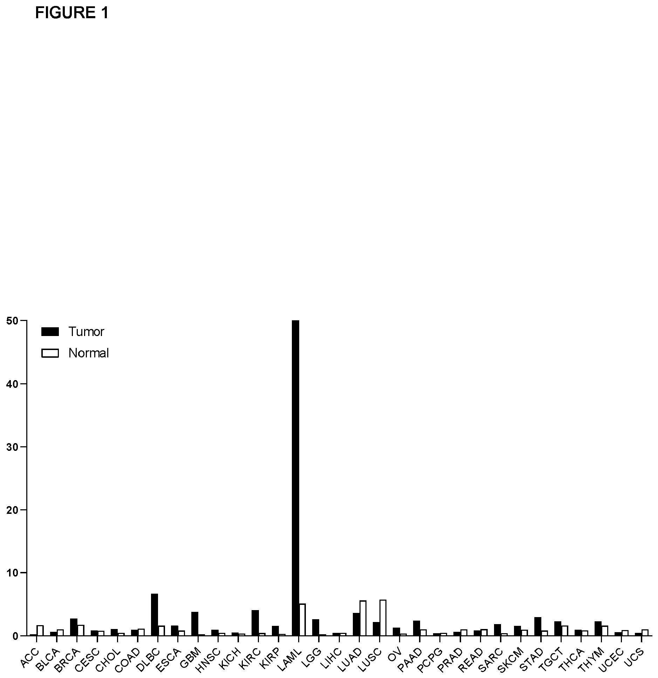

**Figure 1****.** Bar plot representing CD84 (alias LY9B, SLAMF5, hCD84, mCD84) gene expression profile across all tumour samples and paired normal tissues. The height of bar represents the median expression of certain tumour type or normal tissue. From http://gepia.cancer-pku.cn/detail.php?gene=CD84.

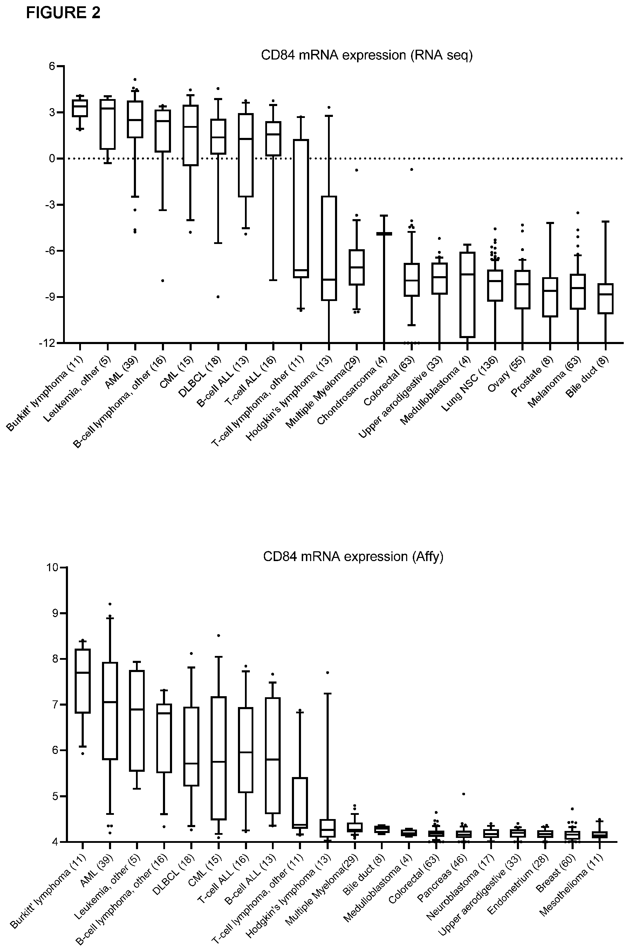

**Figure 2****.** Box and whisker (10-90 percentile) representation of CD84 mRNA expression measured by RNA sequencing (top) or Affymetrix microarrays (bottom) in cell lines obtained from different tumour types. From https://portals.broadinstitute.org/ccle. The figure only shows the 20 tumour types with the highest CD84 expression.

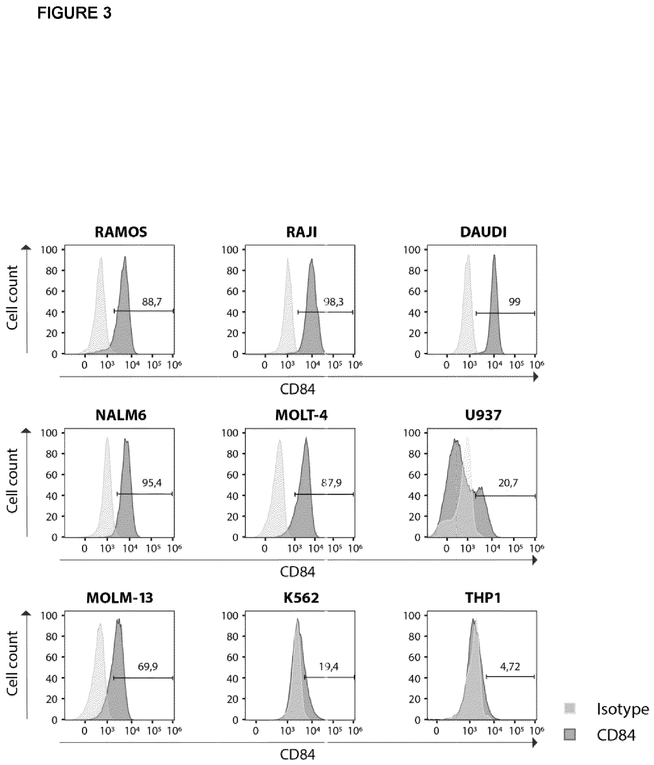

**Figure 3****.** Expression of CD84 in different cell lines. The histogram in light grey represents the staining with an isotype-matched control antibody and the histogram in dark grey the staining with the specific CD84 antibody.

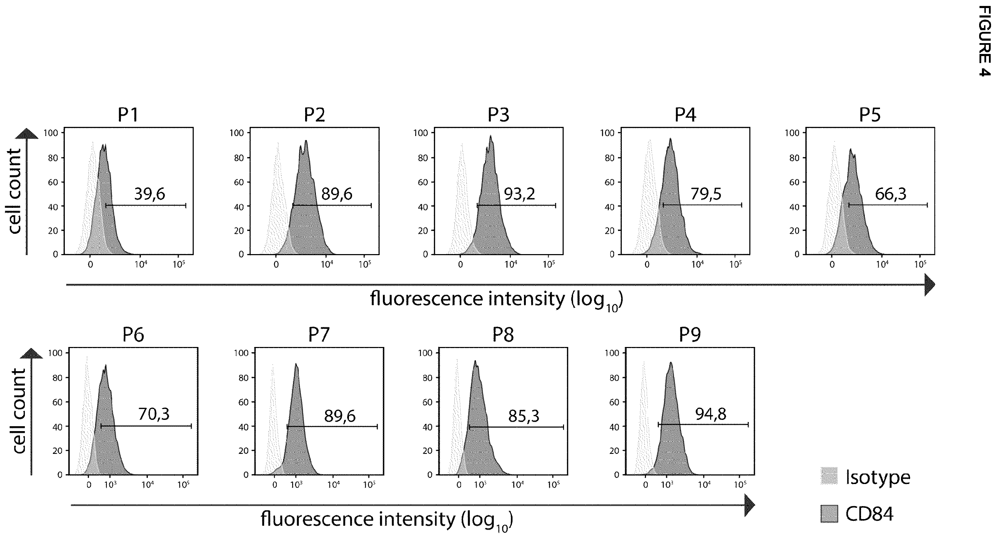

**Figure 4****.** Expression of CD84 in nine samples from patients diagnosed with chronic lymphocytic leukaemia assessed by flow cytometry. The histogram in light grey represents the staining with an isotype-matched control antibody and the histogram in dark grey the staining with the specific CD84 antibody.

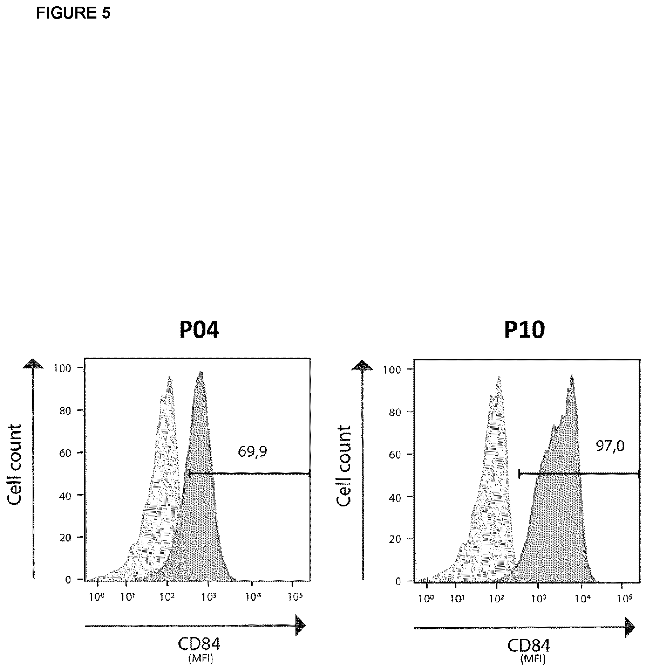

**Figure 5****.** Expression of CD84 in ten samples from patients diagnosed with acute myeloid leukaemia was assessed by flow cytometry. One representative example is shown for moderate expression of CD84, patient P04, and one for high expression of CD84, patient P10. The histogram in light grey represents the staining with an isotype-matched control antibody and the histogram in dark grey the staining with the specific CD84 antibody.

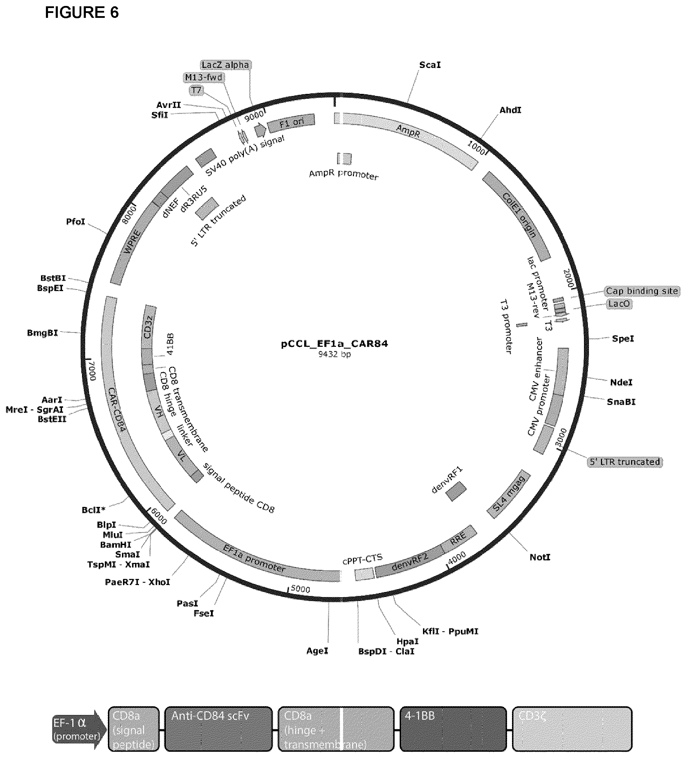

**Figure 6****.** Map of the pCCL-EF1α_CAR84 plasmid vector showing where the CD84 CAR sequence has been inserted (top). Scheme of the anti-CD84 2^{nd} generation CAR construct (bottom).

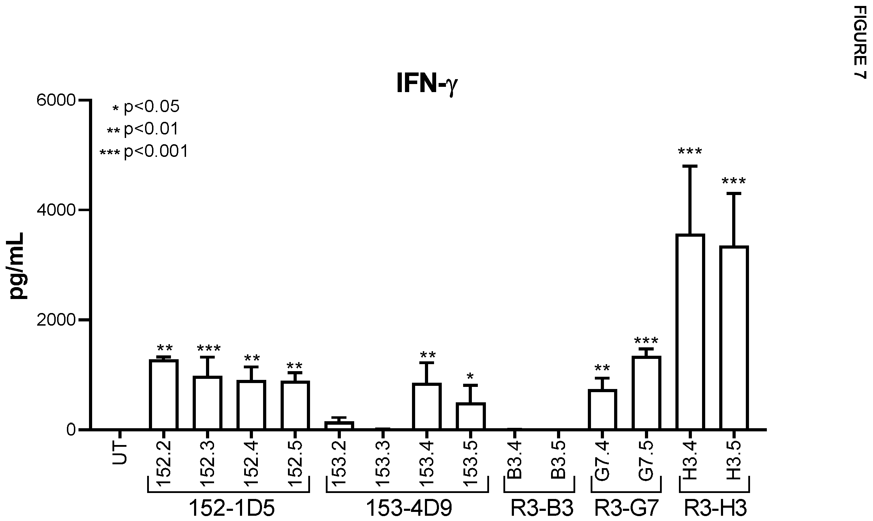

**Figure 7****.** IFN-y secretion by CD84-targeting CAR-T cells in co-culture with Ramos cells at an effector:target ratio of 2:1, following a 24 h incubation. UT: untransduced T cells. Statistical significance was determined with a Kruskal-Wallis (multiple comparisons to UT). The mean of 4 experiments ± SEM is shown. *** p < 0.001, ** p < 0.01, * p < 0.05.

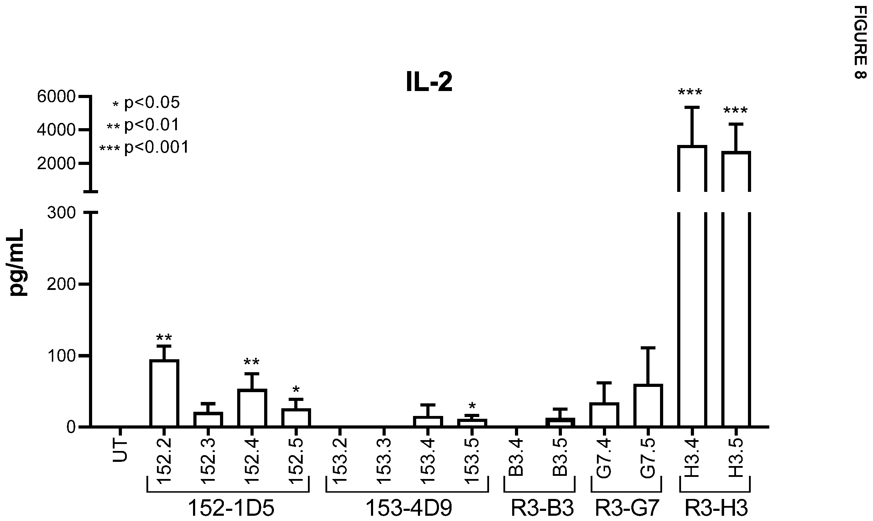

**Figure 8****.** IL-2 secretion by CD84-targeting CART cells in co-culture with Ramos cells at an effector:target ratio of 2:1, following a 24 h incubation. UT: untransduced T cells. Statistical significance was determined with a Kruskal-Wallis (multiple comparisons to UT). The mean of 4 experiments ± SEM is shown. *** p < 0.001, ** p < 0.01, * p < 0.05.

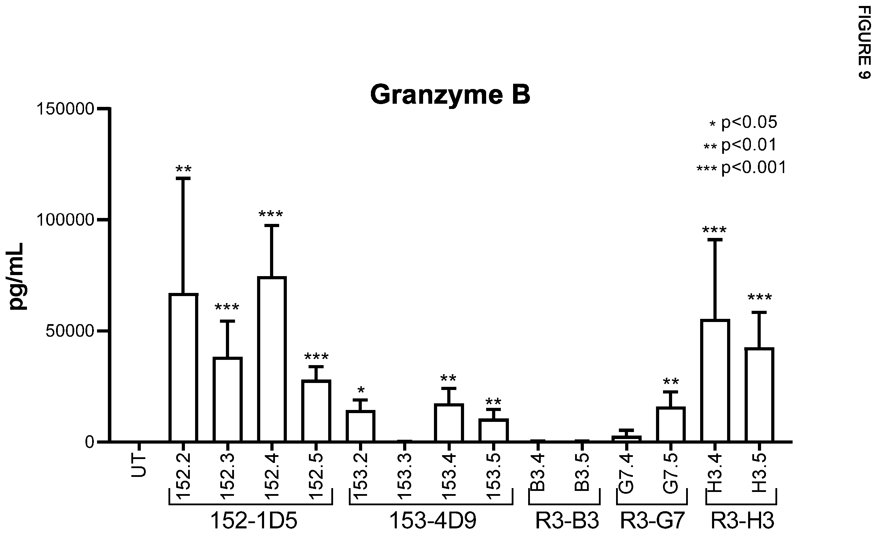

**Figure 9****.** Granzyme B secretion by CD84-targeting CART cells in co-culture with Ramos cells at an effector:target ratio of 2:1, following a 24 h incubation. UT: untransduced T cells. Statistical significance was determined with a Kruskal-Wallis (multiple comparisons to UT). The mean of 4 experiments ± SEM is shown. *** p < 0.001, ** p < 0.01, * p < 0.05.

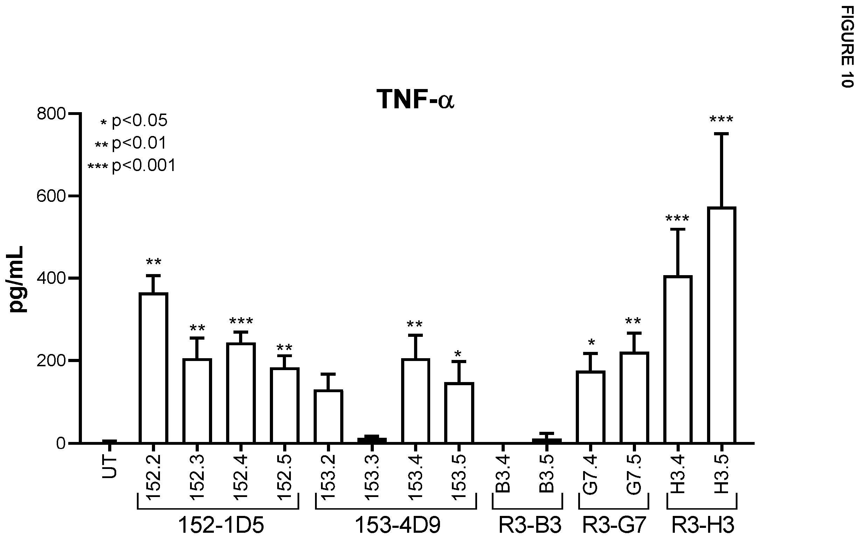

**Figure 10****.** TNF-α secretion by CD84-targeting CART cells in co-culture with Ramos cells at an effector:target ratio of 2:1, following a 24 h incubation. UT: untransduced T cells. Statistical significance was determined with a Kruskal-Wallis (multiple comparisons to UT). The mean of 4 experiments ± SEM is shown. *** p < 0.001, ** p < 0.01, * p < 0.05.

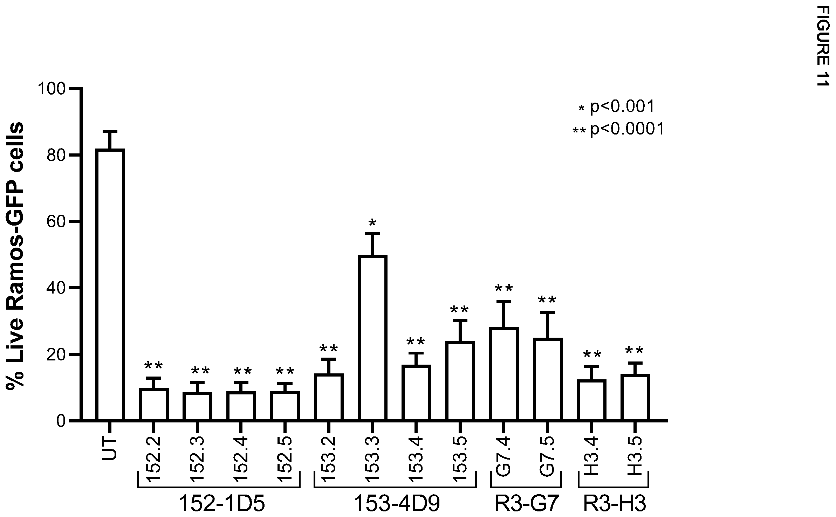

**Figure 11****.** Cytotoxicity assay of different CD84-targeting CART cells vs. Ramos-GFP⁺ cells after an incubation period of 24 hours at an effector:target ratio of 2:1. Percentage of target surviving cells relative to untreated cells (target cells alone) is shown. Mean of 8 independent experiments ± SEM. UT: untransduced T cells. Statistical significance was determined with an ordinary one-way ANOVA (multiple comparisons to UT): ** p < 0.001, * p < 0.01.

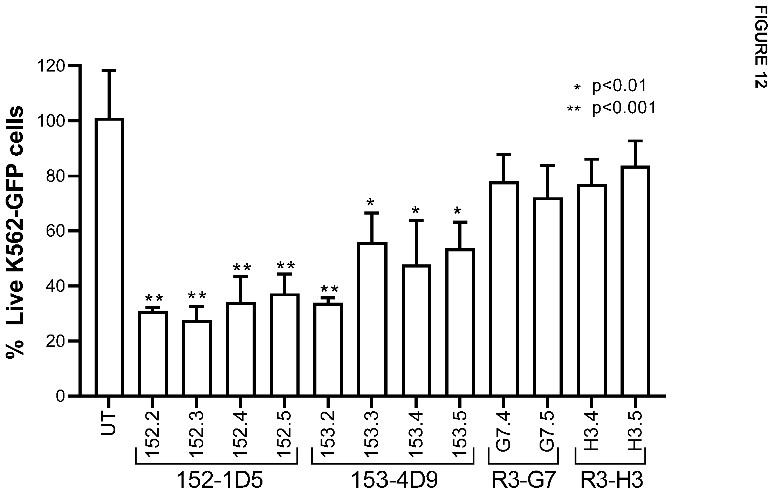

**Figure 12****.** Cytotoxicity assay of different CD84-targeting CART cells vs. K562-GFP⁺ cells after an incubation period of 24 hours at an effector:target ratio of 2:1. Percentage of target surviving cells relative to untreated cells (target cells alone) is shown. Mean of 8 independent experiments ± SEM. UT: untransduced T cells. Statistical significance was determined with an ordinary one-way ANOVA (multiple comparisons to UT): ** p < 0.001, * p < 0.01.

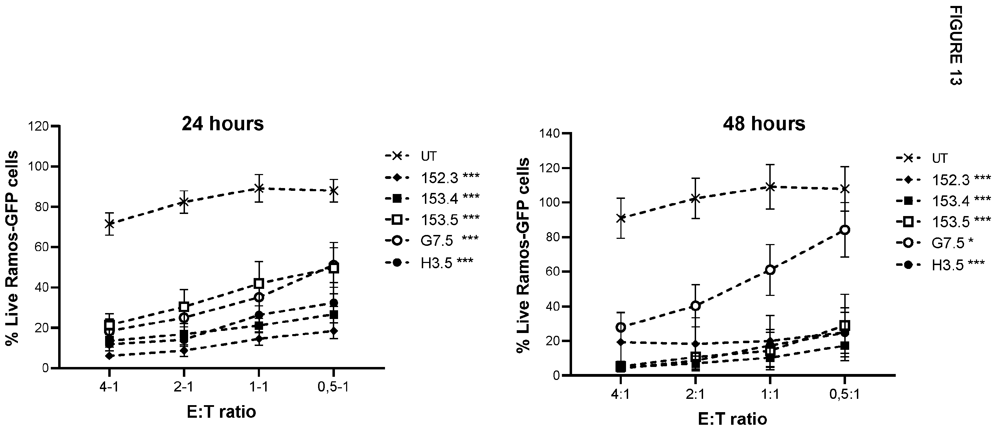

**Figure 13****.** Cytotoxicity assay of different CD84-targeting CART cells vs. Ramos-GFP⁺ cells after an incubation period of 24 and 48 hours at effector:target (E:T) cell ratios of 4:1, 2:1, 1:1 and 0.5:1. Percentage of target surviving cells relative to untreated cells (target cells alone) is shown. Mean of 5 independent experiments ± SEM. UT: untransduced T cells. Statistical significance was determined with an ordinary one-way ANOVA (multiple comparisons to UT): *** p < 0.001, * p < 0.05.

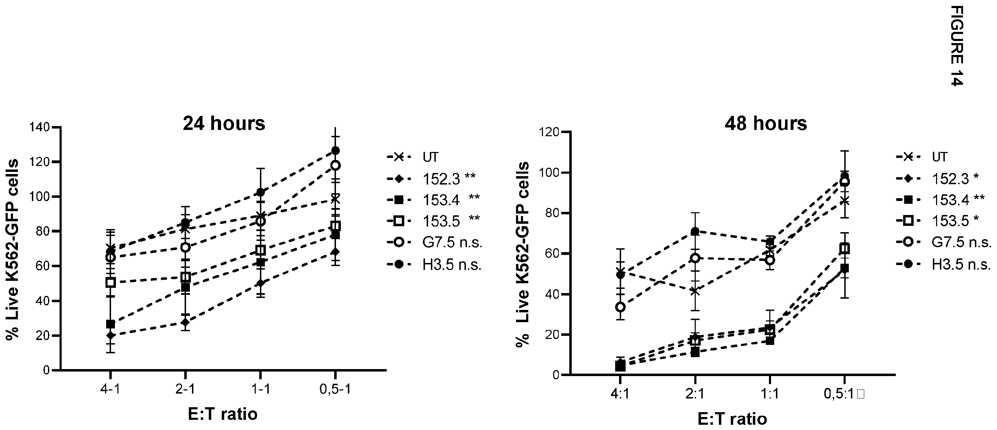

**Figure 14****.** Cytotoxicity assay of different CD84-targeting CART cells vs. K562-GFP⁺ cells after an incubation period of 24 and 48 hours at effector:target (E:T) cell ratios of 4:1, 2:1, 1:1 and 0.5:1. Percentage of target surviving cells relative to untreated cells (target cells alone) is shown. UT: untransduced T cells. Mean of 5 independent experiments ± SEM. Statistical significance was determined with an ordinary one-way ANOVA (multiple comparisons to UT): ** p <0.01, * p < 0.05, n.s.: non significant.

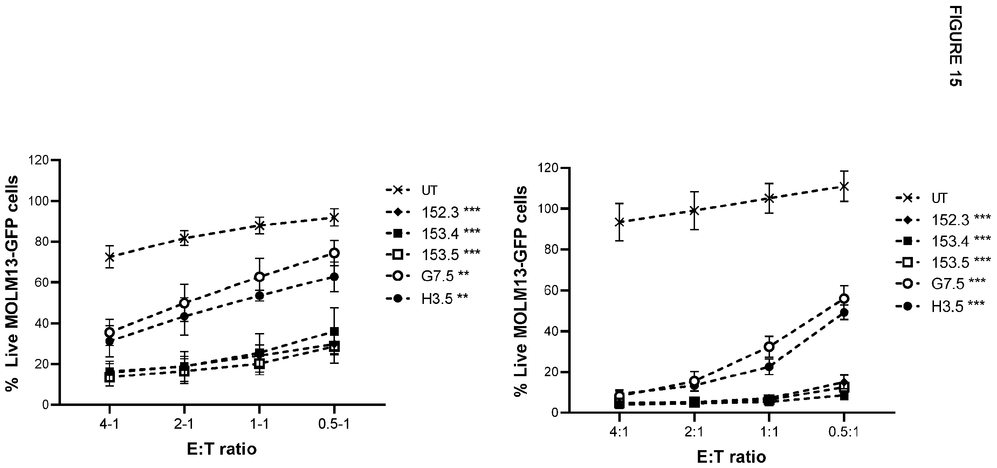

**Figure 15****.** Cytotoxicity assay of different CD84-targeting CART cells *vs.* MOLM-13-GFP⁺ cells after an incubation period of 24 and 48 hours at effector:target (E:T) cell ratios of 4:1, 2:1, 1:1 and 0.5:1. Percentage of target surviving cells relative to untreated cells (target cells alone) is shown. Mean of 3 independent experiments ± SEM, UT: untransduced T cells. Statistical significance was determined with an ordinary one-way ANOVA (multiple comparisons to UT): *** p < 0.001, ** p < 0.01, * p < 0.05.

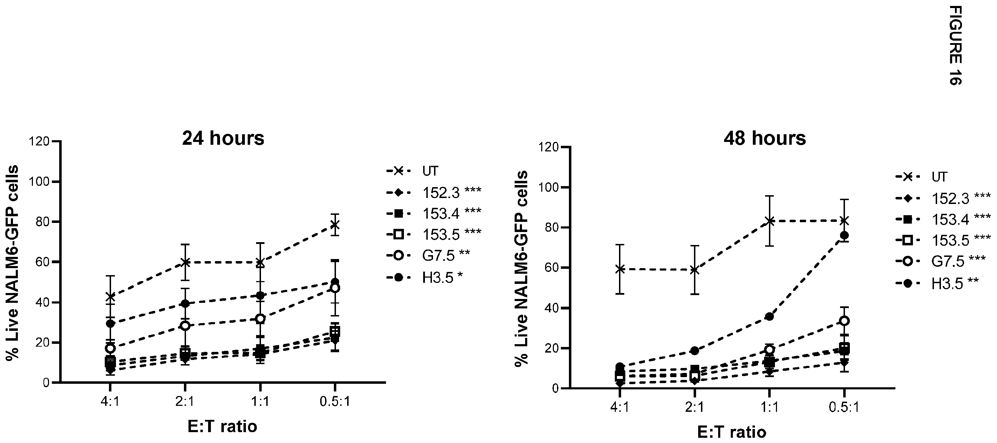

**Figure 16****.** Cytotoxicity assay of different CD84-targeting CART cells vs. NALM6-GFP⁺ cells after an incubation period of 24 and 48 hours at effector:target (E:T) cell ratios of 4:1, 2:1, 1:1 and 0.5:1. Percentage of target surviving cells relative to untreated cells (target cells alone), is shown. Mean of 3 independent experiments ± SEM. UT: untransduced T cells. Statistical significance was determined with an ordinary one-way ANOVA (multiple comparisons to UT): *** p < 0.001, ** p < 0.01, * p < 0.05.

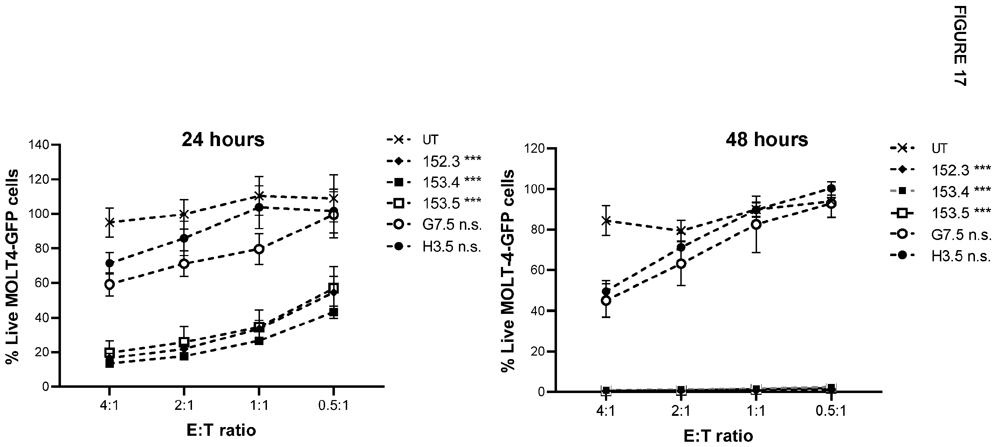

**Figure 17****.** Cytotoxicity assay of different CD84-targeting CART cells vs. MOLT4-GFP⁺ cells after an incubation period of 24 and 48 hours at effector:target ratios of 4:1, 2:1, 1:1 and 0.5:1. Percentage of target surviving cells relative to untreated cells (target cells alone), is shown. Mean of 4 independent experiments ± SEM. UT: untransduced T cells. Statistical significance was determined with an ordinary one-way ANOVA (multiple comparisons to UT): ** p < 0.01, * p < 0.05, ns: non significant.

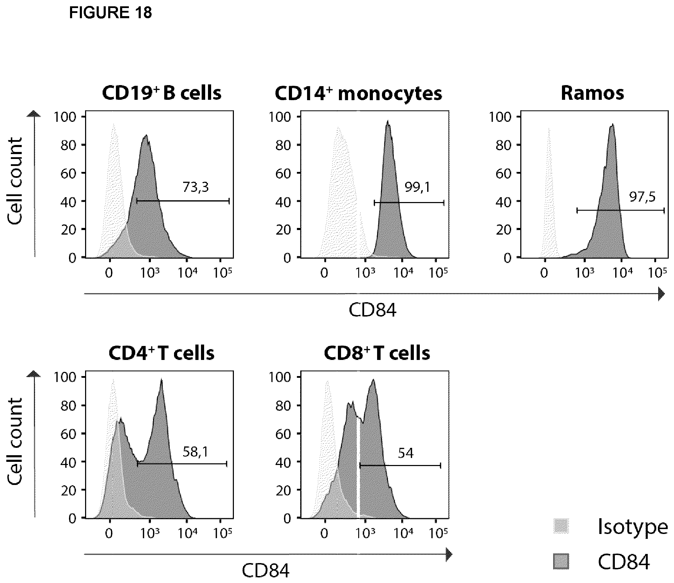

**Figure 18****.** Expression of CD84 in peripheral blood mononuclear cells (PBMC) assessed by flow cytometry. The histogram in light grey represents the staining with an isotype-matched control antibody and the histogram in dark grey the staining with the specific CD84 antibody. PBMC were stained with specific antibodies for CD4, CD8, CD19, and CD14.

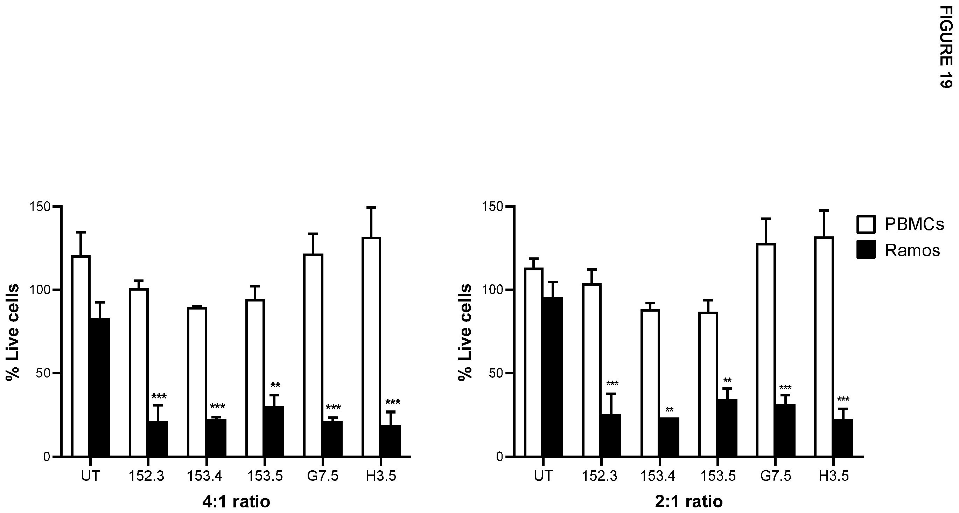

**Figure 19****.** Cytotoxicity assay of different CD84-targeting CART cells *vs*. PBMC labelled with CFSE⁺ and Ramos-GFP⁺ cells after an incubation period of 16 hours. Percentage of target surviving cells relative to untreated cells (target cells alone) is shown. Mean of 3 independent experiments from 3 different donors ± SEM. UT: untransduced T cells. Statistical significance was determined with a paired T test (compared to UT): ** p < 0.01, n.s. = non significant.

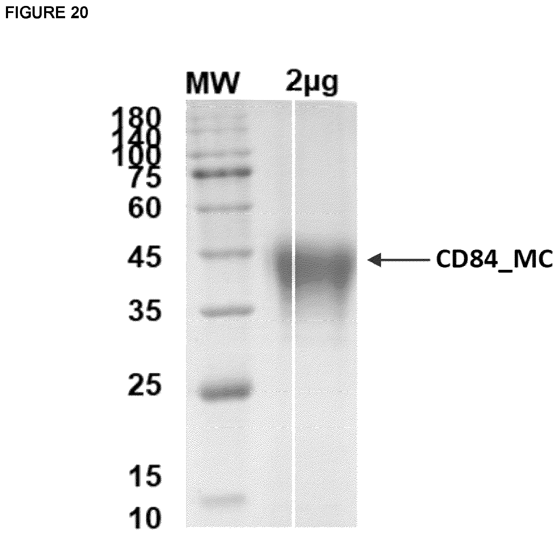

**Figure 20****.** SDS PAGE gel image of CD84 antigen. Reduced SDS PAGE with Coomassie blue staining.

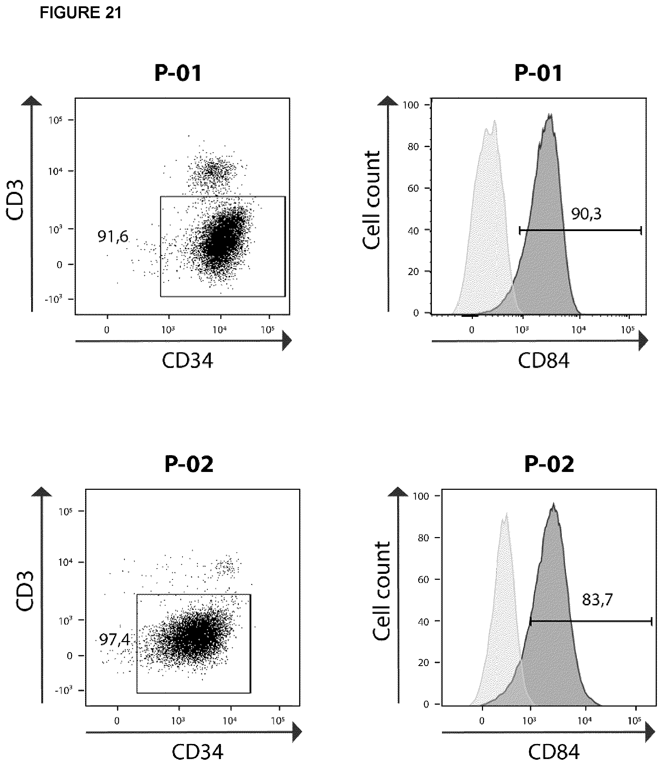

**Figure 21****.** CD84 expression assessed by flow cytometry on leukemic cells from peripheral blood from two patients diagnosed with T-ALL (P01 and P02). The histograms represent CD84 expression on blasts gated for CD34; histograms in light grey represent the staining with an isotype-matched control antibody and those in dark grey, the staining with the specific CD84 antibody.

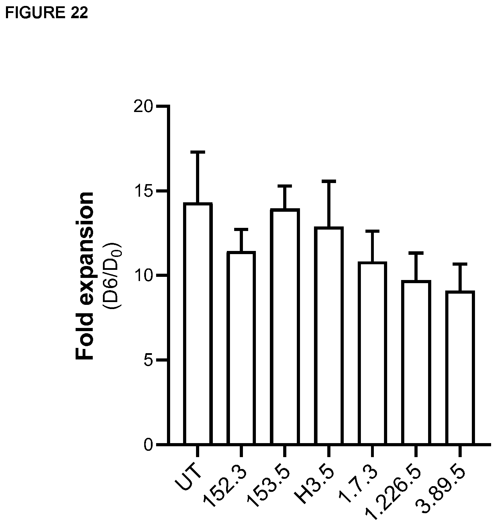

**Figure 22****.** Expansion of CAR T cells 6-7 days after transduction is shown as fold increase over number of T cells at day 0 of culture. The figure represents between 5 and 12 independent expansions, each using cells from a different healthy donor. UT: untransduced T cells.

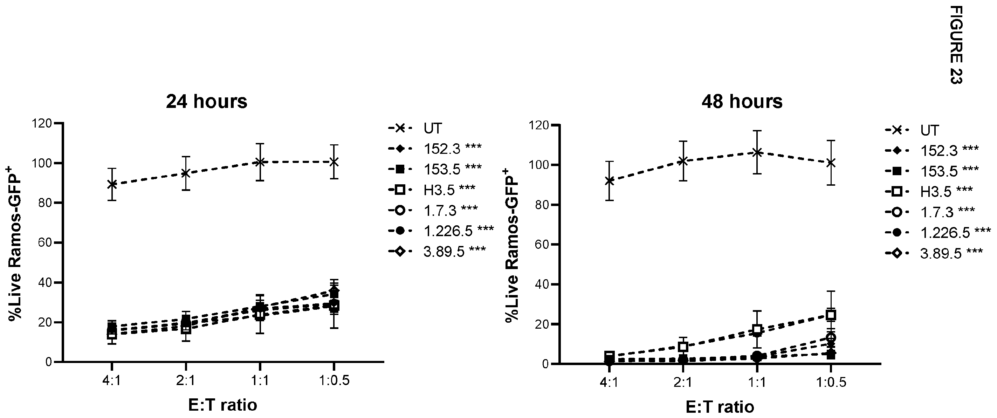

**Figure 23****.** Cytotoxicity assays of different CD84-targeting CART cells vs. Ramos-GFPffLuc cells after an incubation period of 24 and 48 hours at effector:target (E:T) cell ratios of 4:1, 2:1, 1:1 and 0.5:1. Percentage of target surviving cells relative to untreated cells (target cells alone) is shown. Mean of 5 independent experiments ± SEM. UT: untransduced T cells. Statistical significance was determined with a two-way ANOVA test (multiple comparisons to UT): *** p < 0.001, ** p < 0.01, * p < 0.05.

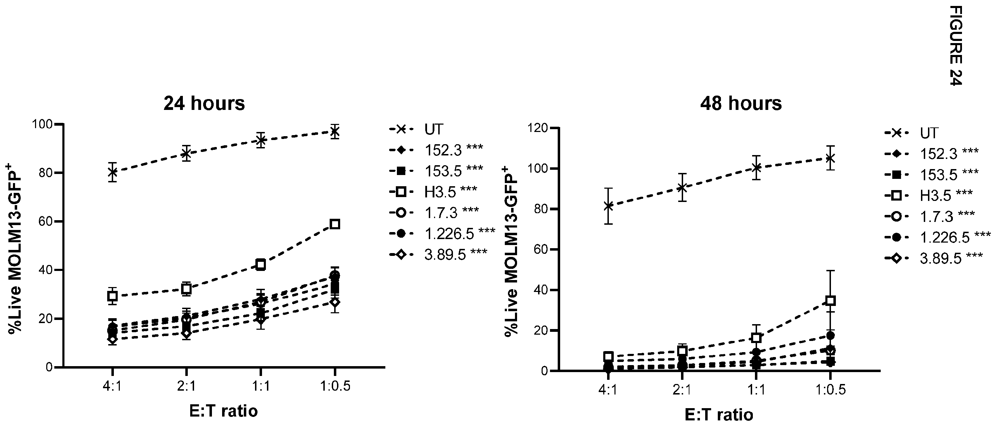

**Figure 24****.** Cytotoxicity assays of different CD84-targeting CART cells *vs.* MOLM-13 GFPffLuc cells after an incubation period of 24 and 48 hours at effector:target (E:T) cell ratios of 4:1, 2:1, 1:1 and 0.5:1. Percentage of target surviving cells relative to untreated cells (target cells alone) is shown. Mean of at least 5 independent experiments ± SEM. UT: untransduced T cells. Statistical significance was determined with a two-way ANOVA test (multiple comparisons to UT): *** p < 0.001, ** p < 0.01, * p < 0.05.

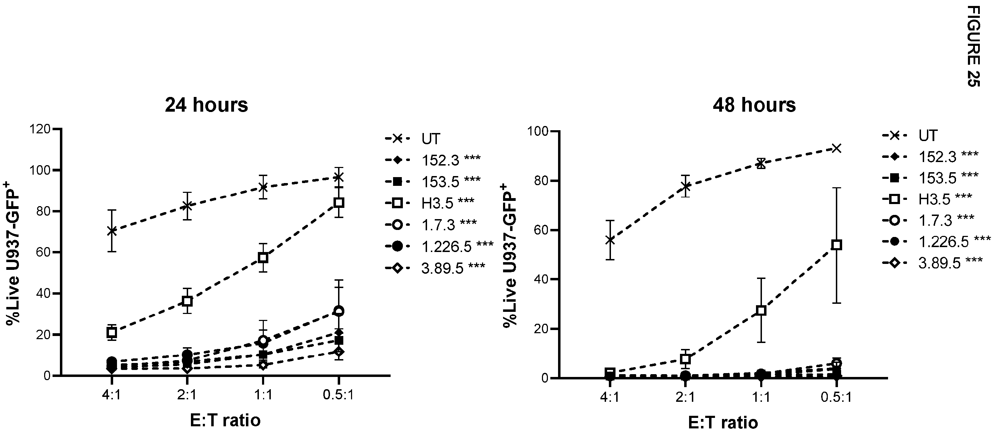

**Figure 25****.** Cytotoxicity assays of different CD84-targeting CART cells vs. U937 GFPffLuc cells after an incubation period of 24 and 48 hours at effector:target (E:T) cell ratios of 4:1, 2:1, 1:1 and 0.5:1. Percentage of target surviving cells relative to untreated cells (target cells alone) is shown. Mean of at least 3 independent experiments ± SEM. UT: untransduced T cells. Statistical significance was determined with a two-way ANOVA test (multiple comparisons to UT): *** p < 0.001, ** p < 0.01, * p < 0.05.

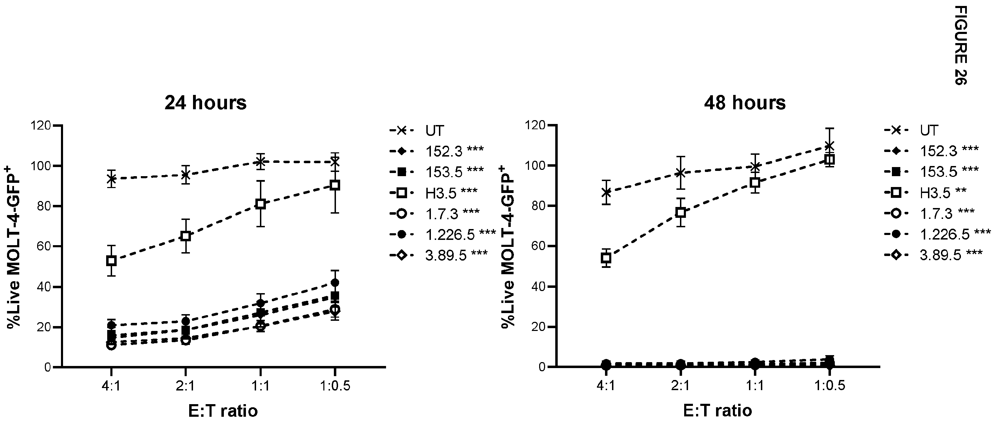

**Figure 26****.** Cytotoxicity assays of different CD84-targeting CART cells *vs.* MOLT-4 GFPffLuc cells after an incubation period of 24 and 48 hours at effector:target (E:T) cell ratios of 4:1, 2:1, 1:1 and 0.5:1. Percentage of target surviving cells relative to untreated cells (target cells alone) is shown. Mean of at least 5 independent experiments ± SEM. UT: untransduced T cells. Statistical significance was determined with a two-way ANOVA test (multiple comparisons to UT): *** p < 0.001, ** p < 0.01, * p < 0.05.

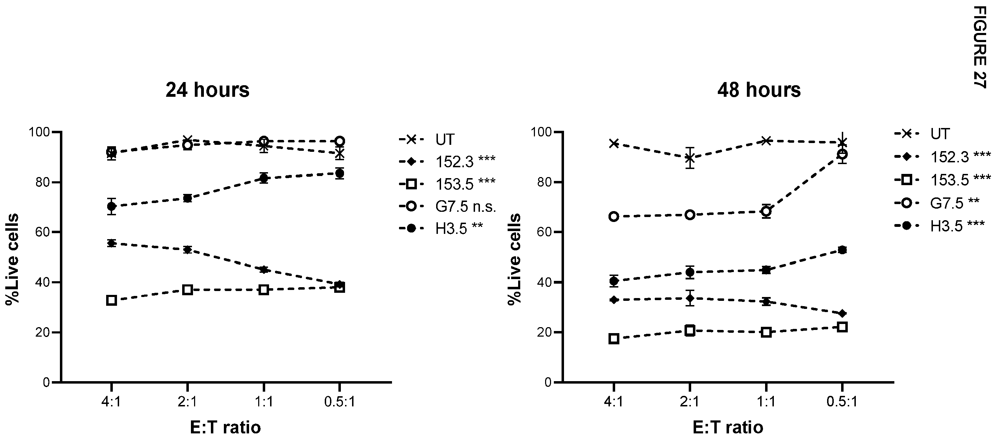

**Figure 27****.** Cytotoxicity assay of different CD84-targeting CART cells *vs*. primary AML cells stained with CFSE after an incubation period of 24 and 48 hours at effector:target (E:T) cell ratios of 4:1, 2:1, 1:1 and 0.5:1. Percentage of target surviving cells relative to untreated cells (target cells alone) is shown. UT: untransduced T cells. Statistical significance was determined with a two-way ANOVA test (multiple comparisons to UT): *** p < 0.001, ** p < 0.01, * p < 0.05.

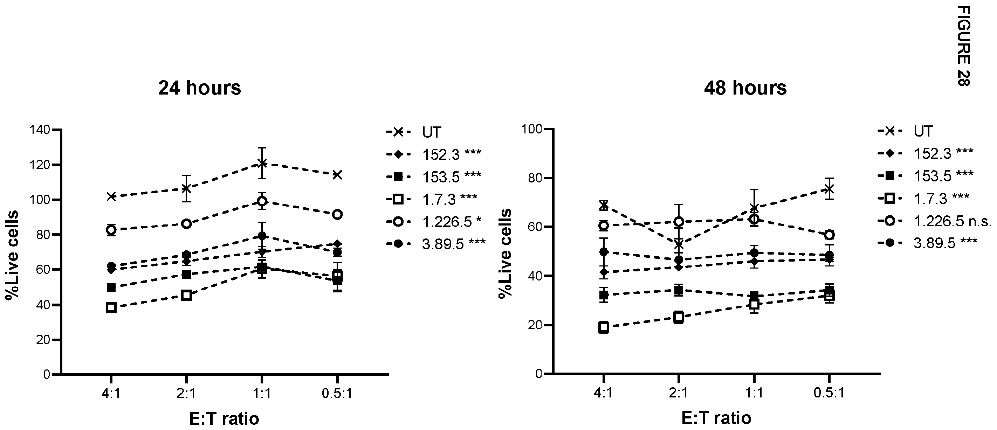

**Figure 28****.** Cytotoxicity assay of different CD84-targeting CART cells *vs*. primary T-ALL cells stained with CFSE after an incubation period of 24 and 48 hours at effector:target (E:T) cell ratios of 4:1, 2:1, 1:1 and 0.5:1. Percentage of target surviving cells relative to untreated cells (target cells alone) is shown. UT: untransduced T cells. Statistical significance was determined with a two-way ANOVA test (multiple comparisons to UT): *** p < 0.001, ** p < 0.01, * p < 0.05.

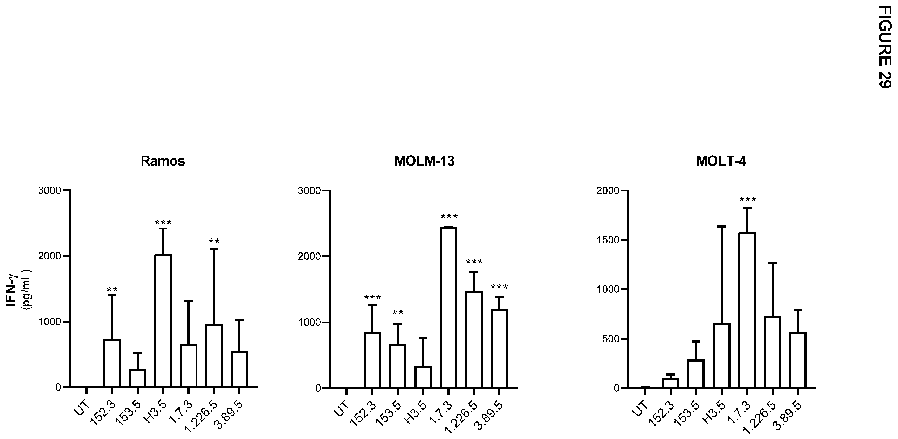

**Figure 29****.** IFN-γ secretion by CD84-targeting CAR-T cells in co-culture with Ramos, MOLM-13 or MOLT-4 cells, as indicated, at an effector:target ratio of 2:1, following a 24 h incubation. UT: untransduced T cells. Statistical analysis was determined with a 2-way ANOVA mixed-effect analysis assuming sphericity and Dunnet post-test for multiple comparisons (*vs*. UT). The mean of at least 3 experiments ± SD is shown. *** p < 0.001, ** p < 0.01, * p < 0.05.

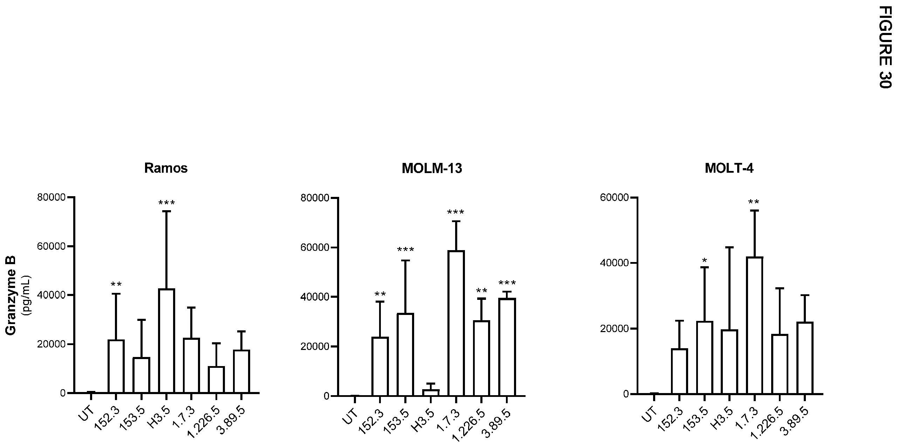

**Figure 30****.** Granzyme B secretion by CD84-targeting CAR-T cells in co-culture with Ramos, MOLM-13 or MOLT-4 cells, as indicated, at an effector:target ratio of 2:1, following a 24 h incubation. UT: untransduced T cells. Statistical analysis was determined with a 2-way ANOVA mixed-effect analysis assuming sphericity and Dunnet post-test for multiple comparisons (*vs*. UT). The mean of at least 3 experiments ± SD is shown. *** p < 0.001, ** p < 0.01, * p < 0.05.

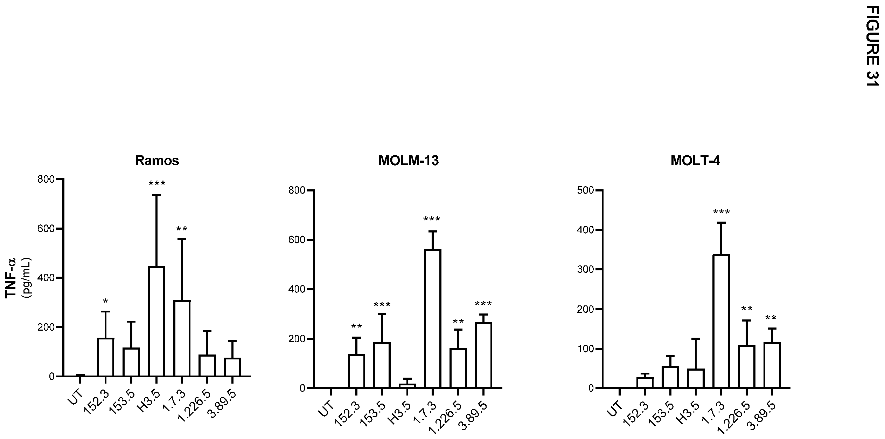

**Figure 31****.** TNF-α secretion by CD84-targeting CAR-T cells in co-culture with Ramos, MOLM-13 or MOLT-4 cells, as indicated, at an effector:target ratio of 2:1, following a 24 h incubation. UT: untransduced T cells. Statistical analysis was determined with a 2-way ANOVA mixed-effect analysis assuming sphericity and Dunnet post-test for multiple comparisons (*vs*. UT). The mean of at least 3 experiments ± SD is shown. *** p < 0.001, ** p < 0.01, * p < 0.05.

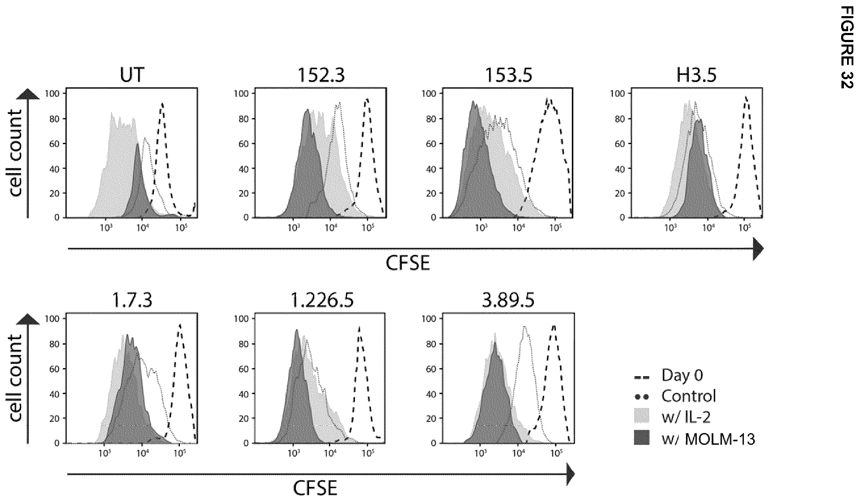

**Figure 32****.** CART-cell proliferation *in vitro* measured by a CFSE assay at a 4-day time point (flow cytometry images). Proliferation on day 0, after 4 days only with media (control), when stimulated with IL-2 or in the presence of MOLM-13 AML cells. UT: untransduced T cells.

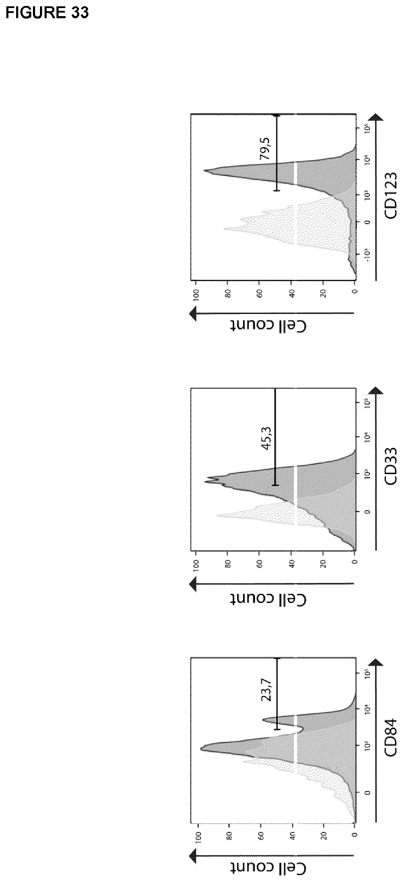

**Figure 33****.** CD84, CD33 and CD123 expression assessed by flow cytometry on CD34⁺ HPSC isolated from the apheresis product of a healthy donor for an allogeneic stem cell transplant. The histograms in light grey represent the staining with an isotype-matched control antibody and those in dark grey, the staining with the specific antibody.

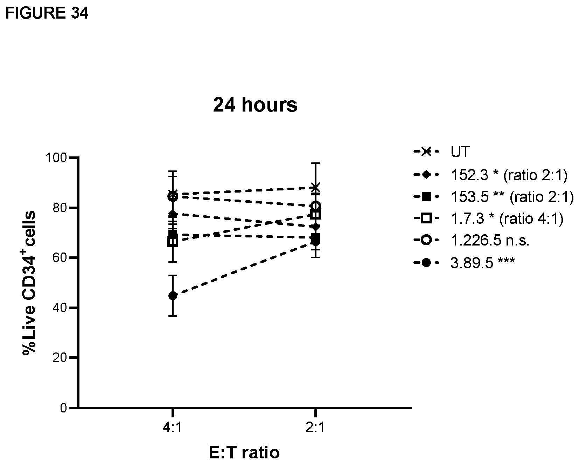

**Figure 34****.** Cytotoxicity assay of different CD84-targeting CART cells *vs*. CD34⁺ HPSC isolated from 5 different cord blood units after an incubation period of 24 hours at effector:target (E:T) cell ratios 4:1 and 2:1. Percentage of target surviving cells relative to untreated cells (target cells alone) is shown. Mean of 5 independent experiments ± SEM. UT: untransduced T cells. Statistical significance was determined with a 2-way ANOVA (multiple comparisons to UT): *** p < 0.001, **p<0.01, *p<0.05.

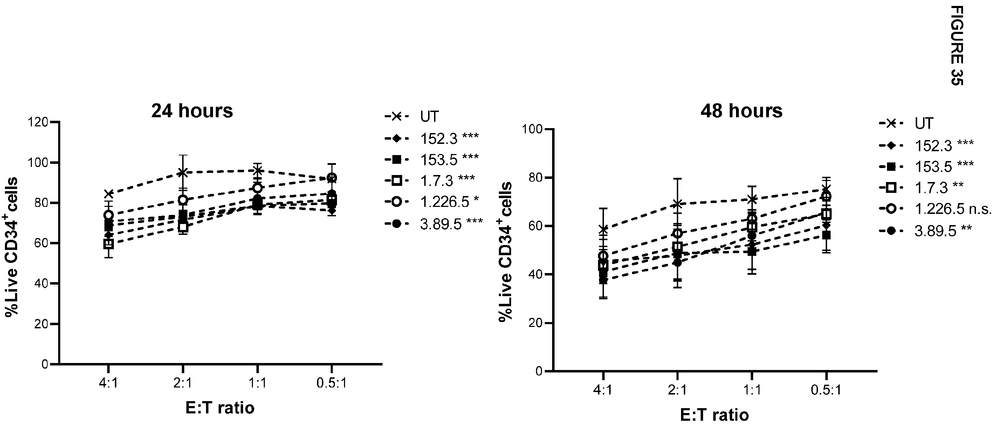

**Figure 35****.** Cytotoxicity assay of different CD84-targeting CART cells vs. CD34⁺ HPSC isolated from the apheresis product of a healthy donor for an allogeneic stem cell transplant, after an incubation period of 24 hours at effector:target (E:T) cell ratios 4:1, 2:1, 1:1 and 0.5:1. Percentage of target surviving cells relative to untreated cells (target cells alone) is shown. Mean of three independent experiments ± SEM. UT: untransduced T cells. Statistical significance was determined with a 2-way ANOVA (multiple comparisons to UT): *** p < 0.001, **p<0.01, *p<0.05.

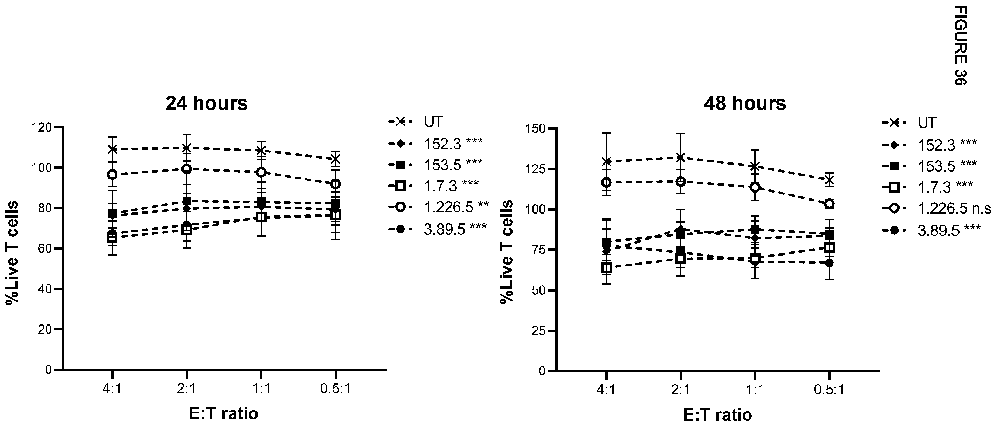

**Figure 36****.** Cytotoxicity assay of different CD84-targeting CART cells vs. CD3⁺ T cells isolated from the same donor, after an incubation period of 24 hours at effector:target (E:T) cell ratios 4:1, 2:1, 1:1 and 0.5:1. Percentage of target surviving T cells relative to untreated cells (target T cells alone) is shown. Mean of 5 independent experiments ± SEM. UT: untransduced T cells. Statistical significance was determined with a 2-way ANOVA (multiple comparisons to UT): *** p < 0.001, **p<0.01, *p<0.05.

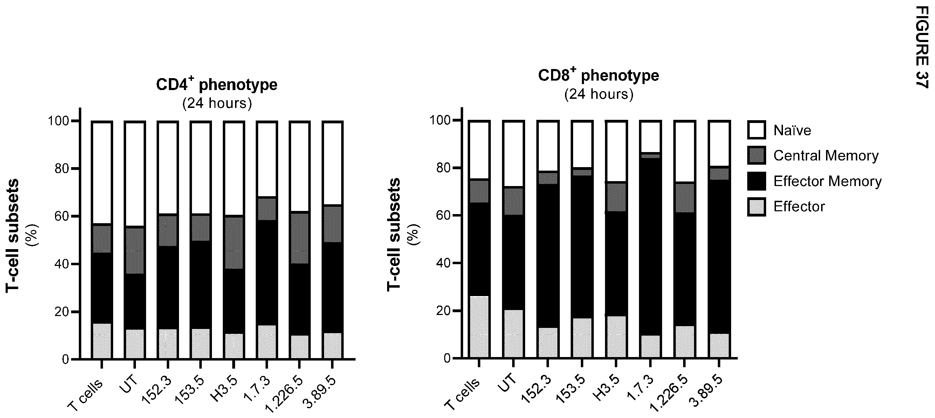

**Figure 37****.** Analysis by flow cytometry of the different T-cell subsets on target T cells before and on surviving target T cells after co-culture with the different CART84. The following T-cell subsets were studied: CD45RA+/CD62L+ naïve (white), CD45RA-/CD62L+ central-memory (dark grey), CD45RA-/CD62L- effector-memory (black), and CD45RA+/CD62L- effector T cells (light grey).

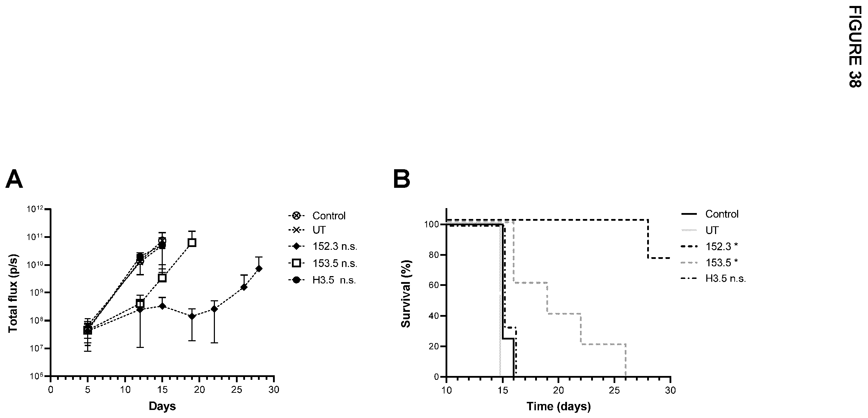

**Figure 38****.** Efficacy of CART84 cells against MOLM-13 cells *in vivo.* MOLM-13 disease progression was monitored weekly by bioluminescence. Bioluminescence quantification (A) and animal survival (B). Statistical analysis of bioluminescence quantification was performed with a 2-way ANOVA model with Dunnett's multiple comparisons vs. UT-treated mice. Statistical significance of survival was determined with a Log-Rank test with corrected p value for 3 comparisons.

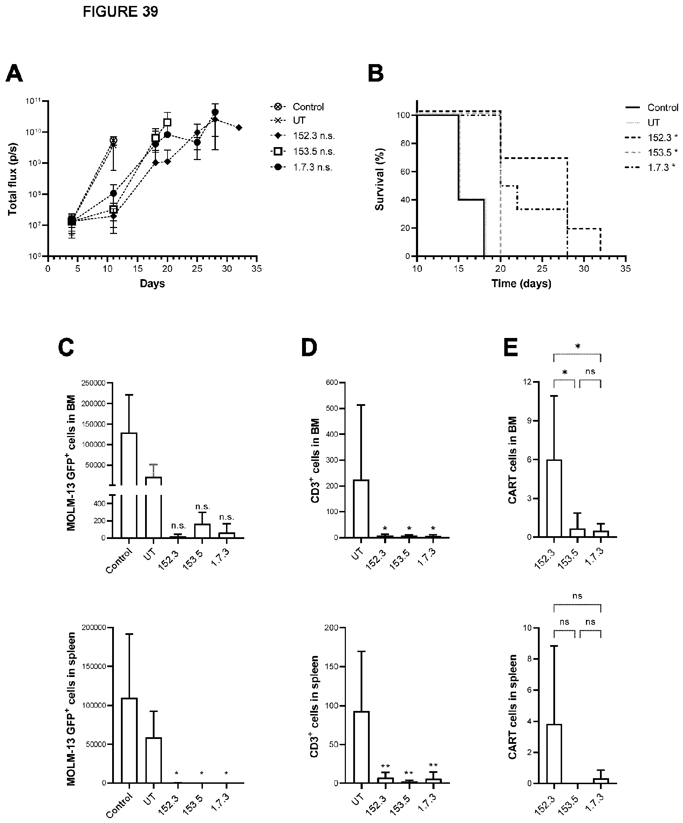

**Figure 39****.** Efficacy of CART84 cells against MOLM-13 cells *in vivo.* MOLM-13 disease progression was monitored weekly by bioluminescence. Bioluminescence quantification (A) and animal survival (B). Total number of MOLM-13 GFP-ffLuc cells (C), CD3⁺T cells (D) and CART cells (E) assessed by flow cytometry in the bone marrow (top) and spleen (bottom) of mice at the experiment end-point. Statistical analysis of bioluminescence quantification was performed with a 2-way ANOVA model with Dunnett's multiple comparisons vs. UT-treated mice. Statistical significance of survival was determined with a Log-Rank test with corrected p value for 3 comparisons. Statistical analysis of MOLM-13 and CD3⁺ T cells quantification was performed with a one-way ANOVA model with Dunnett's multiple comparisons vs. UT-treated mice; and analysis of CART cell quantification with a one-way ANOVA model with Tukey's multiple comparisons.

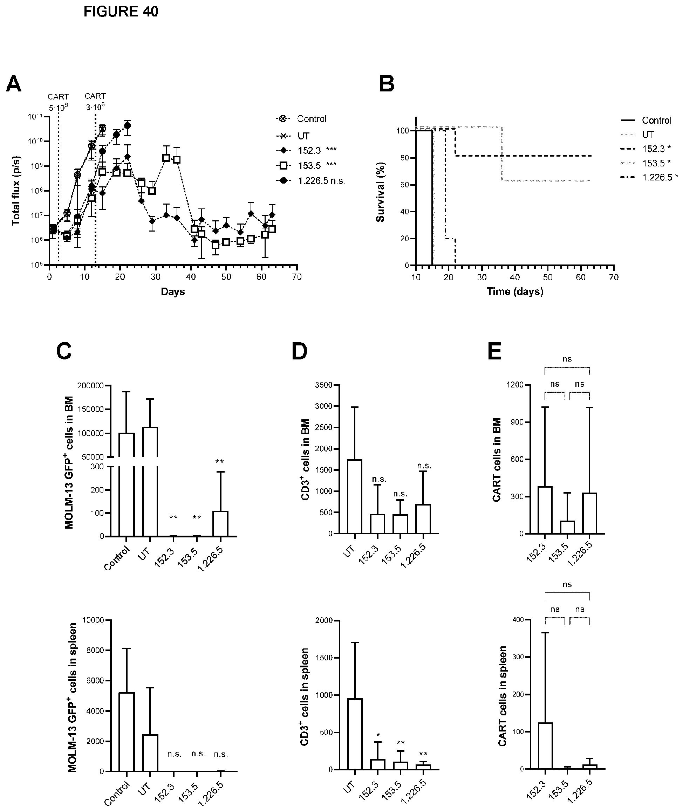

**Figure 40****.** Efficacy of CD84 CART cells against MOLM-13 cells *in vivo.* MOLM-13 disease progression was followed weekly by bioluminescence. Bioluminescence quantification (A) and animal survival (B). Total number of MOLM-13 GFP-ffLuc cells (C), CD3⁺T cells (D) and CART cells (E) assessed by flow cytometry in the bone marrow (top) and spleen (bottom) of mice at the experiment end-point. Statistical analysis of bioluminescence quantification was performed with a 2-way ANOVA model with Dunnett's multiple comparisons vs. UT-treated mice. Statistical significance of survival was determined with a Log-Rank test with corrected p value for 3 comparisons. Statistical analysis of MOLM-13 and CD3⁺ cells quantification was performed with a one-way ANOVA model with Dunnett's multiple comparisons vs. UT-treated mice; and analysis of CART cell quantification with a one-way ANOVA model with Tukey's multiple comparisons.

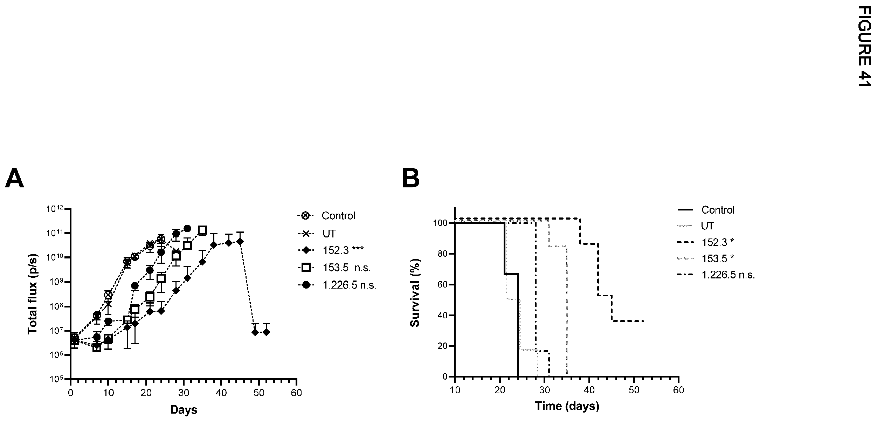

**Figure 41****.** Efficacy of CD84 CART cells against MOLT-4 cells *in vivo.* MOLT-4 disease progression was monitored weekly by bioluminescence. Bioluminescence quantification (A) and animal survival (B). Statistical analysis of bioluminescence quantification was performed with a 2-way ANOVA model with Dunnett's multiple comparisons *vs*. UT-treated mice. Statistical significance of survival was determined with a Log-Rank test with corrected p value for 3 comparisons.

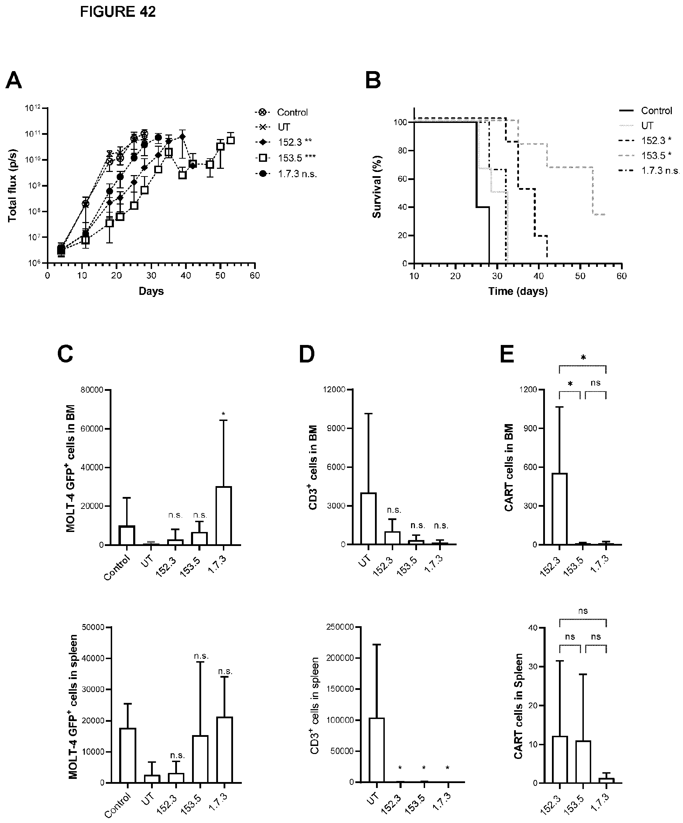

**Figure 42****.** Efficacy of CD84 CART cells against MOLT-4 cells *in vivo.* MOLT-4 disease progression was monitored weekly by bioluminescence. Bioluminescence quantification (A) and animal survival (B). Total number of MOLT-4 GFP-ffLuc cells (C), CD3⁺ T cells (D) and CART cells (E) detected by flow cytometry in the bone marrow (top) and spleen (bottom) of mice at the experiment end-point. Statistical analysis of bioluminescence quantification was performed with a 2-way ANOVA model with Dunnett's multiple comparisons vs. UT-treated mice. Statistical significance of survival was determined with a Log-Rank test with corrected p value for 3 comparisons. Statistical analysis of MOLT-4 and CD3⁺ cells quantification was performed with a one-way ANOVA model with Dunnett's multiple comparisons vs. UT-treated mice; and analysis of CART cell quantification with a one-way ANOVA model with Tukey's multiple comparisons.

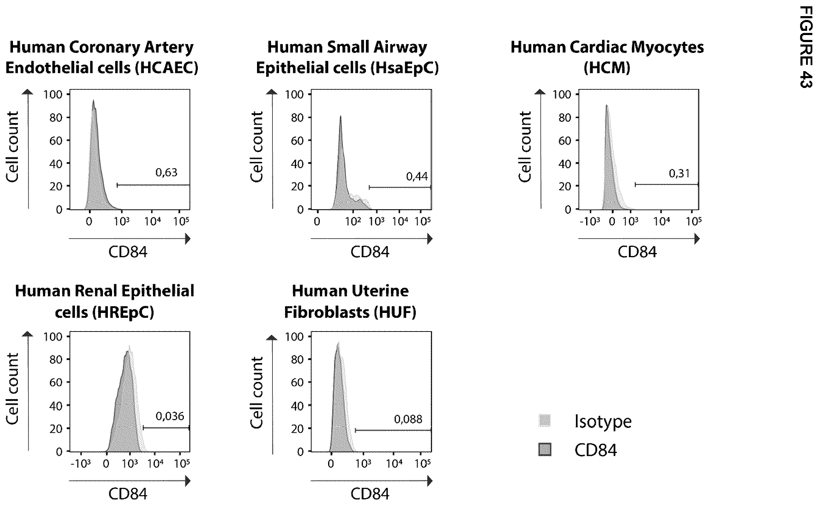

**Figure 43****.** CD84 expression on human primary cells assessed by flow cytometry. The histogram in light grey represents the staining with an isotype-matched control antibody and the histogram in dark grey the staining with the specific CD84 antibody.

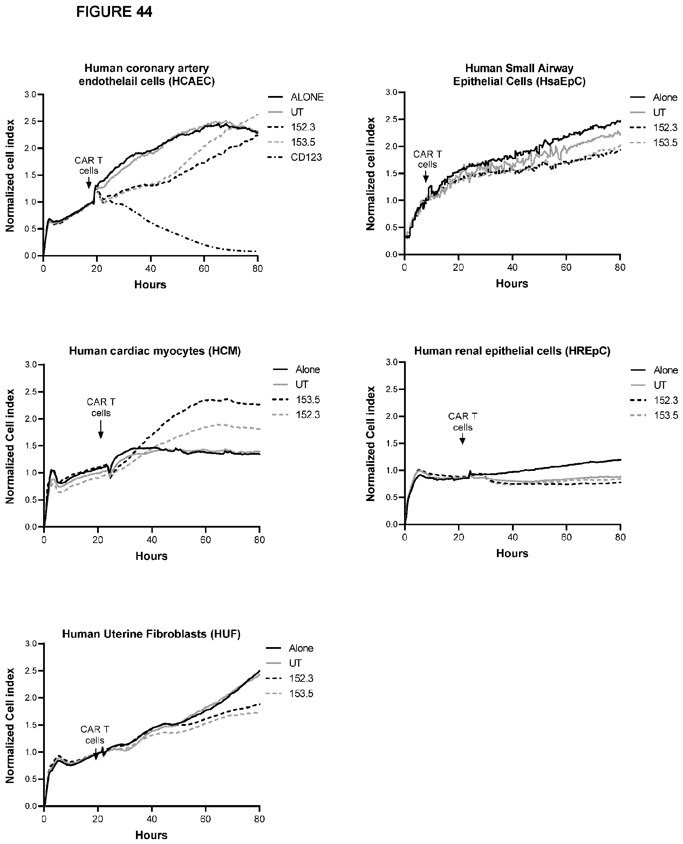

**Figure 44****.** Cytotoxicity assays of CART84 cells 152.3, 153.5 and UT *vs.* human primary cells, measured during 72 hours after CART / UT addition (arrow) to the cell culture using an XCELLigence instrument. UT: untransduced T cells.

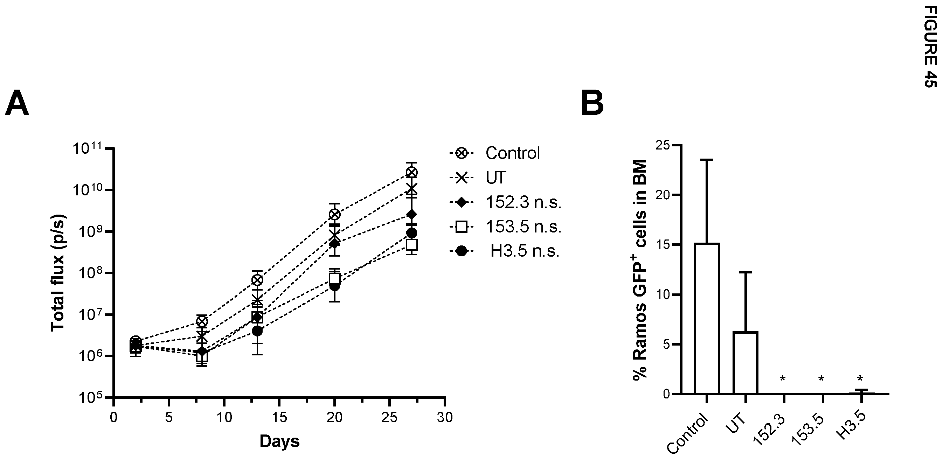

**Figure 45****.** Efficacy of CD84 CART cells against Ramos cells *in vivo.* (A) Ramos disease progression was monitored weekly by bioluminescence. Statistical analysis of bioluminescence quantification was performed with a 2-way ANOVA model with Dunnett's multiple comparisons vs. UT-treated mice. (B) Total number of Ramos GFP-ffLuc cells assessed by flow cytometry in the bone marrow of mice at the end-point. Statistical analysis was performed with a one-way ANOVA model with Dunnett's multiple comparisons *vs*. UT-treated mice. UT: untransduced T cells. * p < 0.05.

### DETAILED DESCRIPTION OF THE INVENTION

The terms "comprising", "comprises" and "comprised of" as used herein are synonymous with "including" or "includes"; or "containing" or "contains", and are inclusive or open-ended and do not exclude additional, non-recited members, elements or steps. The terms "comprising", "comprises" and "comprised of" also include the term "consisting of".

### CD84

CD84 (also known as LY9B and SLAMF5) is a membrane glycoprotein that is a member of the signalling lymphocyte activation molecule (SLAM) family, which itself is a subset of the larger CD2 cell-surface receptor subgroup of the Ig superfamily.

The extracellular part of the CD84 receptor contains a non-canonical IgV distal domain and an IgC2g proximal domain, a structure common to all members of the SLAM family. CD84 functions as an homophilic adhesion molecule and is expressed in numerous immune cell types. The receptor:ligand interaction involves the IgV domain and is independent of the cytoplastic domain. There are specific differences in the homophilic interfaces that prevent the binding of CD84 to other molecules of the SLAM family.

The inventors have determined that CD84 is overexpressed in a range of cell lines derived from malignant haematological diseases, including Burkitt's lymphoma, acute myeloid leukaemia (AML), chronic myeloid leukaemia (CML), B-cell acute lymphoblastic leukaemia (B-ALL), T-cell acute lymphoblastic leukaemia (T-ALL) and histiocytic lymphoma.

Studies have suggested that CD84 is overexpressed in chronic lymphocytic leukaemia (CLL).

An example amino acid sequence of CD84 is:

MAQHHLWILLLCLQTWPEAAGKDSEIFTVNGILGESVTFPVNIQEPRQVKIIAWTSKTSVAYVTPGDSE
TAPVVTVTHRNYYERIHALGPNYNLVISDLRMEDAGDYKADINTQADPYTTTKRYNLQIYRRLGKPKIT
QSLMASVNSTCNVTLTCSVEKEEKNVTYNWSPLGEEGNVLQIFQTPEDQELTYTCTAQNPVSNNSDSIS
ARQLCADIAMGFRTHHTGLLSVLAMFFLLVLILSSVFLFRLFKRRQGRIFPEGSCLNTFTKNPYAASKK
TIYTYIMASRNTQPAESRIYDEILQSKVLPSKEEPVNTVYSEVQFADKMGKASTQDSKPPGTSSYEIVI

(SEQ ID NO: 126)

### Antigen-binding domain

An antigen-binding domain may be a protein or peptide that possesses the ability to recognise and bind to an antigen. The antigen-binding domain includes any naturally occurring, synthetic, semi-synthetic or recombinantly produced binding partner for an antigen of interest.

Illustrative antigen-binding domains include antibodies or antibody fragments or derivatives, extracellular domains of receptors (e.g. TCRs), ligands for cell surface molecules/receptors, or receptor binding domains thereof.

In preferred embodiments, the antigen-binding domain is, or is derived from, an antibody. An antibody-derived domain can be a fragment of an antibody or a genetically engineered product of one or more fragments of the antibody, which fragment is involved in binding with the antigen. Examples include a variable region (Fv), a complementarity determining region (CDR), a Fab, a single chain variable fragment (scFv), a heavy chain variable region (VH), a light chain variable region (VL) and a camelid antibody (VHH).

The antigen-binding domain may be non-human (e.g. murine), chimeric, humanised or fully human.

In preferred embodiments, the antigen-binding domain is a single chain variable fragment (scFv). The scFv may be, for example, a murine, human or humanised scFv.

The term "complementarity determining region" (CDR) with regard to an antibody or antigen-binding fragment thereof refers to a highly variable loop in the variable region of the heavy chain or the light chain of an antibody. CDRs can interact with the antigen conformation and largely determine binding to the antigen (although some framework regions are known to be involved in binding). The heavy chain variable region and the light chain variable region each contain three CDRs.

"Heavy chain variable region" (VH) refers to the variable fragment of the heavy chain of an antibody that contains three CDRs interposed between flanking stretches known as framework regions, which are more highly conserved than the CDRs and form a scaffold to support the CDRs.

"Light chain variable region" (VL) refers to the variable fragment of the light chain of an antibody that contains three CDRs interposed between framework regions.

"Fv" refers to the smallest fragment of an antibody to bear the complete antigen-binding site. An Fv consists of the variable region of a single light chain bound to the variable region of a single heavy chain.

"Single chain variable fragment" (scFv) refers to an engineered antibody consisting of a light chain variable region (VL) and a heavy chain variable region (VH) connected to one another directly or via a peptide linker sequence.

The antigen-binding domain may specifically bind to the antigen, for example bind to the antigen but not bind to other peptides, or bind at a lower affinity to other peptides.

The binding affinity between two molecules (e.g. an antigen-binding domain and an antigen) may be quantified, for example, by determination of the dissociation constant (K_{D}). The K_{D} can be determined by measurement of the kinetics of complex formation and dissociation between the antigen-binding domain and antigen, e.g. using a technique such as surface plasmon resonance (SPR). The rate constants corresponding to the association and the dissociation of a complex are referred to as the association rate constant kₐ (or kₒₙ) and dissociation rate constant k_{d} (or k_{off}), respectively. K_{D} is related to kₐ and k_{d} through the equation K_{D} = k_{d} / kₐ.

Suitably, the antigen-binding domain is a CD84-binding domain.

| **SEQ ID NO.** | **Description** | **Sequence** |
|---|---|---|
| 1 | 152-1D5 | NYWIN |
| | CDR-H1 | |
| 2 | 152-1D5 | DIYPVSGTTNYNEKFKR |
| | CDR-H2 | |
| 3 | 152-1D5 | GTGRFAY |
| | CDR-H3 | |
| 4 | 152-1D5 | RASQSVSTSSYSYMH |
| | CDR-L1 | |
| 5 | 152-1D5 | FASNLES |
| | CDR-L2 | |
| 6 | 152-1D5 | QHSWEIPYT |
| | CDR-L3 | |
| 7 | 153-4D9 | NYWLG |
| | CDR-H1 | |
| 8 | 153-4D9 | DIYPGGGYTNYIEKFKG |
| | CDR-H2 | |
| 9 | 153-4D9 | YEGGYYGNYDAMDY |
| | CDR-H3 | |
| 10 | 153-4D9 | RASESVDNYGISFMN |
| | CDR-L1 | |
| 11 | 153-4D9 | AASNQGS |
| | CDR-L2 | |
| 12 | 153-4D9 | QQSKAVPRT |
| | CDR-L3 | |
| 13 | R3-B3 | GFTFSSYA |
| | CDR-H1 | |
| 14 | R3-B3 | ISGSGGST |
| | CDR-H2 | |
| 15 | R3-B3 | AKWDCSDGRCYWAY |
| | CDR-H3 | |
| 16 | R3-B3 | NIESKD |
| | CDR-L1 | |
| 17 | R3-B3 | DDA |
| | CDR-L2 | |
| 18 | R3-B3 | QVWDSSSDHW |
| | CDR-L3 | |
| 19 | R3-G7 | GFTFSSYP |
| | CDR-H1 | |
| 20 | R3-G7 | ISYHGRNK |
| | CDR-H2 | |
| 21 | R3-G7 | ARDRDATPGGTGVGNHGMAV |
| | CDR-H3 | |
| 22 | R3-G7 | QSLLHSSGYNY |
| | CDR-L1 | |
| 23 | R3-G7 | MGS |
| | CDR-L2 | |
| 24 | R3-G7 | MQGLQTPPT |
| | CDR-L3 | |
| 25 | R3-H3 | GFTFSDNA |
| | CDR-H1 | |
| 26 | R3-H3 | ISGTGRTT |
| | CDR-H2 | |
| 27 | R3-H3 | AKWDCSDGRCYWAY |
| | CDR-H3 | |
| 28 | R3-H3 | QSLVYSDGDTY |
| | CDR-L1 | |
| 29 | R3-H3 | KVS |
| | CDR-L2 | |
| 30 | R3-H3 | MQGTHWPPNT |
| | CDR-L3 | |
| 31 | GYCD84.1-7 | TSGMGVG |
| | CDR-H1 | |
| 32 | GYCD84.1-7 | HIWWDDVKRYNPALKS |
| | CDR-H2 | |
| 33 | GYCD84.1-7 | MRTSYYFDY |
| | CDR-H3 | |
| 34 | GYCD84.1-7 | RASENIFSSLA |
| | CDR-L1 | |
| 35 | GYCD84.1-7 | NAKTLAE |
| | CDR-L2 | |
| 36 | GYCD84.1-7 | QHHYATPFT |
| | CDR-L3 | |
| 37 | GYCD84.1-97 | SYWIN |
| | CDR-H1 | |
| 38 | GYCD84.1-97 | DIYLGSGSTNYNEKFKS |
| | CDR-H2 | |
| 39 | GYCD84.1-97 | SGGYLGY |
| | CDR-H3 | |
| 40 | GYCD84.1-97 | RASQSVSTSSYSYMH |
| | CDR-L1 | |
| 41 | GYCD84.1-97 | FASNLES |
| | CDR-L2 | |
| 42 | GYCD84.1-97 | QHSWEIPYT |
| | CDR-L3 | |
| 43 | GYCD84.1-108 | NYWIG |
| | CDR-H1 | |
| 44 | GYCD84.1-108 | DIYPGGGYTNYNENFKG |
| | CDR-H2 | |
| 45 | GYCD84.1-108 | STTYYSSYWCFDV |
| | CDR-H3 | |
| 46 | GYCD84.1-108 | KSSQSLLNSGNQANYLA |
| | CDR-L1 | |
| 47 | GYCD84.1-108 | GASTRES |
| | CDR-L2 | |
| 48 | GYCD84.1-108 | QNDHSYPFT |
| | CDR-L3 | |
| 49 | GYCD84.1-207 | RYWMS |
| | CDR-H1 | |
| 50 | GYCD84.1-207 | EINPDSSTINYTPSLKD |
| | CDR-H2 | |
| 51 | GYCD84.1-207 | PGPTVVATYWYFDV |
| | CDR-H3 | |
| 52 | GYCD84.1-207 | RSSQSIVHSNGNTYLE |
| | CDR-L1 | |
| 53 | GYCD84.1-207 | KVSSRFS |
| | CDR-L2 | |
| 54 | GYCD84.1-207 | FQGSHVPRT |
| | CDR-L3 | |
| 55 | GYCD84.1-226 | RYWIN |
| | CDR-H1 | |
| 56 | GYCD84.1-226 | DIYPGSGSTNYNEKFKS |
| | CDR-H2 | |
| 57 | GYCD84.1-226 | DTTIAY |
| | CDR-H3 | |
| 58 | GYCD84.1-226 | RASQSVTTSRYSYMH |
| | CDR-L1 | |
| 59 | GYCD84.1-226 | FASNLES |
| | CDR-L2 | |
| 60 | GYCD84.1-226 | QHSWEIPYT |
| | CDR-L3 | |
| 61 | 152-1D5 | QVQLQQPGAEVVQPGTSVKLSCKASGYNFTNYWINWVKLRPGQGLEWIGD IYPVSGTTNYNEKFKRKATLTVDTSSSTGNMQLSSLASEDSALYYCASGT GRFAYWGQGTLVTVSA |
| | VH | |
| 62 | 152-1D5 | DIVLTQSPASLAVSLGQRATISCRASQSVSTSSYSYMHWYQQKPGQPPKL LIKFASNLESGVPARFSGSGSGTDFTLNIHPVEEEDTATYYCQHSWEIPY TFGGGTKLEIK |
| | VL | |
| 63 | 153-4D9 | QVQLQQSGAELVRPGTSVKISCKASGYTFTNYWLGWVKQRPGHGLEWIGD IYPGGGYTNYIEKFKGKATLTADTSSSTAYMQLSSLTSEDSAVYFCARYE GGYYGNYDAMDYWGQGTSVTVSS |
| | VH | |
| 64 | 153-4D9 | DIVLTQSPGSLAVSLGQRATISCRASESVDNYGISFMNWFQQKPGQPPKL LIYAASNQGSGVPARFSGSGSGTDFSLNIHPMEEDDTAMYFCQQSKAVPR TFGGGTKLEIK |
| | VL | |
| 65 | R3-B3 | QVQLVESGGGLVQPGGSLRLSCAASGFTFSSYAMSWVRQAPGKGLEWVSA ISGSGGSTYYADSVKGRFTISRDNSKNTLYLQMNSLRAEDTAVYYCAKWD CSDGRCYWAYWGQGTLVTVSS |
| | VH | |
| 66 | R3-B3 | QPVLTQPPSVSVAPGQTARISCGGDNIESKDVHWYQRKSGQAPVLVVHDD ADRPTGIPDRFSGSNSGNTATLTISRVEAGDEADYYCQVWDSSSDHVVFG GGTQLTVL |
| | VL | |
| 67 | R3-B3 | GGGGSGGGGSGGGGSGGGGAS |
| | Linker | |
| 68 | R3-G7 | QVQLVESGGGVVQPGRSLRLSCTASGFTFSSYPMHWVRQAPGKGLEWVAA ISYHGRNKFYADSVKGRFTISRDDLRSTLYLQMNNLKADDTAVYSCARDR DATPGGTGVGNHGMAVWGQGTTVTVSS |
| | VH | |
| 69 | R3-G7 | EIVLTQSPLSLPVTPGEPASISCKSSQSLLHSSGYNYLDWYLQKPGQSPQ LLIHMGSNRASGVPARFSGSGSGTDFTLRISRVEAEDVGVYYCMQGLQTP PTFGGGTKLEIK |
| | VL | |
| 70 | R3-G7 | GGGGSGGGGSGGGGSGGGGAS |
| | Linker | |
| 71 | R3-H3 | EVQLVESGGGLVQPGGSLRLSCAASGFTFSDNAMSWVRQAPGKGLEWVSA ISGTGRTTYYADSVKGRFTISRDNSKNTLYLQMNSLRAEDTAVYYCAKWD CSDGRCYWAYWGQGTLVTVSS |
| | VH | |
| 72 | R3-H3 | EIVLTQSPLSLPVTLGQPASISCRSSQSLVYSDGDTYLNWFQQRPGQSPR RLIYKVSNRDSGVPDRFSGSGSGTDFTLKISRVEAEDVGVYYCMQGTHWP PNTFGQGTRLEIK |
| | VL | |
| 73 | R3-H3 | GGGGSGGGGSGGGGSGGGGAS |
| | Linker | |
| 74 | GYCD84.1-7 | QVTLKESGPGILQPSQTLSLTCSFSGFSLSTSGMGVGWIRQPSGKGLEWL AHIWWDDVKRYNPALKSRLTISKDTSSSQVFLKIASVDTADTATYYCARM RTSYYFDYWGQGTTLTVSS |
| | VH | |
| 75 | GYCD84.1-7 | DIQMTQSPASLSASVGETVTITCRASENIFSSLAWYQQRQGKSPQLLVYN AKTLAEGVPSRFSGSGSGTQFSLKINSLQPEDFGSYYCQHHYATPFTFGS GTKLEIK |
| | VL | |
| 76 | GYCD84.1-97 | QVQLQQPGAELVKPGTSVKLSCKASGYNFTSYWINWVKLRPGQGLEWIGD IYLGSGSTNYNEKFKSKATLTVDTSSSSAYMQLSSLVSEDSALYYCATSG GYLGYWGQGTTLTVSS |
| | VH | |
| 77 | GYCD84.1-97 | DIVLTQSPASLAVSLGQRATISCRASQSVSTSSYSYMHWYQQKPGQPPKL LIKFASNLESGVPARFSGSGSGTDFTLNIHPVEEEDTATYYCQHSWEIPY TFGGGTKLEIK |
| | VL | |
| 78 | GYCD84.1-108 | QVQLQQSGAELVRPGTSVKMSCKAAGYTFTNYWIGWVRQRPGHGLEWIGD IYPGGGYTNYNENFKGKATLTADTSSSTAYMQLSSLTSEDSAIYYCARST TYYSSSYWCFDVWGPGTTVTVSS |
| | VH | |
| 79 | GYCD84.1-108 | DIVMTQSPSSLSVSAGEKVTMSCKSSQSLLNSGNQANYLAWYQQKPGQPP KLLINGASTRESGVPDRFTGSGSGTDFTLTISSVQAEDLAVYYCQNDHSY PFTFGSGTKLEIK |
| | VL | |
| 80 | GYCD84.1-207 | EVKLLESGGGLVQPGGSLKLSCAASGFDFSRYWMSWVRQAPGKGLEWIGE INPDSSTINYTPSLKDKFIISRDNAKNTLYLQMSKVRSEDTALYYCARPG PTVVATYWYFDVWGAGTTVTVSS |
| | VH | |
| 81 | GYCD84.1-207 | DVLMTQTPLSLPVSLGDQASISCRSSQSIVHSNGNTYLEWYLQKPGQSPK LLIYKVSSRFSGVPDRFSGSASGTDFTLKISRVEAEDLGVYYCFQGSHVP RTFGGGTKLEIK |
| | VL | |
| 82 | GYCD84.1-226 | QVQLQQPGAELVKPGTSVKLSCKASGYNFTRYWINWVKLRPGQGLEWIGD IYPGSGSTNYNEKFKSKATLTVDTSSSTAYMQLSRLASEDSALYYCARDT TIAYWGQGTLVTVSA |
| | VH | |
| 83 | GYCD84.1-226 | DIVLTQSPTSLAVSLGQRATISCRASQSVTTSRYSYMHWYQQKPGQPPKL LIKFASNLESGVPARFSGSGSGTDFTLNIHPVEEEDTATYYCQHSWEIPY TFGGGTKLEIK |
| | VL | |
| 131 | GYCD84.2.32 | GYNMN |
| | CDR-H1 | |
| 132 | GYCD84.2.32 | NIDPYYGGTNYNQKFKG |
| | CDR-H2 | |
| 133 | GYCD84.2.32 CDR-H3 | GLLSGSFPY |
| 134 | GYCD84.2.32 | RASENIYSYLA |
| | CDR-L1 | |
| 135 | GYCD84.2.32 | NAKTLAE |
| | CDR-L2 | |
| 136 | GYCD84.2.32 | QHHYGSPLT |
| | CDR-L3 | |
| 137 | GYCD84.3.89 | RSWMS |
| | CDR-H1 | |
| 138 | GYCD84.3.89 | EINPDSSTINYTPSLKD |
| | CDR-H2 | |
| 139 | GYCD84.3.89 | FYDGYSIYWYFDV |
| | CDR-H3 | |
| 140 | GYCD84.3.89 | RSSQSIVHSNGDTYLE |
| | CDR-L1 | |
| 141 | GYCD84.3.89 | KVSNRFS |
| | CDR-L2 | |
| 142 | GYCD84.3.89 | FQGSHVPRT |
| | CDR-L3 | |
| 143 | GYCD84.3.29 | TSGMGVG |
| | 8 CDR-H1 | |
| 144 | GYCD84.3.29 | HIWWDDVKRYNPALRS |
| | 8 CDR-H2 | |
| 145 | GYCD84.3.29 | IAVTYFFDF |
| | 8 CDR-H3 | |
| 146 | GYCD84.3.29 | RASENIFSSFA |
| | 8 CDR-L1 | |
| 147 | GYCD84.3.29 | NARTLAE |
| | 8 CDR-L2 | |
| 148 | GYCD84.3.29 | QHHYASPFT |
| | 8 CDR-L3 | |
| 149 | GYCD84.2.27 CDR-H1 | TSGMGVG |
| 150 | GYCD84.2.27 | HIWWDDVKRYNPALKS |
| | CDR-H2 | |
| 151 | GYCD84.2.27 | MSTSYYFDY |
| | CDR-H3 | |
| 152 | GYCD84.2.27 | KASQSLFTSVA |
| | CDR-L1 | |
| 153 | GYCD84.2.27 | SASYRYT |
| | CDR-L2 | |
| 154 | GYCD84.2.27 | QQHYSSPFT |
| | CDR-L3 | |
| 155 | GYCD84.5.17 | IYAMN |
| | 1 CDR-H1 | |
| 156 | GYCD84.5.17 | RIRSKSNNYARFYADSVKD |
| | 1 CDR-H2 | |
| 157 | GYCD84.5.17 | PLRSYFSMDY |
| | 1 CDR-H3 | |
| 158 | GYCD84.5.17 | KASENVDTYVS |
| | 1 CDR-L1 | |
| 159 | GYCD84.5.17 | GASNRYT |
| | 1 CDR-L2 | |
| 160 | GYCD84.5.17 | GQTYSYPWT |
| | 1 CDR-L3 | |
| 161 | GYCD84.2.32 | EVQLQQSGPELEKPGASVKISCKASGYSFTGYNMNWVKQSNGKSLEWIGN IDPYYGGTNYNQKFKGKATLTVDKSSSTAYMQLKSLTSEDSAVYYCARGL LSGSFPYWGQGTLVTVSA |
| | VH | |
| 162 | GYCD84.2.32 | DIQMTQSPASLSASVGETVTITCRASENIYSYLAWYQQKQGKSPQLLVYN AKTLAEGVPSRFSGSGSGTQFSLKINSLHPEDFGSYFCQHHYGSPLTFGA GTKLEVK |
| | VL | |
| 163 | GYCD84.3.89 | EVKLLESGGGLVQPGGSLKLSCAASGFGFSRSWMSWVRQAPGKGLEWIGE INPDSSTINYTPSLKDKFIISRDNAKNTLFLQMSKVRSEDTALYYCATFY DGYSIYWYFDVWGAGTTVTVSS |
| | VH | |
| 164 | GYCD84.3.89 | DVLMTQTPLSLPVSLGDQASISCRSSQSIVHSNGDTYLEWYLQKPGQSPK LLIYKVSNRFSGVPDRFSGSGSGTDFTLKISRVEAEDLGVYYCFQGSHVP RTFGGGTKLEIK |
| | VL | |
| 165 | GYCD84.3.29 | QVTLKESGPGILQPSQTLSLTCSFSGFSLSTSGMGVGWIRQPSGKGLEWL AHIWWDDVKRYNPALRSRLTISKDTSSSQVFLNIASVDTADTATYYCTRI AVTYFFDFWGQGTTLTVSS |
| | 8 VH | |
| 166 | GYCD84.3.29 | DIQMTQSPASLSASVGETVTITCRASENIFSSFAWYQQKRGRSPQLLVYN ARTLAEGVPSRFSGSGSGTQFSLKINSLQPEDFGTYFCQHHYASPFTFGS GTELGVK |
| | 8 VL | |
| 167 | GYCD84.2.27 | QVTLKESGPGILQPSQTLSLTCSFSGFSLTTSGMGVGWIRQPSGKGLEWL AHIWWDDVKRYNPALKSRLTISKDTSSSQVFLKIASVDTADTASYYCARM STSYYFDYWGQGTTLTVSS |
| | VH | |
| 168 | GYCD84.2.27 | DIVMTQSHKFMSTSLGDRVSISCKASQSLFTSVAWYQQKPGQSPKLLIYS ASYRYTGVPDRFTGSGSGTDFTFTISSVQAEDLAVYYCQQHYSSPFTFGS GTKLGIK |
| | VL | |
| 169 | GYCD84.5.17 | EVQLVESGGGLVQPKGSLKLACAASGFTFNIYAMNWVRQAPGKGLEWVAR IRSKSNNYARFYADSVKDRFTISRDDSQSMLYLQMNNLKTEDTAMYYCVR PLRSYFSMDYWGQGTSVTVSS |
| | 1 VH | |
| 170 | GYCD84.5.17 | SIVMTQSPKSMSMSVGERVTLSCKASENVDTYVSWYQQKPEQSPKLLIYG ASNRYTGVPDRFTGSGSATDFTLTISSVKAEDLADYHCGQTYSYPWTFGG GTKLEIK |
| | 1 VL | |

In some embodiments, the antigen-binding domain comprises a heavy chain variable region (VH) having complementarity determining regions (CDRs) with the sequences: CDR1 - NYWIN (SEQ ID NO: 1); CDR2 - DIYPVSGTTNYNEKFKR (SEQ ID NO: 2); and CDR3 - GTGRFAY (SEQ ID NO: 3); and a light chain variable region (VL) having CDRs with the sequences: CDR1 - RASQSVSTSSYSYMH (SEQ ID NO: 4); CDR2 - FASNLES (SEQ ID NO: 5); and CDR3 - QHSWEIPYT (SEQ ID NO: 6).

In some embodiments, the antigen-binding domain comprises a heavy chain variable region (VH) having complementarity determining regions (CDRs) with the sequences: CDR1 - NYWLG (SEQ ID NO: 7); CDR2 - DIYPGGGYTNYIEKFKG (SEQ ID NO: 8); and CDR3 - YEGGYYGNYDAMDY (SEQ ID NO: 9); and a light chain variable region (VL) having CDRs with the sequences: CDR1 - RASESVDNYGISFMN (SEQ ID NO: 10); CDR2 - AASNQGS (SEQ ID NO: 11); and CDR3 - QQSKAVPRT (SEQ ID NO: 12).

In some embodiments, the antigen-binding domain comprises a heavy chain variable region (VH) having complementarity determining regions (CDRs) with the sequences: CDR1 - GFTFSSYP (SEQ ID NO: 19); CDR2 - ISYHGRNK (SEQ ID NO: 20); and CDR3 - ARDRDATPGGTGVGNHGMAV (SEQ ID NO: 21); and a light chain variable region (VL) having CDRs with the sequences: CDR1 - QSLLHSSGYNY (SEQ ID NO: 22); CDR2 - MGS (SEQ ID NO: 23); and CDR3 - MQGLQTPPT (SEQ ID NO: 24).

In some embodiments, the antigen-binding domain comprises a heavy chain variable region (VH) having complementarity determining regions (CDRs) with the sequences: CDR1 - GFTFSDNA (SEQ ID NO: 25); CDR2 - ISGTGRTT (SEQ ID NO: 26); and CDR3 - AKWDCSDGRCYWAY (SEQ ID NO: 27); and a light chain variable region (VL) having CDRs with the sequences: CDR1 - QSLVYSDGDTY (SEQ ID NO: 28); CDR2 - KVS (SEQ ID NO: 29); and CDR3 - MQGTHWPPNT (SEQ ID NO: 30).

In some embodiments, the antigen-binding domain comprises a VH domain having the sequence of SEQ ID NO: 61; and a VL domain having the sequence of SEQ ID NO: 62 or 108, or variants thereof each having at least 90%, at least 95%, at least 96%, at least 97%, at least 98% or at least 99%, preferably at least 90%, sequence identity thereto.

In some embodiments, the antigen-binding domain comprises a VH domain having the sequence of SEQ ID NO: 63; and a VL domain having the sequence of SEQ ID NO: 64 or 109, or variants thereof each having at least 90%, at least 95%, at least 96%, at least 97%, at least 98% or at least 99%, preferably at least 90%, sequence identity thereto.

In some embodiments, the antigen-binding domain comprises a VH domain having the sequence of SEQ ID NO: 68; and a VL domain having the sequence of SEQ ID NO: 69, or variants thereof each having at least 90%, at least 95%, at least 96%, at least 97%, at least 98% or at least 99%, preferably at least 90%, sequence identity thereto.

In some embodiments, the antigen-binding domain comprises a VH domain having the sequence of SEQ ID NO: 71; and a VL domain having the sequence of SEQ ID NO: 72, or variants thereof each having at least 90%, at least 95%, at least 96%, at least 97%, at least 98% or at least 99%, preferably at least 90%, sequence identity thereto.

The CD84-binding domain may be a scFv. A scFv may comprise the heavy variable region (VH) and light chain variable region (VL) of an antibody, connected with a short linker peptide, for example of about 10 to 25 amino acids. The scFv may be in the orientation (from N- to C-terminus) VH-VL or VL-VH. In some embodiments, the scFv is in the orientation (from N- to C-terminus) VH-VL. In some embodiments, the scFv is in the orientation (from N- to C-terminus) VL-VH.

Example linker sequences for connecting VH and VL domains include:
GGGGSGGGGSGGGGSGGGGAS (SEQ ID NO: 67)
GGGGSGGGGSGGGGS (SEQ ID NO: 110)
GGGGSGGGGSGGGGSGGGGS (SEQ ID NO: 111)

The antigen-binding domain may comprise or consist of the sequence of any one of SEQ ID NOs: 61, 63, 112-119 or 122-125, or a variant thereof having at least 75%, at least 80%, at least 85%, at least 90%, at least 95%, at least 96%, at least 97%, at least 98% or at least 99%, preferably at least 90%, sequence identity thereto.

Suitably, the variant may bind to CD84 at least as well as the corresponding antigen-binding domain shown as SEQ ID NO: 61, 63, 112-119 or 122-125. For example, the variant may specifically bind to CD84 with a binding affinity which is at least equivalent to the binding affinity between the corresponding antigen-binding domain shown as SEQ ID NO: 61, 63, 112-119 or 122-125 and CD84.

| **Antigen-binding domain** | **Sequence** |
|---|---|
| **152.1** (VH) | QVQLQQPGAEVVQPGTSVKLSCKASGYNFTNYWINWVKLRPGQGL EWIGDIYPVSGTTNYNEKFKRKATLTVDTSSSTGNMQLSSLASED SALYYCASGTGRFAYWGQGTLVTVSA (SEQ ID NO: 61) |
| **152.2** (VLS3VH) | DIVLTQSPASLAVSLGQRATISCRASQSVSTSSYSYMHWYQQKPG QPPKLLIKFASNLESGVPARFSGSGSGTDFTLNIHPVEEEDTATY |
| | YCQHSWEIPYTFGGGTKLEIKRAGGGGSGGGGSGGGGSQVQLQQP GAEVVQPGTSVKLSCKASGYNFTNYWINWVKLRPGQGLEWIGDIY PVSGTTNYNEKFKRKATLTVDTSSSTGNMQLSSLASEDSALYYCA SGTGRFAYWGQGTLVTVSA (SEQ ID NO: 112) |
| **152.3** (VLS4VH) | DIVLTQSPASLAVSLGQRATISCRASQSVSTSSYSYMHWYQQKPG QPPKLLIKFASNLESGVPARFSGSGSGTDFTLNIHPVEEEDTATY YCQHSWEIPYTFGGGTKLEIKRAGGGGSGGGGSGGGGSGGGGSQV QLQQPGAEVVQPGTSVKLSCKASGYNFTNYWINWVKLRPGQGLEW IGDIYPVSGTTNYNEKFKRKATLTVDTSSSTGNMQLSSLASEDSA LYYCASGTGRFAYWGQGTLVTVSA (SEQ ID NO: 113) |
| **152.4** (VHS3VL) | QVQLQQPGAEVVQPGTSVKLSCKASGYNFTNYWINWVKLRPGQGL EWIGDIYPVSGTTNYNEKFKRKATLTVDTSSSTGNMQLSSLASED SALYYCASGTGRFAYWGQGTLVTVSAGGGGSGGGGSGGGGSDIVL TQSPASLAVSLGQRATISCRASQSVSTSSYSYMHWYQQKPGQPPK LLIKFASNLESGVPARFSGSGSGTDFTLNIHPVEEEDTATYYCQH SWEIPYTFGGGTKLEIKRA (SEQ ID NO: 114) |
| **152.5** (VHS4VL) | QVQLQQPGAEVVQPGTSVKLSCKASGYNFTNYWINWVKLRPGQGL EWIGDIYPVSGTTNYNEKFKRKATLTVDTSSSTGNMQLSSLASED SALYYCASGTGRFAYWGQGTLVTVSAGGGGSGGGGSGGGGSGGGG SDIVLTQSPASLAVSLGQRATISCRASQSVSTSSYSYMHWYQQKP GQPPKLLIKFASNLESGVPARFSGSGSGTDFTLNIHPVEEEDTAT YYCQHSWEIPYTFGGGTKLEIKRA (SEQ ID NO: 115) |
| **153.1** (VH) | QVQLQQSGAELVRPGTSVKISCKASGYTFTNYWLGWVKQRPGHGL EWIGDIYPGGGYTNYIEKFKGKATLTADTSSSTAYMQLSSLTSED SAVYFCARYEGGYYGNYDAMDYWGQGTSVTVSS (SEQ ID NO: 63) |
| **153.2** (VLS3VH) | DIVLTQSPGSLAVSLGQRATISCRASESVDNYGISFMNWFQQKPG QPPKLLIYAASNQGSGVPARFSGSGSGTDFSLNIHPMEEDDTAMY FCQQSKAVPRTFGGGTKLEIKRAGGGGSGGGGSGGGGSQVQLQQS GAELVRPGTSVKISCKASGYTFTNYWLGWVKQRPGHGLEWIGDIY PGGGYTNYIEKFKGKATLTADTSSSTAYMQLSSLTSEDSAVYFCA RYEGGYYGNYDAMDYWGQGTSVTVSS (SEQ ID NO: 116) |
| **153.3** (VLS4VH) | DIVLTQSPGSLAVSLGQRATISCRASESVDNYGISFMNWFQQKPG QPPKLLIYAASNQGSGVPARFSGSGSGTDFSLNIHPMEEDDTAMY FCQQSKAVPRTFGGGTKLEIKRAGGGGSGGGGSGGGGSGGGGSQV QLQQSGAELVRPGTSVKISCKASGYTFTNYWLGWVKQRPGHGLEW IGDIYPGGGYTNYIEKFKGKATLTADTSSSTAYMQLSSLTSEDSA VYFCARYEGGYYGNYDAMDYWGQGTSVTVSS (SEQ ID NO: 117) |
| **153.4** (VHS3VL) | QVQLQQSGAELVRPGTSVKISCKASGYTFTNYWLGWVKQRPGHGL EWIGDIYPGGGYTNYIEKFKGKATLTADTSSSTAYMQLSSLTSED SAVYFCARYEGGYYGNYDAMDYWGQGTSVTVSSGGGGSGGGGSGG GGSDIVLTQSPGSLAVSLGQRATISCRASESVDNYGISFMNWFQQ KPGQPPKLLIYAASNQGSGVPARFSGSGSGTDFSLNIHPMEEDDT AMYFCQQSKAVPRTFGGGTKLEIKRA (SEQ ID NO: 118) |
| **153.5** (VHS4VL) | QVQLQQSGAELVRPGTSVKISCKASGYTFTNYWLGWVKQRPGHGL EWIGDIYPGGGYTNYIEKFKGKATLTADTSSSTAYMQLSSLTSED SAVYFCARYEGGYYGNYDAMDYWGQGTSVTVSSGGGGSGGGGSGG GGSGGGGSDIVLTQSPGSLAVSLGQRATISCRASESVDNYGISFM NWFQQKPGQPPKLLIYAASNQGSGVPARFSGSGSGTDFSLNIHPM |
| | EEDDTAMYFCQQSKAVPRTFGGGTKLEIKRA (SEQ ID NO: 119) |
| **B3.4** (VHS3VL) | QVQLVESGGGLVQPGGSLRLSCAASGFTFSSYAMSWVRQAPGKGL EWVSAISGSGGSTYYADSVKGRFTISRDNSKNTLYLQMNSLRAED TAVYYCAKWDCSDGRCYWAYWGQGTLVTVSSGGGGSGGGGSGGGG SQPVLTQPPSVSVAPGQTARISCGGDNIESKDVHWYQRKSGQAPV LVVHDDADRPTGIPDRFSGSNSGNTATLTISRVEAGDEADYYCQV WDSSSDHVVFGGGTQLTVL (SEQ ID NO: 120) |
| **B3.5** (VHS4VL) | QVQLVESGGGLVQPGGSLRLSCAASGFTFSSYAMSWVRQAPGKGL EWVSAISGSGGSTYYADSVKGRFTISRDNSKNTLYLQMNSLRAED TAVYYCAKWDCSDGRCYWAYWGQGTLVTVSSGGGGSGGGGSGGGG SGGGGSQPVLTQPPSVSVAPGQTARISCGGDNIESKDVHWYQRKS GQAPVLVVHDDADRPTGIPDRFSGSNSGNTATLTISRVEAGDEAD YYCQVWDSSSDHVVFGGGTQLTVL (SEQ ID NO: 121) |
| **G7.4** (VHS3VL) | QVQLVESGGGVVQPGRSLRLSCTASGFTFSSYPMHWVRQAPGKGL EWVAAISYHGRNKFYADSVKGRFTISRDDLRSTLYLQMNNLKADD TAVYSCARDRDATPGGTGVGNHGMAVWGQGTTVTVSSGGGGSGGG GSGGGGSEIVLTQSPLSLPVTPGEPASISCKSSQSLLHSSGYNYL DWYLQKPGQSPQLLIHMGSNRASGVPARFSGSGSGTDFTLRISRV EAEDVGVYYCMQGLQTPPTFGGGTKLEIK (SEQ ID NO: 122) |
| **G7.5** (VHS4VL) | QVQLVESGGGVVQPGRSLRLSCTASGFTFSSYPMHWVRQAPGKGL EWVAAISYHGRNKFYADSVKGRFTISRDDLRSTLYLQMNNLKADD TAVYSCARDRDATPGGTGVGNHGMAVWGQGTTVTVSSGGGGSGGG GSGGGGSGGGGSEIVLTQSPLSLPVTPGEPASISCKSSQSLLHSS GYNYLDWYLQKPGQSPQLLIHMGSNRASGVPARFSGSGSGTDFTL RISRVEAEDVGVYYCMQGLQTPPTFGGGTKLEIK (SEQ ID NO: 123) |
| **H3.4** (VHS3VL) | EVQLVESGGGLVQPGGSLRLSCAASGFTFSDNAMSWVRQAPGKGL EWVSAISGTGRTTYYADSVKGRFTISRDNSKNTLYLQMNSLRAED TAVYYCAKWDCSDGRCYWAYWGQGTLVTVSSGGGGSGGGGSGGGG SEIVLTQSPLSLPVTLGQPASISCRSSQSLVYSDGDTYLNWFQQR PGQSPRRLIYKVSNRDSGVPDRFSGSGSGTDFTLKISRVEAEDVG VYYCMQGTHWPPNTFGQGTRLEIK (SEQ ID NO: 124) |
| **H3.5** (VHS4VL) | EVQLVESGGGLVQPGGSLRLSCAASGFTFSDNAMSWVRQAPGKGL EWVSAISGTGRTTYYADSVKGRFTISRDNSKNTLYLQMNSLRAED TAVYYCAKWDCSDGRCYWAYWGQGTLVTVSSGGGGSGGGGSGGGG SGGGGSEIVLTQSPLSLPVTLGQPASISCRSSQSLVYSDGDTYLN WFQQRPGQSPRRLIYKVSNRDSGVPDRFSGSGSGTDFTLKISRVE AEDVGVYYCMQGTHWPPNTFGQGTRLEIK (SEQ ID NO: 125) |
| **1.7.3** (VLS4VH) | DIQMTQSPASLSASVGETVTITCRASENIFSSLAWYQQRQGKSPQ LLVYNAKTLAEGVPSRFSGSGSGTQFSLKINSLQPEDFGSYYCQH HYATPFTFGSGTKLEIKGGGGSGGGGSGGGGSGGGGSQVTLKESG PGILQPSQTLSLTCSFSGFSLSTSGMGVGWIRQPSGKGLEWLAHI WWDDVKRYNPALKSRLTISKDTSSSQVFLKIASVDTADTATYYCA RMRTSYYFDYWGQGTTLTVSS (SEQ ID NO: 181) |
| **1.7.5** (VHS4VL) | QVTLKESGPGILQPSQTLSLTCSFSGFSLSTSGMGVGWIRQPSGK GLEWLAHIWWDDVKRYNPALKSRLTISKDTSSSQVFLKIASVDTA DTATYYCARMRTSYYFDYWGQGTTLTVSSGGGGSGGGGSGGGGSG GGGSDIQMTQSPASLSASVGETVTITCRASENIFSSLAWYQQRQG KSPQLLVYNAKTLAEGVPSRFSGSGSGTQFSLKINSLQPEDFGSY YCQHHYATPFTFGSGTKLEIK (SEQ ID NO: 182) |
| **1.226.5** (VHS4VL) | QVQLQQPGAELVKPGTSVKLSCKASGYNFTRYWINWVKLRPGQGL EWIGDIYPGSGSTNYNEKFKSKATLTVDTSSSTAYMQLSRLASED SALYYCARDTTIAYWGQGTLVTVSAGGGGSGGGGSGGGGSGGGGS DIVLTQSPTSLAVSLGQRATISCRASQSVTTSRYSYMHWYQQKPG |
| | QPPKLLIKFASNLESGVPARFSGSGSGTDFTLNIHPVEEEDTATY YCQHSWEIPYTFGGGTKLEIK (SEQ ID NO: 183) |
| **3.89.5** (VHS4VL) | EVKLLESGGGLVQPGGSLKLSCAASGFGFSRSWMSWVRQAPGKGL EWIGEINPDSSTINYTPSLKDKFIISRDNAKNTLFLQMSKVRSED TALYYCATFYDGYSIYWYFDVWGAGTTVTVSSGGGGSGGGGSGGG GSGGGGSDVLMTQTPLSLPVSLGDQASISCRSSQSIVHSNGDTYL EWYLQKPGQSPKLLIYKVSNRFSGVPDRFSGSGSGTDFTLKISRV EAEDLGVYYCFQGSHVPRTFGGGTKLEIK (SEQ ID NO: 184) |

In some embodiments, the antigen-binding domain comprises or consists of the sequence of SEQ ID NO: 61, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 63, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 112, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 113, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 114, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 115, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 116, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 117, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 118, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 119, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 122, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 123, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 124, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

In some embodiments, the antigen-binding domain comprises or consists of the sequence of SEQ ID NO: 125, or a variant thereof having at least 75%, at least 80%, at least 85%, at least 90%, at least 95%, at least 96%, at least 97%, at least 98% or at least 99%, preferably at least 90%, sequence identity thereto, and preferably retaining the ability to bind to CD84.

### Chimeric antigen receptor (CAR)

"Chimeric antigen receptor" (CAR or CARs) as used herein refers to engineered receptors which can confer an antigen specificity onto cells (for example, T cells, such as naive T cells, central memory T cells, effector memory T cells or combinations thereof). CARs are also known as artificial T-cell receptors, chimeric T-cell receptors or chimeric immunoreceptors. CARs can also confer an antigen specificity onto other immune cells such as NK cells (e.g. from cord blood (CB), induced pluripotent stem cells (iPSCs), bone marrow (BM), human embryonic stem cells (hESCs), a cell line (e.g. NK92 or YT), or peripheral blood (PB) (Mehta et al. (2018) Front. Immunol. 9: 283; Liu et al. (2020) N. Engl. J. Med. 382: 545-553). CARs used in NK cells may have other transmembrane domains (such as NKG2D or DAP12) and other co-stimulatory domains (such as NKG2D or 2B4) and they may incorporate genes for IL-2 or IL-15 within the CAR construct to constantly provide cytokine support to the CAR-NK cells.

CARs may, for example, comprise an antigen-binding domain, a transmembrane domain and an intracellular signaling domain (endodomain).

In some embodiments, the CAR comprises a CD84-binding domain, a transmembrane domain and an intracellular signaling domain. The CAR may comprise one or more co-stimulatory domain.

The antigen-binding domain (e.g. CD84-binding domain) of the CAR may be an antigen-binding domain as disclosed herein.

In another aspect, the invention provides a chimeric antigen receptor (CAR) comprising or consisting of the sequence of any one of SEQ ID NOs: 172-176, or a variant thereof having at least 75%, at least 80%, at least 85%, at least 90%, at least 95%, at least 96%, at least 97%, at least 98% or at least 99%, preferably at least 90%, sequence identity thereto.

Suitably, the variant may function at least as well as the corresponding CAR shown as SEQ ID NO: 172-176. For example, the variant may specifically bind to CD84 with a binding affinity which is at least equivalent to the binding affinity between the corresponding CAR shown as SEQ ID NO: 172-176 and CD84.

In another aspect, the invention provides a chimeric antigen receptor (CAR) comprising or consisting of the sequence of SEQ ID NO: 172, or a variant thereof having at least 75%, at least 80%, at least 85%, at least 90%, at least 95%, at least 96%, at least 97%, at least 98% or at least 99%, preferably at least 90%, sequence identity thereto.

In another aspect, the invention provides a chimeric antigen receptor (CAR) comprising or consisting of the sequence of SEQ ID NO: 173, or a variant thereof having at least 75%, at least 80%, at least 85%, at least 90%, at least 95%, at least 96%, at least 97%, at least 98% or at least 99%, preferably at least 90%, sequence identity thereto.

In another aspect, the invention provides a chimeric antigen receptor (CAR) comprising or consisting of the sequence of SEQ ID NO: 174, or a variant thereof having at least 75%, at least 80%, at least 85%, at least 90%, at least 95%, at least 96%, at least 97%, at least 98% or at least 99%, preferably at least 90%, sequence identity thereto.

In another aspect, the invention provides a chimeric antigen receptor (CAR) comprising or consisting of the sequence of SEQ ID NO: 175, or a variant thereof having at least 75%, at least 80%, at least 85%, at least 90%, at least 95%, at least 96%, at least 97%, at least 98% or at least 99%, preferably at least 90%, sequence identity thereto.

In another aspect, the invention provides a chimeric antigen receptor (CAR) comprising or consisting of the sequence of SEQ ID NO: 176, or a variant thereof having at least 75%, at least 80%, at least 85%, at least 90%, at least 95%, at least 96%, at least 97%, at least 98% or at least 99%, preferably at least 90%, sequence identity thereto.

### Transmembrane domain

The CAR may comprise a transmembrane domain.

The transmembrane domain may comprise the transmembrane sequence from any protein which has a transmembrane domain, including any of the type I, type II or type III transmembrane proteins. The transmembrane domain of the CAR may also comprise an artificial hydrophobic sequence. The transmembrane domains of the CAR may be selected so as not to dimerise.

Examples of transmembrane (TM) regions used in CAR constructs are: a) the CD28 TM region (Pulè et al., Mol Ther, 2005, Nov;12(5):933-41; Brentjens et al., CCR, 2007, Sep 15;13(18 Pt 1):5426-35; Casucci et al., Blood, 2013, Nov 14;122(20):3461-72.); b) the OX40 TM region (Pulè et al., Mol Ther, 2005, Nov;12(5):933-41); c) the 4-1BB TM region (Brentjens et al, CCR, 2007, Sep 15;13 (18 Pt 1):5426-35); d) the CD3-zeta TM region (Pulè et al., Mol Ther, 2005, Nov;12(5):933-41; Di Stasi et al. Blood, 2009, Jun 18;113(25):6392-402.); e) the CD8a **TM** region (Maher et al., Nat Biotechnol, 2002, Jan;20(1):70-5.; Imai C et al., Leukemia, 2004, Apr;18(4):676-84; Brentjens et al., CCR, 2007, Sep 15;13(18 Pt 1):5426-35; Milone et al., Mol Ther, 2009, Aug;17(8):1453-64.); f) the DAP12 TM region (Müller, N. et al. J. Immunother. 2015, June 01; 38, 197); g) the 2B4 TM region (Altvater, B. et al. Clin. Cancer Res, 2009, July 22; (15) 4857-4866) and h) the NKG2D TM region (Li, Y et al, Cell Stem Cell, 2018, Aug 2; (23):181-192).

Additional transmembrane domains will be apparent to those of skill in the art.

An example amino acid sequence of a CD8a transmembrane domain is:
IYIWAPLAGTCGVLLLSLVITLYC
(SEQ ID NO: 127)

An example amino acid sequence of a CD28 transmembrane domain is:
FWVLVVVGGVLACYSLLVTVAFIIFWV
(SEQ ID NO: 128)

In some embodiments, the transmembrane domain comprises or consists of the sequence of SEQ ID NO: 127 or 128, or a variant thereof having at least 75%, at least 80%, at least 85%, at least 90%, at least 95%, at least 96%, at least 97%, at least 98% or at least 99%, preferably at least 90%, sequence identity thereto.

### Signal peptide

The CAR may comprise a signal peptide such that when the CAR is expressed in a cell the protein is directed to the endoplasmic reticulum and subsequently to the cell surface.

Signal peptides may be recognised and cleaved by a signal peptidase during or after translocation to generate a mature protein.

In some embodiments, the signal peptide is a CD8a signal peptide.

### Spacer

The CAR may comprise a spacer that joins the antigen-binding domain to the transmembrane domain.

The spacer sequence may, for example, comprise an IgG1 Fc region, an IgG1 hinge or a human or mouse CD8 stalk.

In some embodiments, the CAR comprises a CD8a stalk.

### Intracellular signalling domain

The intracellular domain may provide for signal-transmission in a CAR.

The intracellular signalling domain may comprise one or more immunoreceptor tyrosine-based activation motif (ITAM), which is a conserved sequence of four amino acids repeated twice in the cytoplasmic tails of certain cell-surface proteins of the immune system. The motif contains a tyrosine separated from a leucine or isoleucine by any two other amino acids (YxxL/I). The tyrosine residues of these motifs may be phosphorylated following interaction of the receptor molecules with their ligands and may form binding sites for other proteins involved in signalling pathways.

The intracellular signalling domain may comprise or consist of the CD3-zeta endodomain, which contains three ITAMs. The CD3-zeta endodomain may transmit an activation signal to the T cell after the antigen is bound.

The CAR may comprise one or more co-stimulatory domain. For example, 4-1BB (also known as CD137) can be used with CD3-zeta, or CD28, OX40 and/or ICOS can be used with CD3-zeta to transmit a proliferative / survival signal. Also NKG2D, 2B4 (also known as CD244), DAP12 and/or DAP10 can be used with CD3-zeta to transmit a proliferative / survival signal.

In some embodiments, the CAR comprises a CD3-zeta signalling domain and lacks any co-stimulatory domains. In some embodiments, the CAR comprises a CD3-zeta signalling domain and one or more co-stimulatory domain.

In some embodiments, the CAR comprises one or more co-stimulatory domains selected from the group consisting of a 4-1BB co-stimulatory domain, a CD28 co-stimulatory domain and an OX40 co-stimulatory domain.

In preferred embodiments, the CAR comprises a 4-1BB co-stimulatory domain. In some embodiments, the CAR comprises a CD28 co-stimulatory domain. In some embodiments, the CAR comprises a OX40 co-stimulatory domain.

In preferred embodiments, the CAR comprises a 4-1BB co-stimulatory domain and a CD3-zeta signalling domain.

An example amino acid sequence of a CD3-zeta signalling domain is:

RVKFSRSADAPAYQQGQNQLYNELNLGRREEYDVLDKRRGRDPEMGGKPQRRKNPQEGLYNE
LQKDKMAEAYSEIGMKGERRRGKGHDGLYQGLSTATKDTYDALHMQALPPR
(SEQ ID NO: 129)

In some embodiments, the signalling domain comprises or consists of the sequence of SEQ ID NO: 129, or a variant thereof having at least 75%, at least 80%, at least 85%, at least 90%, at least 95%, at least 96%, at least 97%, at least 98% or at least 99%, preferably at least 90%, sequence identity thereto.

An example amino acid sequence of a 4-1BB co-stimulatory domain is:
KRGRKKLLYIFKQPFMRPVQTTQEEDGCSCRFPEEEEGGCEL
(SEQ ID NO: 130)

An example amino acid sequence of a CD28 co-stimulatory domain is:
RSKRSRLLHSDYMNMTPRRPGPTRKHQYPYAPPRDFAAYRS
(SEQ ID NO: 171)

In some embodiments, the co-stimulatory domain comprises or consists of the sequence of SEQ ID NO: 130 or 171, or a variant thereof having at least 75%, at least 80%, at least 85%, at least 90%, at least 95%, at least 96%, at least 97%, at least 98% or at least 99%, preferably at least 90%, sequence identity thereto.

### Polynucleotide

Polynucleotides of the invention may comprise DNA or RNA, preferably DNA. They may be single-stranded or double-stranded. Preferably the polynucleotides are isolated polynucleotides. It will be understood by a skilled person that numerous different polynucleotides can encode the same polypeptide as a result of the degeneracy of the genetic code. In addition, it is to be understood that skilled persons may, using routine techniques, make nucleotide substitutions that do not affect the polypeptide sequence encoded by the polynucleotides of the invention to reflect the codon usage of any particular host organism in which the polypeptides of the invention are to be expressed.

The polynucleotides may be modified by any method available in the art. Such modifications may be carried out in order to enhance the *in vivo* activity or lifespan of the polynucleotides of the invention.

Polynucleotides such as DNA polynucleotides may be produced recombinantly, synthetically or by any means available to those of skill in the art. They may also be cloned by standard techniques.

Longer polynucleotides will generally be produced using recombinant means, for example using polymerase chain reaction (PCR) cloning techniques. This will involve making a pair of primers (e.g. of about 15 to 30 nucleotides) flanking the target sequence which it is desired to clone, bringing the primers into contact with mRNA or cDNA obtained from an animal or human cell, performing a PCR under conditions which bring about amplification of the desired region, isolating the amplified fragment (e.g. by purifying the reaction mixture with an agarose gel) and recovering the amplified DNA. The primers may be designed to contain suitable restriction enzyme recognition sites so that the amplified DNA can be cloned into a suitable vector.

The polynucleotide may comprise a promoter and/or enhancer operably linked to the one or more nucleotide sequence encoding the antigen-binding domain, antibody or CAR of the invention. The term "operably linked", as used herein, may mean that two components are linked together in a manner, which enables both to carry out their function substantially unhindered. For example, the promoter and/or enhancer may facilitate and/or enhance expression of the antigen-binding domain, antibody or CAR.

In some embodiments, the promoter is a EF1α promoter.

### Vectors

In some embodiments, the polynucleotide is a vector.

Preferably the vector is a viral vector, such as a retroviral vector, lentiviral vector, adeno-associated viral (AAV) vector or adenoviral vector. In some embodiments the polynucleotide is a viral genome.

In another aspect the invention provides a viral vector comprising the polynucleotide of the invention.

In some embodiments the viral vector is in the form of a viral vector particle.

A vector is a tool that allows or facilitates the transfer of an entity from one environment to another. In accordance with the invention, and by way of example, some vectors used in recombinant nucleic acid techniques allow entities, such as a segment of nucleic acid (e.g. a heterologous DNA segment, such as a heterologous cDNA segment), to be transferred into a target cell. The vector may serve the purpose of maintaining the heterologous nucleic acid (DNA or RNA) within the cell, facilitating the replication of the vector comprising a segment of nucleic acid and/or facilitating the expression of the protein encoded by a segment of nucleic acid.

Vectors comprising polynucleotides used in the invention may be introduced into cells using a variety of techniques known in the art, such as transfection, transduction and transformation.

Transfection may refer to a general process of incorporating a nucleic acid into a cell and includes a process using a non-viral vector to deliver a polynucleotide to a cell. Transduction may refer to a process of incorporating a nucleic acid into a cell using a viral vector.

### Retroviral and lentiviral vectors

A retroviral vector may be derived from or may be derivable from any suitable retrovirus. A large number of different retroviruses have been identified. Examples include murine leukaemia virus (MLV), human T-cell leukaemia virus (HTLV), mouse mammary tumour virus (MMTV), Rous sarcoma virus (RSV), Fujinami sarcoma virus (FuSV), Moloney murine leukaemia virus (Mo-MLV), FBR murine osteosarcoma virus (FBR MSV), Moloney murine sarcoma virus (Mo-MSV), Abelson murine leukaemia virus (A-MLV), avian myelocytomatosis virus-29 (MC29) and avian erythroblastosis virus (AEV). A detailed list of retroviruses may be found in Coffin, J.M. et al. (1997) Retroviruses, Cold Spring Harbour Laboratory Press, 758-63.

Retroviruses may be broadly divided into two categories, "simple" and "complex". Retroviruses may be even further divided into seven groups. Five of these groups represent retroviruses with oncogenic potential. The remaining two groups are the lentiviruses and the spumaviruses.

The basic structures of retrovirus and lentivirus genomes share many common features such as a 5' LTR and a 3' LTR. Between or within these are located a packaging signal to enable the genome to be packaged, a primer-binding site, integration sites to enable integration into a host cell genome, and gag, pol and env genes encoding the packaging components - these are polypeptides required for the assembly of viral particles. Lentiviruses have additional features, such as rev and RRE sequences in HIV, which enable the efficient export of RNA transcripts of the integrated provirus from the nucleus to the cytoplasm of an infected target cell.

In the provirus, these genes are flanked at both ends by regions called long terminal repeats (LTRs). The LTRs are responsible for proviral integration and transcription. LTRs also serve as enhancer-promoter sequences and can control the expression of the viral genes.

The LTRs themselves are identical sequences that can be divided into three elements: U3, R and U5. U3 is derived from the sequence unique to the 3' end of the RNA. R is derived from a sequence repeated at both ends of the RNA. U5 is derived from the sequence unique to the 5' end of the RNA. The sizes of the three elements can vary considerably among different retroviruses.

In a defective retroviral vector genome gag, pol and env may be absent or not functional.

In a typical retroviral vector, at least part of one or more protein coding regions essential for replication may be removed from the virus. This makes the viral vector replication-defective.

Lentivirus vectors are part of the larger group of retroviral vectors. A detailed list of lentiviruses may be found in Coffin, J.M. et al. (1997) Retroviruses, Cold Spring Harbour Laboratory Press, 758-63. In brief, lentiviruses can be divided into primate and non-primate groups. Examples of primate lentiviruses include but are not limited to human immunodeficiency virus (HIV), the causative agent of human acquired immunodeficiency syndrome (AIDS); and simian immunodeficiency virus (SIV). Examples of non-primate lentiviruses include the prototype "slow virus" visna/maedi virus (VMV), as well as the related caprine arthritis-encephalitis virus (CAEV), equine infectious anaemia virus (EIAV), and the more recently described feline immunodeficiency virus (FIV) and bovine immunodeficiency virus (BIV).

The lentivirus family differs from retroviruses in that lentiviruses have the capability to infect both dividing and non-dividing cells (Lewis, P et al. (1992) EMBO J. 11: 3053-8; Lewis, P.F. et al. (1994) J. Virol. 68: 510-6). In contrast, other retroviruses, such as MLV, are unable to infect non-dividing or slowly dividing cells such as those that make up, for example, muscle, brain, lung and liver tissue.

A lentiviral vector, as used herein, is a vector, which comprises at least one component part derivable from a lentivirus. Preferably, that component part is involved in the biological mechanisms by which the vector infects cells, expresses genes or is replicated.

The lentiviral vector may be a "primate" vector. The lentiviral vector may be a "non-primate" vector (i.e. derived from a virus which does not primarily infect primates, especially humans). Examples of non-primate lentiviruses may be any member of the family of lentiviridae, which does not naturally infect a primate.

As examples of lentivirus-based vectors, HIV-1- and HIV-2-based vectors are described below.

The HIV-1 vector contains cis-acting elements that are also found in simple retroviruses. It has been shown that sequences that extend into the gag open reading frame are important for packaging of HIV-1. Therefore, HIV-1 vectors often contain the relevant portion of gag in which the translational initiation codon has been mutated. In addition, most HIV-1 vectors also contain a portion of the env gene that includes the RRE. Rev binds to RRE, which permits the transport of full-length or singly spliced mRNAs from the nucleus to the cytoplasm. In the absence of Rev and/or RRE, full-length HIV-1 RNAs accumulate in the nucleus. Alternatively, a constitutive transport element from certain simple retroviruses such as Mason-Pfizer monkey virus can be used to relieve the requirement for Rev and RRE. Efficient transcription from the HIV-1 LTR promoter requires the viral protein Tat.

Most HIV-2-based vectors are structurally very similar to HIV-1 vectors. Similar to HIV-1-based vectors, HIV-2 vectors also require RRE for efficient transport of the full-length or singly spliced viral RNAs.

Preferably, the viral vector used in the present invention has a minimal viral genome.

By "minimal viral genome" it is to be understood that the viral vector has been manipulated so as to remove the non-essential elements and to retain the essential elements in order to provide the required functionality to infect, transduce and deliver a nucleotide sequence of interest to a target host cell. Further details of this strategy can be found in WO 1998/017815.

Preferably, the plasmid vector used to produce the viral genome within a host cell/packaging cell will have sufficient lentiviral genetic information to allow packaging of an RNA genome, in the presence of packaging components, into a viral particle which is capable of infecting a target cell, but is incapable of independent replication to produce infectious viral particles within the final target cell. Preferably, the vector lacks a functional gag-pol and/or env gene and/or other genes essential for replication.

However, the plasmid vector used to produce the viral genome within a host cell/packaging cell will also include transcriptional regulatory control sequences operably linked to the lentiviral genome to direct transcription of the genome in a host cell/packaging cell. These regulatory sequences may be the natural sequences associated with the transcribed viral sequence (i.e. the 5' U3 region), or they may be a heterologous promoter, such as another viral promoter (e.g. the CMV promoter).

The vectors may be self-inactivating (SIN) vectors in which the viral enhancer and promoter sequences have been deleted. SIN vectors can be generated and transduce non-dividing cells *in vivo* with an efficacy similar to that of wild-type vectors. The transcriptional inactivation of the long terminal repeat (LTR) in the SIN provirus should prevent mobilisation by replication-competent virus. This should also enable the regulated expression of genes from internal promoters by eliminating any cis-acting effects of the LTR.

The vectors may be integration-defective. Integration defective lentiviral vectors (IDLVs) can be produced, for example, either by packaging the vector with catalytically inactive integrase (such as an HIV integrase bearing the D64V mutation in the catalytic site; Naldini, L. et al. (1996) Science 272: 263-7; Naldini, L. et al. (1996) Proc. Natl. Acad. Sci. USA 93: 11382-8; Leavitt, A.D. et al. (1996) J. Virol. 70: 721-8) or by modifying or deleting essential att sequences from the vector LTR (Nightingale, S.J. et al. (2006) Mol. Ther. 13: 1121-32), or by a combination of the above.

### Cells

In another aspect the invention provides a cell comprising the polynucleotide, the vector, or the CAR of the invention.

In some embodiments the cell is a T cell, lymphocyte or stem cell, such as a hematopoietic stem cell, cord blood stem cell (CB) or induced pluripotent stem cell (iPSC).

For example, the cell may be selected from the group consisting of CD4 cells, CD8 cells, Th0 cells, Tc0 cells, Th1 cells, Tc1 cells, Th2 cells, Tc2 cells, Th17 cells, Th22 cells, gamma/delta T cells, natural killer (NK) cells, natural killer T (NKT) cells, double-negative T cells, naive T cells, memory stem T cells, central memory T cells, effector memory T cells, effector T cells, cytokine-induced killer (CIK) cells, hematopoietic stem cells and induced pluripotent stem cells (iPSC).

In some embodiments, the cell is a T cell or NK cell, preferably a T cell. In some embodiments, the T cell is an autologous or allogeneic T cell.

The cell may have been isolated from a subject.

The cell of the invention may be provided for use in adoptive cell transfer. As used herein the term "adoptive cell transfer" refers to the administration of a cell population to a patient. Typically, the cells are T cells isolated from a subject and then genetically modified and cultured *in vitro* before being administered to the patient.

Adoptive cell transfer may be allogenic or autologous.

By "autologous cell transfer" it is to be understood that the starting population of cells (which are then transduced with a polynucleotide or vector according to the invention) is obtained from the same subject as that to which the transduced cell population is administered. Autologous transfer is advantageous as it avoids problems associated with immunological incompatibility and is available to subjects irrespective of the availability of a genetically matched donor.

By "allogeneic cell transfer" it is to be understood that the starting population of cells (which are then transduced with a polynucleotide or vector according to the invention) is obtained from a different subject as that to which the transduced cell population is administered. Preferably, the donor will be genetically matched to the subject to which the cells are administered to minimise the risk of immunological incompatibility. Alternatively, the donor may be mismatched and unrelated to the patient.

### Method of treatment

In another aspect, the invention provides the CAR, the polynucleotide, the vector, the cell or the pharmaceutical composition of the invention for use in therapy.

In some embodiments, the therapy is treatment of cancer.

In preferred embodiments, the cancer is a haematological malignancy.

In preferred embodiments, the cancer cells express CD84, for example the haematological malignancy may be a CD84-expressing haematological malignancy.

In some embodiments, the cancer is selected from the group consisting of chronic lymphocytic leukaemia (CLL), B-cell lymphoma, diffuse large B-cell lymphoma (DLBCL), Burkitt's lymphoma, follicular lymphoma, mantle cell lymphoma, B-cell acute lymphoblastic leukaemia (B-ALL), acute myeloid leukaemia (AML), myelodysplastic syndrome, T-cell acute lymphoblastic leukaemia/lymphoma (T-ALL), chronic myeloproliferative syndrome, chronic myeloid leukaemia (CML), chronic myelomonocytic leukaemia, dendritic-cell neoplasm and histiocytic sarcoma.

In some embodiments, the cancer is selected from the group consisting of chronic lymphocytic leukaemia (CLL), diffuse large B-cell lymphoma (DLBCL), Burkitt's lymphoma, follicular lymphoma, mantle cell lymphoma, B-cell acute lymphoblastic leukaemia (B-ALL), T-cell acute lymphoblastic leukaemia/lymphoma (T-ALL), acute myeloid leukemia (AML) and histiocytic sarcoma.

In preferred embodiments, the cancer is selected from the group consisting of CLL, B-cell lymphoma, B-ALL, T-ALL and AML.

In preferred embodiments, the cancer is selected from the group consisting of B-cell lymphoma, B-ALL, T-ALL and AML.

In another aspect, the invention provides the CAR, the polynucleotide, the vector, the cell or the pharmaceutical composition of the invention for use in treating AML.

In another aspect, the invention provides the CAR, the polynucleotide, the vector, the cell or the pharmaceutical composition of the invention for use in treating T-ALL.

In another aspect, the invention provides the CAR, the polynucleotide, the vector, the cell or the pharmaceutical composition of the invention for use in treating B-cell lymphoma.

In another aspect, the invention provides the CAR, the polynucleotide, the vector, the cell or the pharmaceutical composition of the invention for use in treating Burkitt's lymphoma.

In some embodiments, the cancer is not a T-cell lymphoma. In some embodiments, the cancer is not a mature T-cell lymphoma. In some embodiments, the cancer is not CLL. In some embodiments, the cancer is not a solid tumour.

The treatment of mammals, particularly humans, is preferred. Both human and veterinary treatments are within the scope of the invention.

### Pharmaceutical compositions and injected solutions

Although the agents for use in the invention can be administered alone, they will generally be administered in admixture with a pharmaceutical carrier, excipient or diluent, particularly for human therapy.

The medicaments, for example cells or vector particles, of the invention may be formulated into pharmaceutical compositions. These compositions may comprise, in addition to the medicament, a pharmaceutically acceptable carrier, diluent, excipient, buffer, stabiliser or other materials well known in the art. Such materials should be non-toxic and should not interfere with the efficacy of the active ingredient. The precise nature of the carrier or other material may be determined by the skilled person according to the route of administration, e.g. intravenous or intra-arterial.

The pharmaceutical composition is typically in liquid form. Liquid pharmaceutical compositions generally include a liquid carrier such as water, petroleum, animal or vegetable oils, mineral oil or synthetic oil. Physiological saline solution, magnesium chloride, dextrose or other saccharide solution, or glycols such as ethylene glycol, propylene glycol or polyethylene glycol may be included. In some cases, a surfactant, such as pluronic acid (PF68) 0.001% may be used. In some cases, serum albumin may be used in the composition.

For injection, the active ingredient may be in the form of an aqueous solution, which is pyrogen-free, and has suitable pH, isotonicity and stability. The skilled person is well able to prepare suitable solutions using, for example, isotonic vehicles such as Sodium Chloride Injection, Ringer's Injection or Lactated Ringer's Injection. Preservatives, stabilisers, buffers, antioxidants and/or other additives may be included as required.

For delayed release, the medicament may be included in a pharmaceutical composition which is formulated for slow release, such as in microcapsules formed from biocompatible polymers or in liposomal carrier systems according to methods known in the art.

Handling of the cell therapy products is preferably performed in compliance with FACT-JACIE International Standards for cellular therapy.

### Administration

In some embodiments, the CAR, the polynucleotide, the vector, the cell or the pharmaceutical composition of the invention is administered to a subject systemically.

In some embodiments, the CAR, the polynucleotide, the vector, the cell or the pharmaceutical composition of the invention is administered to a subject locally.

The term "systemic delivery" or "systemic administration" as used herein means that the agent of the invention is administered into the circulatory system, for example to achieve broad distribution of the agent. In contrast, topical or local administration restricts the delivery of the agent to a localised area.

In some embodiments, the CAR, the polynucleotide, the vector, the cell or the pharmaceutical composition of the invention is administered intravascularly, intravenously or intra-arterially.

In preferred embodiments, the CAR, the polynucleotide, the vector, the cell or the pharmaceutical composition of the invention is administered intravenously.

### Dosage

The skilled person can readily determine an appropriate dose of an agent of the invention to administer to a subject. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. There can of course be individual instances where higher or lower dosage ranges are merited, and such are within the scope of the invention.

### Subject

The term "subject" as used herein refers to either a human or non-human animal.

Examples of non-human animals include vertebrates, for example mammals, such as non-human primates (particularly higher primates), dogs, rodents (e.g. mice, rats or guinea pigs), pigs and cats. The non-human animal may be a companion animal.

Preferably, the subject is human.

### Variants, derivatives, analogues, homologues and fragments

In addition to the specific proteins and nucleotides mentioned herein, the invention also encompasses variants, derivatives, analogues, homologues and fragments thereof.

In the context of the invention, a "variant" of any given sequence is a sequence in which the specific sequence of residues (whether amino acid or nucleic acid residues) has been modified in such a manner that the polypeptide or polynucleotide in question retains at least one of its endogenous functions. A variant sequence can be obtained by addition, deletion, substitution, modification, replacement and/or variation of at least one residue present in the naturally occurring polypeptide or polynucleotide.

The term "derivative" as used herein in relation to proteins or polypeptides of the invention includes any substitution of, variation of, modification of, replacement of, deletion of and/or addition of one (or more) amino acid residues from or to the sequence, providing that the resultant protein or polypeptide retains at least one of its endogenous functions.

The term "analogue" as used herein in relation to polypeptides or polynucleotides includes any mimetic, that is, a chemical compound that possesses at least one of the endogenous functions of the polypeptides or polynucleotides, which it mimics.

Typically, amino acid substitutions may be made, for example from 1, 2 or 3, to 10 or 20 substitutions, provided that the modified sequence retains the required activity or ability. Amino acid substitutions may include the use of non-naturally occurring analogues.

Proteins used in the invention may also have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity and/or the amphipathic nature of the residues as long as the endogenous function is retained. For example, negatively charged amino acids include aspartic acid and glutamic acid; positively charged amino acids include lysine and arginine; and amino acids with uncharged polar head groups having similar hydrophilicity values include asparagine, glutamine, serine, threonine and tyrosine.

Conservative substitutions may be made, for example according to the table below. Amino acids in the same block in the second column and preferably in the same line in the third column may be substituted for each other:

| | | |
|---|---|---|
| ALIPHATIC | Non-polar | GAP |
| | | I L V |
| | Polar - uncharged | CSTM |
| | | N Q |
| | Polar - charged | D E |
| | | KRH |
| AROMATIC | | FWY |

The term "homologue" as used herein means an entity having a certain homology with the wild type amino acid sequence or the wild type nucleotide sequence. The term "homology" can be equated with "identity".

In the present context, a homologous sequence is taken to include an amino acid sequence which may be at least 50%, 55%, 65%, 75%, 85% or 90% identical, preferably at least 95%, 96% or 97% or 98% or 99% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, a homologous sequence is taken to include a nucleotide sequence which may be at least 50%, 55%, 65%, 75%, 85% or 90% identical, preferably at least 95%, 96% or 97% or 98% or 99% identical to the subject sequence. Although homology can also be considered in terms of similarity, in the context of the present invention it is preferred to express homology in terms of sequence identity.

Preferably, reference to a sequence which has a percent identity to any one of the SEQ ID NOs detailed herein refers to a sequence which has the stated percent identity over the entire length of the SEQ ID NO referred to.

Homology comparisons can be conducted by eye, or more usually, with the aid of readily available sequence comparison programs. These commercially available computer programs can calculate percent homology or identity between two or more sequences.

Percent homology may be calculated over contiguous sequences, i.e. one sequence is aligned with the other sequence and each amino acid or nucleotide in one sequence is directly compared with the corresponding amino acid or nucleotide in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion in the amino acid or nucleotide sequence may cause the following residues or codons to be put out of alignment, thus potentially resulting in a large reduction in percent homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids or nucleotides, a sequence alignment with as few gaps as possible, reflecting higher relatedness between the two compared sequences, will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example, when using the GCG Wisconsin Bestfit package the default gap penalty for amino acid sequences is -12 for a gap and -4 for each extension.

Calculation of maximum percent homology therefore firstly requires the production of an optimal alignment, taking into consideration gap penalties. A suitable computer program for carrying out such an alignment is the GCG Wisconsin Bestfit package (University of Wisconsin, USA; Devereux et al. (1984) Nucleic Acids Research 12: 387). Examples of other software that can perform sequence comparisons include, but are not limited to, the BLAST package (see Ausubel *et al.* (1999) ibid - Ch. 18), FASTA (Atschul et al. (1990) J. Mol. Biol. 403-410) and the GENEWORKS suite of comparison tools. Both BLAST and FASTA are available for offline and online searching (see Ausubel *et al.* (1999) ibid, pages 7-58 to 7-60).

However, for some applications, it is preferred to use the GCG Bestfit program. Another tool, BLAST 2 Sequences, is also available for comparing protein and nucleotide sequences (FEMS Microbiol. Lett. (1999) 174: 247-50; FEMS Microbiol. Lett. (1999) 177: 187-8).

Although the final percent homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix (the default matrix for the BLAST suite of programs). GCG Wisconsin programs generally use either the public default values or a custom symbol comparison table if supplied (see the user manual for further details). For some applications, it is preferred to use the public default values for the GCG package, or in the case of other software, the default matrix, such as BLOSUM62.

Once the software has produced an optimal alignment, it is possible to calculate percent homology, preferably percent sequence identity. The software typically does this as part of the sequence comparison and generates a numerical result.

"Fragments" are also variants and the term typically refers to a selected region of the polypeptide or polynucleotide that is of interest either functionally or, for example, in an assay. "Fragment" thus refers to an amino acid or nucleic acid sequence that is a portion of a full-length polypeptide or polynucleotide.

Such variants may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site may be made. The flanking regions will contain convenient restriction sites corresponding to sites in the naturally-occurring sequence so that the sequence may be cut with the appropriate enzyme(s) and the synthetic DNA ligated into the cut. The DNA is then expressed in accordance with the invention to make the encoded protein. These methods are only illustrative of the numerous standard techniques known in the art for manipulation of DNA sequences and other known techniques may also be used.

### Codon optimisation

The polynucleotides used in the invention may be codon-optimised. Codon optimisation has previously been described in WO 1999/41397 and WO 2001/79518. Different cells differ in their usage of particular codons. This codon bias corresponds to a bias in the relative abundance of particular tRNAs in the cell type. By altering the codons in the sequence so that they are tailored to match with the relative abundance of corresponding tRNAs, it is possible to increase expression. By the same token, it is possible to decrease expression by deliberately choosing codons for which the corresponding tRNAs are known to be rare in the particular cell type. Thus, an additional degree of translational control is available. Codon usage tables are known in the art for mammalian cells, as well as for a variety of other organisms.

Preferred features and embodiments of the invention will now be described by way of nonlimiting examples.

The practice of the present invention will employ, unless otherwise indicated, conventional techniques of chemistry, biochemistry, molecular biology, microbiology and immunology, which are within the capabilities of a person of ordinary skill in the art. Such techniques are explained in the literature. See, for example, Sambrook, J., Fritsch, E.F. and Maniatis, T. (1989) Molecular Cloning: A Laboratory Manual, 2nd Edition, Cold Spring Harbor Laboratory Press; Ausubel, F.M. et al. (1995 and periodic supplements) Current Protocols in Molecular Biology, Ch. 9, 13 and 16, John Wiley & Sons; Roe, B., Crabtree, J. and Kahn, A. (1996) DNA Isolation and Sequencing: Essential Techniques, John Wiley & Sons; Polak, J.M. and McGee, J.O'D. (1990) In Situ Hybridization: Principles and Practice, Oxford University Press; Gait, M.J. (1984) Oligonucleotide Synthesis: A Practical Approach, IRL Press; and Lilley, D.M. and Dahlberg, J.E. (1992) Methods in Enzymology: DNA Structures Part A: Synthesis and Physical Analysis of DNA, Academic Press.

### EXAMPLES

### EXAMPLE 1: Expression of CD84 in haematological malignancies

We have observed that CD84 is overexpressed in a range of malignant haematological diseases. Using GEPIA, an interactive web server for analysing the RNA sequencing expression data of 9,736 tumours and 8,587 normal samples from the TCGA and the GTEx projects (http://gepia.cancer-pku.cn/index.html; Tang et al. GEPIA: a web server for cancer and normal gene expression profiling and interactive analyses. Nucleic Acids Res. 2017; 10.1093/nar/gkx247) we have found that:
- CD84 expression is 4.2 times higher in 47 Lymphoid Neoplasm Diffuse Large B-cell Lymphoma (in Figure 1 presented as DLBC) than in 337 blood samples.
- CD84 expression is 10.1 times higher in 173 Acute Myeloid Leukaemia (in Figure 1 presented as LAML) than in 70 bone marrow samples.

We also interrogated the Cancer Cell Line Encyclopaedia (CCLE) dataset, which contains mRNA expression data for a panel of more than 1,100 cell lines derived from different types of solid and haematological tumours (https://portals.broadinstitute.org/ccle). Both RNAseq and Affymetrix analysis of mRNA expression showed that CD84 is expressed at high levels in cell lines derived from Burkitt's lymphoma, acute myeloid leukaemia (AML), B-cell lymphomas, chronic myeloid leukaemia (CML), B-cell acute lymphoblastic leukaemia (B-ALL) and T-cell acute lymphoblastic leukaemia (T-ALL), but not in T-cell lymphomas or solid tumours (Figure 2).

We then assessed the expression of CD84 on the surface of cell lines derived from different haematological malignancies. Table 1 shows the cell lines that were used, the haematological malignancy from which they originate and a qualitative assessment of CD84 expression based on the cytometry analysis shown in Figure 3.

**Table 1. List of cells lines derived from haematological malignancies used to assess surface CD84 expression and qualitative assessment of CD84 expression (the acute myeloblastic leukaemia cell line in the experiments herein described as MOLM-13 was previously indicated to be the acute myeloblastic leukaemia cell line Kasumi-1; references to Kasumi-1 have been updated accordingly).**

| **Cell Line** | **Haematological malignancy** | **CD84 expression** |
|---|---|---|
| Ramos | Burkitt's Lymphoma | High |
| Raji | Burkitt's Lymphoma | High |
| Daudi | Burkitt's Lymphoma | High |
| NALM6 | B-cell acute lymphoblastic leukaemia | High |
| MOLT-4 | T-cell acute lymphoblastic leukaemia | High |
| U937 | Histiocytic lymphoma | Low |
| MOLM-13 | Acute myeloblastic leukaemia | Moderate |
| K562 | Chronic myelogenous leukaemia (in blast crisis) | Low |
| THP-1 | Acute monocytic leukaemia | Low |

We analysed the expression of CD84 in 9 samples from patients with CLL by flow cytometry. Table 2 shows a qualitative assessment of CD84 expression based on the cytometry analysis shown in Figure 4. The expression of CD84 on the leukemic cells was compared to the expression of CD84 in lymphocytes (CD84^{moderate}) and monocytes (CD84^{high}). The histogram in light grey represents the staining with an isotype-matched control antibody and the histogram in dark grey the staining with the specific CD84 antibody.

**Table 2. Expression of CD84 in leukemic cells from 9 patients with chronic lymphocytic leukaemia (CLL) assessed by flow cytometry.**

| **Patient number** | **CD84 expression** |
|---|---|
| P01 | Moderate |
| P02 | High |
| P03 | High |
| P04 | High |
| P05 | High |
| P06 | High |
| P07 | High |
| P08 | High |
| P09 | High |

While certain genomic databases suggest that CD84 is overexpressed at mRNA level in AML, we sought to confirm if CD84 is expressed on the surface of malignant cells from patients (n=10) diagnosed with AML (Table 3).

**Table 3. Expression of CD84 in leukemic cells of 10 patients with acute myeloid leukaemia (AML) assessed by flow cytometry.**

| **Patient number** | **CD84 expression** |
|---|---|
| P01 | Moderate |
| P02 | Moderate |
| P03 | High |
| P04 | Moderate |
| P05 | Moderate |
| P06 | Moderate |
| P07 | Moderate |
| P08 | Moderate |
| P09 | Moderate |
| P10 | High |

Two representative examples of flow cytometry data are shown in Figure 5: samples from patient 04 and patient 10 show moderate and high expression of CD84, respectively.

### EXAMPLE 2: CD84 antibodies

### Mouse monoclonal antibodies

We studied and determined the sequences of two anti-CD84 mouse monoclonal antibodies (152-1D5 and 153-4D9; Engel et al. B-cell antigens section report, in Schlossman S (ed): Leucocyte Typing V. Oxford, UK, Oxford University Press, 1995, page 483; Palou et al. Genomic characterization of CD84 reveals the existence of five isoforms differing in their cytoplasmic domains. Tissue Antigens. 2000; 55(2):118-27).

In addition, we generated a number of new anti-CD84 mouse monoclonal antibodies as described in the Methods by immunizing BALB/c mice with the human CD84 protein. Table 3 summarizes the features of these antibodies, characterized as described in Methods. Binding to 300.19-CD84⁺, Raji and Ramos cells, lymphocytes and monocytes was carried out using hybridoma supernatants with equal amounts/concentration of anti-CD84 antibody.

**Table 4. Summary of features of anti-CD84 mouse monoclonal antibodies.**

| **Antibody** | **Class** | **Domain** | **300.19-CD84 binding** | **Raji binding** | **Ramos binding** | **Lymphocyte binding** | **Monocyte binding** |
|---|---|---|---|---|---|---|---|
| 152-1D5 | IgG1, κ | hD1 | 92.5% | 97.6% | 73.4% | 24.4% | 720% |
| 153-4D9 | IgG1, κ | hD1 | 88.0% | 65.8% | 34.3% | 15.9% | 47.7% |
| GYCD84.1 108 | IaG2a, κ | hD1 | 93.9% | 30.3% | 31.2% | 13.6% | 41.3% |
| GYCD84.1 226 | IgG1, κ | hD1 | 98.1% | 99.9% | 80.2% | 28.7% | 76.1% |
| GYCD84.2 32 | IgG1, κ | hD1 | 99.0% | 99.5% | 786% | 287% | 78.2% |
| GYCD84.3 89 | IgG1, κ | hD1 | 83.0% | 99.1% | 91.3% | 24.9% | 72.7% |
| GYCD84.1 97 | IgG2a, κ | hD1 | 97.7% | na | na | na | na |
| GYCD84.1 207 | IgG1, κ | hD1 | 94.2% | na | na | na | na |
| GYCD84.1 7 | IgG1, κ | hD2 | 92.6% | 91.4% | 59.0% | 19.0% | 64.4% |
| GYCD84.3 298 | IgG1, κ | hD2 | 93.5% | 45.9% | 28.3% | 16.6% | 53.9% |
| GYCD84.2 27 | IgG1, κ | hD2 | 96.3% | 97.5% | 66.3% | 25.4% | 77.5% |
| GYCD84.5 171 | IgG1, κ | hD2 | 93.2% | 73.9% | 35.1% | 26.8% | 64.8% |

The sequence corresponding to the variable region from the light chain (VL) and the sequence corresponding to the variable region from the heavy chain (VH) were determined from the different hybridomas using the Mouse Ig-Primer Set (Novagen). Sanger sequencing of this region was carried out by AbsoluteAntibody (United Kingdom). The antibody-sequence analysis was performed as follows: the complementarity-determining regions (CDR) and framework regions (FR) were identified using Abysis (sequences were defined by the Kabat-numbered scheme), IMGT and IgBLAST databases.

Table 5 shows the VH and VL sequence of these antibodies. The three CDRs in each sequence are highlighted in bold and underlined.

**Table 5. VH and VL sequences of anti-CD84 mouse monoclonal antibodies.**

| **Antibody** | **VH** | **VL** |
|---|---|---|
| 152-1D5 | QVQLQQPGAEVVQPGTSVKLSCKASGYNF TNYWINWVKLRPGQGLEWIGDIYPVSGTT NYNEKFKRKATLTVDTSSSTGNMQLSSLA SEDSALYYCASGTGRFAYWGQGTLVTVSA (SEQ ID NO: 61) | DIVLTQSPASLAVSLGQRATISCRASQSV STSSYSYMHWYQQKPGQPPKLLIKFASNL ESGVPARFSGSGSGTDFTLNIHPVEEEDT ATYYCQHSWEIPYTFGGGTKLEIK (SEQ ID NO: 62) |
| 153-4D9 | QVQLQQSGAELVRPGTSVKISCKASGYTF TNYWLGWVKQRPGHGLEWIGDIYPGGGYT | DIVLTQSPGSLAVSLGQRATISCRASESV DNYGISFMNWFQQKPGQPPKLLIYAASNQ |
| | NYIEKFKGKATLTADTSSSTAYMQLSSLT SEDSAVYFCARYEGGYYGNYDAMDYWGQG TSVTVSS (SEQ ID NO: 63) | GSGVPARFSGSGSGTDFSLNIHPMEEDDT AMYFCQQSKAVPRTFGGGTKLEIK (SEQ ID NO: 64) |
| GYCD84.1-7 | QVTLKESGPGILQPSQTLSLTCSFSGFSL STSGMGVGWIRQPSGKGLEWLAHIWWDDV KRYNPALKSRLTISKDTSSSQVFLKIASV DTADTATYYCARMRTSYYFDYWGQGTTLT VSS (SEQ ID NO: 74) | DIQMTQSPASLSASVGETVTITCRASENI FSSLAWYQQRQGKSPQLLVYNAKTLAEGV PSRFSGSGSGTQFSLKINSLQPEDFGSYY CQHHYATPFTFGSGTKLEIK (SEQ ID NO: 75) |
| GYCD84.1-97 | QVQLQQPGAELVKPGTSVKLSCKASGYNF TSYWINWVKLRPGQGLEWIGDIYLGSGST NYNEKFKSKATLTVDTSSSSAYMQLSSLV SEDSALYYCATSGGYLGYWGQGTTLTVSS (SEQ ID NO: 76) | DIVLTQSPASLAVSLGQRATISCRASQSV STSSYSYMHWYQQKPGQPPKLLIKFASNL ESGVPARFSGSGSGTDFTLNIHPVEEEDT ATYYCQHSWEIPYTFGGGTKLEIK (SEQ ID NO: 77) |
| GYCD84.1-108 | QVQLQQSGAELVRPGTSVKMSCKAAGYTF TNYWIGWVRQRPGHGLEWIGDIYPGGGYT NYNENFKGKATLTADTSSSTAYMQLSSLT SEDSAIYYCARSTTYYSSYWCFDVWGPGT TVTVSS (SEQ ID NO: 78) | DIVMTQSPSSLSVSAGEKVTMSCKSSQSL LNSGNQANYLAWYQQKPGQPPKLLINGAS TRESGVPDRFTGSGSGTDFTLTISSVQAE DLAVYYCQNDHSYPFTFGSGTKLEIK (SEQ ID NO: 79) |
| GYCD84.1-207 | EVKLLESGGGLVQPGGSLKLSCAASGFDF SRYWMSWVRQAPGKGLEWIGEINPDSSTI NYTPSLKDKFIISRDNAKNTLYLQMSKVR SEDTALYYCARPGPTVVATYWYFDVWGAG TTVTVSS (SEQ ID NO: 80) | DVLMTQTPLSLPVSLGDQASISCRSSQSI VHSNGNTYLEWYLQKPGQSPKLLIYKVSS RFSGVPDRFSGSASGTDFTLKISRVEAED LGVYYCFQGSHVPRTFGGGTKLEIK (SEQ ID NO: 81) |
| GYCD84.1-226 | QVQLQQPGAELVKPGTSVKLSCKASGYNF TRYWINWVKLRPGQGLEWIGDIYPGSGST NYNEKFKSKATLTVDTSSSTAYMQLSRLA SEDSALYYCARDTTIAYWGQGTLVTVSA (SEQ ID NO: 82) | DIVLTQSPTSLAVSLGQRATISCRASQSV TTSRYSYMHWYQQKPGQPPKLLIKFASNL ESGVPARFSGSGSGTDFTLNIHPVEEEDT ATYYCQHSWEIPYTFGGGTKLEIK (SEQ ID NO: 83) |
| GYCD84.2.32 | EVQLQQSGPELEKPGASVKISCKASGYSF TGYNMNWVKQSNGKSLEWIGNIDPYYGGT NYNQKFKGKATLTVDKSSSTAYMQLKSLT SEDSAVYYCARGLLSGSFPYWGQGTLVTV SA (SEQ ID NO: 161) | DIQMTQSPASLSASVGETVTITCRASENI YSYLAWYQQKQGKSPQLLVYNAKTLAEGV PSRFSGSGSGTQFSLKINSLHPEDFGSYF CQHHYGSPLTFGAGTKLEVK (SEQ ID NO: 162) |
| GYCD84.3.89 | EVKLLESGGGLVQPGGSLKLSCAASGFGF SRSWMSWVRQAPGKGLEWIGEINPDSSTI NYTPSLKDKFIISRDNAKNTLFLQMSKVR SEDTALYYCATFYDGYSIYWYFDVWGAGT TVTVSS (SEQ ID NO: 163) | DVLMTQTPLSLPVSLGDQASISCRSSQSI VHSNGDTYLEWYLQKPGQSPKLLIYKVSN RFSGVPDRFSGSGSGTDFTLKISRVEAED LGVYYCFQGSHVPRTFGGGTKLEIK (SEQ ID NO: 164) |
| GYCD84.3.298 | QVTLKESGPGILQPSQTLSLTCSFSGFSL STSGMGVGWIRQPSGKGLEWLAHIWWDDV KRYNPALRSRLTISKDTSSSQVFLNIASV DTADTATYYCTRIAVTYFFDFWGQGTTLT VSS (SEQ ID NO: 165) | DIQMTQSPASLSASVGETVTITCRASENI FSSFAWYQQKRGRSPQLLVYNARTLAEGV PSRFSGSGSGTQFSLKINSLQPEDFGTYF CQHHYASPFTFGSGTELGVK (SEQ ID NO: 166) |
| GYCD84.2.27 | QVTLKESGPGILQPSQTLSLTCSFSGFSL TTSGMGVGWIRQPSGKGLEWLAHIWWDDV KRYNPALKSRLTISKDTSSSQVFLKIASV DTADTASYYCARMSTSYYFDYWGQGTTLT VSS (SEQ ID NO: 167) | DIVMTQSHKFMSTSLGDRVSISCKASQSL FTSVAWYQQKPGQSPKLLIYSASYRYTGV PDRFTGSGSGTDFTFTISSVQAEDLAVYY CQQHYSSPFTFGSGTKLGIK (SEQ ID NO: 168) |
| GYCD84.5.171 | EVQLVESGGGLVQPKGSLKLACAASGFTF NIYAMNWVRQAPGKGLEWVARIRSKSNNY ARFYADSVKDRFTISRDDSQSMLYLQMNN LKTEDTAMYYCVRPLRSYFSMDYWGQGTS VTVSS (SEQ ID NO: 169) | SIVMTQSPKSMSMSVGERVTLSCKASENV DTYVSWYQQKPEQSPKLLIYGASNRYTGV PDRFTGSGSATDFTLTISSVKAEDLADYH CGQTYSYPWTFGGGTKLEIK (SEQ ID NO: 170) |

### Human scFvs

Phage display screening was used to identify novel human scFvs that bind to CD84. Three different scFvs were identified (R3-B3, R3-G7 and R3-H3); the sequence of the CDR and FR in the variable domains of the heavy (VH) and light (VL) chains is shown in Table 6.

**Table 6. VL and VH sequences of anti-CD84 human scFvs.**

| | **R3-B3** | **R3-G7** | **R3-H3** |
|---|---|---|---|
| **VH FR1** | QVQLVESGGGLVQPGGSLRL SCAAS (SEQ ID NO: 84) | QVQLVESGGGVVQPGRSLRL SCTAS (SEQ ID NO: 92) |  (SEQ ID NO: 100) |
| **VH CDR1** | GFTFSSYA (SEQ ID NO: 13) | GFTFSSYP (SEQ ID NO: 19) | GFTFSDNA (SEQ ID NO: 25) |
| **VH FR2** | MSWVRQAPGKGLEWVSA (SEQ ID NO: 85) | MHWVRQAPGKGLEWVAA (SEQ ID NO: 93) | MSWVRQAPGKGLEWVSA (SEQ ID NO: 101) |
| **VH CDR2** | ISGSGGST (SEQ ID NO: 14) | ISYHGRNK (SEQ ID NO: 20) | ISGTGRTT (SEQ ID NO: 26) |
| **VH FR3** | YYADSVKGRFTISRDNSKNT LYLQMNSLRAEDTAVYYC (SEQ ID NO: 86) | FYADSVKGRFTISRDDLRST LYLQMNNLKADDTAVYSC (SEQ ID NO: 94) | YYADSVKGRFTISRDNSKNT LYLQMNSLRAEDTAVYYC (SEQ ID NO: 102) |
| **VH CDR3** | AKWDCSDGRCYWAY (SEQ ID NO: 15) | ARDRDATPGGTGVGNHGMAV (SEQ ID NO: 21) | AKWDCSDGRCYWAY (SEQ ID NO: 27) |
| **VH FR4** | WGQGTLVTVSS (SEQ ID NO: 87) | WGQGTTVTVSS (SEQ ID NO: 95) | WGQGTLVTVSS (SEQ ID NO: 103) |
| **VH** | SEQ ID NO: 65 | SEQ ID NO: 68 | SEQ ID NO: 71 |
| **Linker** | GGGGSGGGGSGGGGSGGGGA S (SEQ ID NO: 67) | GGGGSGGGGSGGGGSGGGGA S (SEQ ID NO: 70) | GGGGSGGGGSGGGGSGGGGA S (SEQ ID NO: 73) |
| **VL FR1** | QPVLTQPPSVSVAPGQTARI SCGGD (SEQ ID NO: 88) | EIVLTQSPLSLPVTPGEPAS ISCKSS (SEQ ID NO: 96) | EIVLTQSPLSLPVTLGQPAS ISCRSS (SEQ ID NO: 104) |
| **VL CDR1** | NIESKD (SEQ ID NO: 16) | QSLLHSSGYNY (SEQ ID NO: 22) | QSLVYSDGDTY (SEQ ID NO: 28) |
| **VL FR2** | VHWYQRKSGQAPVLVVH (SEQ ID NO: 89) | LDWYLQKPGQSPQLLIH (SEQ ID NO: 97) | LNWFQQRPGQSPRRLIY (SEQ ID NO: 105) |
| **VL CDR2** | DDA (SEQ ID NO: 17) | MGS (SEQ ID NO: 23) | KVS (SEQ ID NO: 29) |
| **VL FR3** | DRPTGIPDRFSGSNSGNTAT LTISRVEAGDEADYYC (SEQ ID NO: 90) | NRASGVPARFSGSGSGTDFT LRISRVEAEDVGVYYC (SEQ ID NO: 98) | NRDSGVPDRFSGSGSGTDFT LKISRVEAEDVGVYYC (SEQ ID NO: 106) |
| **VL CDR3** | QVWDSSSDHW (SEQ ID NO: 18) | MQGLQTPPT (SEQ ID NO: 24) | MQGTHWPPNT (SEQ ID NO: 30) |
| **VL FR4** | FGGGTQLTVL (SEQ ID NO: 91) | FGGGTKLEIK (SEQ ID NO: 99) | FGQGTRLEIK (SEQ ID NO: 107) |
| **VL** | SEQ ID NO: 66 | SEQ ID NO: 69 | SEQ ID NO: 72 |

### EXAMPLE 3: CD84 CAR engineering

We engineered several anti-CD84 CAR constructs (CAR84). The complete CAR84 sequence was cloned into the third-generation lentiviral vector pCCL (Dull et al. A Third-Generation Lentivirus Vector with a Conditional Packaging System. J. Virol. 1998:72:8463-8471), including the signal peptide, the scFv specific for CD84, the CD8a hinge and transmembrane regions, the co-stimulatory domain 4-1BB and the signalling domain CD3-zeta, under the control of an EF1α promoter (Figure 6).

Different versions of the scFv domain were designed based on the antibodies described above. These scFv differ in the order of VH and VL sequences (with some versions having only the VH chain) and in the linker used between the VH and VL, which uses either three (S3) or four (S4) motifs (Gly-Gly-Gly-Gly-Ser). The name attributed to each CAR version reflects the design of the scFv. The sequences of the different scFv domains synthesized and cloned in the pCCL vector are listed in Table 7.

**Table 7. Sequence and structure of the scFv domains used in each CD84 CAR designed.**

| **CAR name** | **Antibody/ scFv** | **1st domain** | **Linker** | **2nd domain** |
|---|---|---|---|---|
| **152.1** (VH) | 152-1D5 | VH: QVQLQQPGAEVVQPGTSVKLS CKASGYNFTNYWINWVKLRPG QGLEWIGDIYPVSGTTNYNEK FKRKATLTVDTSSSTGNMQLS SLASEDSALYYCASGTGRFAY WGQGTLVTVSA (SEQ ID NO: 61) | | |
| **152.2** (VLS3VH) | 152-1D5 | VL: DIVLTQSPASLAVSLGQRATI SCRASQSVSTSSYSYMHWYQQ KPGQPPKLLIKFASNLESGVP ARFSGSGSGTDFTLNIHPVEE EDTATYYCQHSWEIPYTFGGG TKLEIKRA (SEQ ID NO: 108) | GGGGS GGGGS GGGGS (SEQ ID NO: 110) | VH: QVQLQQPGAEVVQPGTSVKLS CKASGYNFTNYWINWVKLRPG QGLEWIGDIYPVSGTTNYNEK FKRKATLTVDTSSSTGNMQLS SLASEDSALYYCASGTGRFAY WGQGTLVTVSA (SEQ ID NO: 61) |
| **152.3** (VLS4VH) | 152-1D5 | VL: DIVLTQSPASLAVSLGQRATI SCRASQSVSTSSYSYMHWYQQ KPGQPPKLLIKFASNLESGVP ARFSGSGSGTDFTLNIHPVEE EDTATYYCQHSWEIPYTFGGG TKLEIKRA (SEQ ID NO: 108) | GGGGS GGGGS GGGGS GGGGS (SEQ ID NO: 111) | VH: QVQLQQPGAEVVQPGTSVKLS CKASGYNFTNYWINWVKLRPG QGLEWIGDIYPVSGTTNYNEK FKRKATLTVDTSSSTGNMQLS SLASEDSALYYCASGTGRFAY WGQGTLVTVSA (SEQ ID NO: 61) |
| **152.4** (VHS3VL) | 152-1D5 | VH: QVQLQQPGAEVVQPGTSVKLS CKASGYNFTNYWINWVKLRPG QGLEWIGDIYPVSGTTNYNEK FKRKATLTVDTSSSTGNMQLS SLASEDSALYYCASGTGRFAY WGQGTLVTVSA (SEQ ID NO: 61) | GGGGS GGGGS GGGGS (SEQ ID NO: 110) | VL: DIVLTQSPASLAVSLGQRATI SCRASQSVSTSSYSYMHWYQQ KPGQPPKLLIKFASNLESGVP ARFSGSGSGTDFTLNIHPVEE EDTATYYCQHSWEIPYTFGGG TKLEIKRA (SEQ ID NO: 108) |
| **152.5** (VHS4VL) | 152-1D5 | VH: QVQLQQPGAEVVQPGTSVKLS CKASGYNFTNYWINWVKLRPG QGLEWIGDIYPVSGTTNYNEK FKRKATLTVDTSSSTGNMQLS | GGGGS GGGGS GGGGS GGGGS (SEQ | VL: DIVLTQSPASLAVSLGQRATI SCRASQSVSTSSYSYMHWYQQ KPGQPPKLLIKFASNLESGVP ARFSGSGSGTDFTLNIHPVEE |
| | | SLASEDSALYYCASGTGRFAY WGQGTLVTVSA (SEQ ID NO: 61) | ID NO: 111) | EDTATYYCQHSWEIPYTFGGG TKLEIKRA (SEQ ID NO: 108) |
| **153.1** (VH) | 153-4D9 | VH: QVQLQQSGAELVRPGTSVKIS CKASGYTFTNYWLGWVKQRPG HGLEWIGDIYPGGGYTNYIEK FKGKATLTADTSSSTAYMQLS SLTSEDSAVYFCARYEGGYYG NYDAMDYWGQGTSVTVSS (SEQ ID NO: 63) | | |
| **153.2** (VLS3VH) | 153-4D9 | VL: DIVLTQSPGSLAVSLGQRATI SCRASESVDNYGISFMNWFQQ KPGQPPKLLIYAASNQGSGVP ARFSGSGSGTDFSLNIHPMEE DDTAMYFCQQSKAVPRTFGGG TKLEIKRA (SEQ ID NO: 109) | GGGGS GGGGS GGGGS (SEQ ID NO: 110) | VH: QVQLQQSGAELVRPGTSVKIS CKASGYTFTNYWLGWVKQRPG HGLEWIGDIYPGGGYTNYIEK FKGKATLTADTSSSTAYMQLS SLTSEDSAVYFCARYEGGYYG NYDAMDYWGQGTSVTVSS (SEQ ID NO: 63) |
| 153.3 (VLS4VH) | | VL: DIVLTQSPGSLAVSLGQRATI SCRASESVDNYGISFMNWFQQ KPGQPPKLLIYAASNQGSGVP ARFSGSGSGTDFSLNIHPMEE DDTAMYFCQQSKAVPRTFGGG TKLEIKRA (SEQ ID NO: 109) | GGGGS GGGGS GGGGS GGGGS (SEQ ID NO: 111) | VH: QVQLQQSGAELVRPGTSVKIS CKASGYTFTNYWLGWVKQRPG HGLEWIGDIYPGGGYTNYIEK FKGKATLTADTSSSTAYMQLS SLTSEDSAVYFCARYEGGYYG NYDAMDYWGQGTSVTVSS (SEQ ID NO: 63) |
| **153.4** (VHS3VL) | 153-4D9 | VH: QVQLQQSGAELVRPGTSVKIS CKASGYTFTNYWLGWVKQRPG HGLEWIGDIYPGGGYTNYIEK FKGKATLTADTSSSTAYMQLS SLTSEDSAVYFCARYEGGYYG NYDAMDYWGQGTSVTVSS (SEQ ID NO: 63) | GGGGS GGGGS GGGGS (SEQ ID NO: 110) | VL: DIVLTQSPGSLAVSLGQRATI SCRASESVDNYGISFMNWFQQ KPGQPPKLLIYAASNQGSGVP ARFSGSGSGTDFSLNIHPMEE DDTAMYFCQQSKAVPRTFGGG TKLEIKRA (SEQ ID NO: 109) |
| **153.5** (VHS4VL) | 153-4D9 | VH: QVQLQQSGAELVRPGTSVKIS CKASGYTFTNYWLGWVKQRPG HGLEWIGDIYPGGGYTNYIEK FKGKATLTADTSSSTAYMQLS SLTSEDSAVYFCARYEGGYYG NYDAMDYWGQGTSVTVSS (SEQ ID NO: 63) | GGGGS GGGGS GGGGS GGGGS (SEQ ID NO: 111) | VL: DIVLTQSPGSLAVSLGQRATI SCRASESVDNYGISFMNWFQQ KPGQPPKLLIYAASNQGSGVP ARFSGSGSGTDFSLNIHPMEE DDTAMYFCQQSKAVPRTFGGG TKLEIKRA (SEQ ID NO: 109) |
| **B3.4** (VHS3VL) | R3-B3 | VH: QVQLVESGGGLVQPGGSLRLS CAASGFTFSSYAMSWVRQAPG KGLEWVSAISGSGGSTYYADS VKGRFTISRDNSKNTLYLQMN SLRAEDTAVYYCAKWDCSDGR CYWAYWGQGTLVTVSS (SEQ ID NO: 65) | GGGGS GGGGS GGGGS (SEQ ID NO: 110) | VL: QPVLTQPPSVSVAPGQTARIS CGGDNIESKDVHWYQRKSGQA PVLVVHDDADRPTGIPDRFSG SNSGNTATLTISRVEAGDEAD YYCQVWDSSSDHVVFGGGTQL TVL (SEQ ID NO: 66) |
| **B3.5** (VHS4VL) | R3-B3 | VH: QVQLVESGGGLVQPGGSLRLS CAASGFTFSSYAMSWVRQAPG KGLEWVSAISGSGGSTYYADS VKGRFTISRDNSKNTLYLQMN | GGGGS GGGGS GGGGS GGGGS (SEQ | VL: QPVLTQPPSVSVAPGQTARIS CGGDNIESKDVHWYQRKSGQA PVLVVHDDADRPTGIPDRFSG SNSGNTATLTISRVEAGDEAD |
| | | SLRAEDTAVYYCAKWDCSDGR CYWAYWGQGTLVTVSS (SEQ ID NO: 65) | ID NO: 111) | YYCQVWDSSSDHVVFGGGTQL TVL (SEQ ID NO: 66) |
| **G7.4** (VHS3VL) | R3-G7 | VH: QVQLVESGGGVVQPGRSLRLS CTASGFTFSSYPMHWVRQAPG KGLEWVAAISYHGRNKFYADS VKGRFTISRDDLRSTLYLQMN NLKADDTAVYSCARDRDATPG GTGVGNHGMAVWGQGTTVTVS S (SEQ ID NO: 68) | GGGGS GGGGS GGGGS (SEQ ID NO: 110) | VL: EIVLTQSPLSLPVTPGEPASI SCKSSQSLLHSSGYNYLDWYL QKPGQSPQLLIHMGSNRASGV PARFSGSGSGTDFTLRISRVE AEDVGVYYCMQGLQTPPTFGG GTKLEIK (SEQ ID NO: 69) |
| **G7. 5** (VHS4VL) | R3-G7 | VH: QVQLVESGGGVVQPGRSLRLS CTASGFTFSSYPMHWVRQAPG KGLEWVAAISYHGRNKFYADS VKGRFTISRDDLRSTLYLQMN NLKADDTAVYSCARDRDATPG GTGVGNHGMAVWGQGTTVTVS S (SEQ ID NO: 68) | GGGGS GGGGS GGGGS GGGGS (SEQ ID NO: 111) | VL: EIVLTQSPLSLPVTPGEPASI SCKSSQSLLHSSGYNYLDWYL QKPGQSPQLLIHMGSNRASGV PARFSGSGSGTDFTLRISRVE AEDVGVYYCMQGLQTPPTFGG GTKLEIK (SEQ ID NO: 69) |
| **H3.4** (VHS3VL) | R3-H3 | VH: EVQLVESGGGLVQPGGSLRLS CAASGFTFSDNAMSWVRQAPG KGLEWVSAISGTGRTTYYADS VKGRFTISRDNSKNTLYLQMN SLRAEDTAVYYCAKWDCSDGR CYWAYWGQGTLVTVSS (SEQ ID NO: 71) | GGGGS GGGGS GGGGS (SEQ ID NO: 110) | VL: EIVLTQSPLSLPVTLGQPASI SCRSSQSLVYSDGDTYLNWFQ QRPGQSPRRLIYKVSNRDSGV PDRFSGSGSGTDFTLKISRVE AEDVGVYYCMQGTHWPPNTFG QGTRLEIK (SEQ ID NO: 72) |
| **H3.5** (VHS4VL) | R3-H3 | VH: EVQLVESGGGLVQPGGSLRLS CAASGFTFSDNAMSWVRQAPG KGLEWVSAISGTGRTTYYADS VKGRFTISRDNSKNTLYLQMN SLRAEDTAVYYCAKWDCSDGR CYWAYWGQGTLVTVSS (SEQ ID NO: 71) | GGGGS GGGGS GGGGS GGGGS (SEQ ID NO: 111) | VL: EIVLTQSPLSLPVTLGQPASI SCRSSQSLVYSDGDTYLNWFQ QRPGQSPRRLIYKVSNRDSGV PDRFSGSGSGTDFTLKISRVE AEDVGVYYCMQGTHWPPNTFG QGTRLEIK (SEQ ID NO: 72) |

Complete CAR sequences are shown in Table 8.

**Table 8. Complete CAR sequences.**

| **CAR name** | **Antibody/ scFv** | **CAR sequence** |
|---|---|---|
| **152.3** (VLS4VH) | 152-1D5 | MALPVTGLLLSLGLLLHAARPTGDIVLTQSPASLAVSLGQRATISCRASQSV STSSYSYMHWYQQKPGQPPKLLIKFASNLESGVPARFSGSGSGTDFTLNIHP VEEEDTATYYCQHSWEIPYTFGGGTKLEIKRAGGGGSGGGGSGGGGSGGGGS QVQLQQPGAEVVQPGTSVKLSCKASGYNFTNYWINWVKLRPGQGLEWIGDIY PVSGTTNYNEKFKRKATLTVDTSSSTGNMQLSSLASEDSALYYCASGTGRFA YWGQGTLVTVSATTTPAPRPPTPAPTIASQPLSLRPEACRPAAGGAVHTRGL DFACDIYIWAPLAGTCGVLLLSLVITLYCKRGRKKLLYIFKQPFMRPVQTTQ EEDGCSCRFPEEEEGGCELRVKFSRSADAPAYQQGQNQLYNELNLGRREEYD VLDKRRGRDPEMGGKPQRRKNPQEGLYNELQKDKMAEAYSEIGMKGERRRGK GHDGLYQGLSTATKDTYDALHMQALPPR (SEQ ID NO: 172) |
| **153.4** (VHS3VL) | 153-4D9 | MALPVTGLLLSLGLLLHAARPTGQVQLQQSGAELVRPGTSVKISCKASGYTF TNYWLGWVKQRPGHGLEWIGDIYPGGGYTNYIEKFKGKATLTADTSSSTAYM QLSSLTSEDSAVYFCARYEGGYYGNYDAMDYWGQGTSVTVSSGGGGSGGGGS GGGGSDIVLTQSPGSLAVSLGQRATISCRASESVDNYGISFMNWFQQKPGQP |
| | | PKLLIYAASNQGSGVPARFSGSGSGTDFSLNIHPMEEDDTAMYFCQQSKAVP RTFGGGTKLEIKRATTTPAPRPPTPAPTIASQPLSLRPEACRPAAGGAVHTR GLDFACDIYIWAPLAGTCGVLLLSLVITLYCKRGRKKLLYIFKQPFMRPVQT TQEEDGCSCRFPEEEEGGCELRVKFSRSADAPAYQQGQNQLYNELNLGRREE YDVLDKRRGRDPEMGGKPQRRKNPQEGLYNELQKDKMAEAYSEIGMKGERRR GKGHDGLYQGLSTATKDTYDALHMQALPPR (SEQ ID NO: 173) |
| **153.5** (VHS4VL) | 153-4D9 | MALPVTGLLLSLGLLLHAARPTGQVQLQQSGAELVRPGTSVKISCKASGYTF TNYWLGWVKQRPGHGLEWIGDIYPGGGYTNYIEKFKGKATLTADTSSSTAYM QLSSLTSEDSAVYFCARYEGGYYGNYDAMDYWGQGTSVTVSSGGGGSGGGGS GGGGSGGGGSDIVLTQSPGSLAVSLGQRATISCRASESVDNYGISFMNWFQQ KPGQPPKLLIYAASNQGSGVPARFSGSGSGTDFSLNIHPMEEDDTAMYFCQQ SKAVPRTFGGGTKLEIKRATTTPAPRPPTPAPTIASQPLSLRPEACRPAAGG AVHTRGLDFACDIYIWAPLAGTCGVLLLSLVITLYCKRGRKKLLYIFKQPFM RPVQTTQEEDGCSCRFPEEEEGGCELRVKFSRSADAPAYQQGQNQLYNELNL GRREEYDVLDKRRGRDPEMGGKPQRRKNPQEGLYNELQKDKMAEAYSEIGMK GERRRGKGHDGLYQGLSTATKDTYDALHMQALPPR (SEQ ID NO: 174) |
| **G7.5** (VHS4VL) | R3-G7 | MALPVTGLLLSLGLLLHAARPTGQVQLVESGGGVVQPGRSLRLSCTASGFTF SSYPMHWVRQAPGKGLEWVAAISYHGRNKFYADSVKGRFTISRDDLRSTLYL QMNNLKADDTAVYSCARDRDATPGGTGVGNHGMAVWGQGTTVTVSSGGGGSG GGGSGGGGSGGGGSEIVLTQSPLSLPVTPGEPASISCKSSQSLLHSSGYNYL DWYLQKPGQSPQLLIHMGSNRASGVPARFSGSGSGTDFTLRISRVEAEDVGV YYCMQGLQTPPTFGGGTKLEIKTTTPAPRPPTPAPTIASQPLSLRPEACRPA AGGAVHTRGLDFACDIYIWAPLAGTCGVLLLSLVITLYCKRGRKKLLYIFKQ PFMRPVQTTQEEDGCSCRFPEEEEGGCELRVKFSRSADAPAYQQGQNQLYNE LNLGRREEYDVLDKRRGRDPEMGGKPQRRKNPQEGLYNELQKDKMAEAYSEI GMKGERRRGKGHDGLYQGLSTATKDTYDALHMQALPPR (SEQ ID NO: 175) |
| **H3.5** (VHS4VL) | R3-H3 | MALPVTGLLLSLGLLLHAARPTGEVQLVESGGGLVQPGGSLRLSCAASGFTF SDNAMSWVRQAPGKGLEWVSAISGTGRTTYYADSVKGRFTISRDNSKNTLYL QMNSLRAEDTAVYYCAKWDCSDGRCYWAYWGQGTLVTVSSGGGGSGGGGSGG GGSEIVLTQSPLSLPVTLGQPASISCRSSQSLVYSDGDTYLNWFQQRPGQSP RRLIYKVSNRDSGVPDRFSGSGSGTDFTLKISRVEAEDVGVYYCMQGTHWPP NTFGQGTRLEIKTTTPAPRPPTPAPTIASQPLSLRPEACRPAAGGAVHTRGL DFACDIYIWAPLAGTCGVLLLSLVITLYCKRGRKKLLYIFKQPFMRPVQTTQ EEDGCSCRFPEEEEGGCELRVKFSRSADAPAYQQGQNQLYNELNLGRREEYD VLDKRRGRDPEMGGKPQRRKNPQEGLYNELQKDKMAEAYSEIGMKGERRRGK GHDGLYQGLSTATKDTYDALHMQALPPR (SEQ ID NO: 176) |
| **1.7.3** (VLS4VH) | GYCD84. 1-7 | MALPVTGLLLSLGLLLHAARPTGDIQMTQSPASLSASVGETVTITCRASENI FSSLAWYQQRQGKSPQLLVYNAKTLAEGVPSRFSGSGSGTQFSLKINSLQPE DFGSYYCQHHYATPFTFGSGTKLEIKGGGGSGGGGSGGGGSGGGGSQVTLKE SGPGILQPSQTLSLTCSFSGFSLSTSGMGVGWIRQPSGKGLEWLAHIWWDDV KRYNPALKSRLTISKDTSSSQVFLKIASVDTADTATYYCARMRTSYYFDYWG QGTTLTVSSTTTPAPRPPTPAPTIASQPLSLRPEACRPAAGGAVHTRGLDFA CDIYIWAPLAGTCGVLLLSLVITLYCKRGRKKLLYIFKQPFMRPVQTTQEED GCSCRFPEEEEGGCELRVKFSRSADAPAYQQGQNQLYNELNLGRREEYDVLD KRRGRDPEMGGKPQRRKNPQEGLYNELQKDKMAEAYSEIGMKGERRRGKGHD GLYQGLSTATKDTYDALHMQALPPR (SEQ ID NO: 177) |
| **1.7.5** (VHS4VL) | GYCD84. 1-7 | MALPVTGLLLSLGLLLHAARPTGQVTLKESGPGILQPSQTLSLTCSFSGFSL STSGMGVGWIRQPSGKGLEWLAHIWWDDVKRYNPALKSRLTISKDTSSSQVF LKIASVDTADTATYYCARMRTSYYFDYWGQGTTLTVSSGGGGSGGGGSGGGG SGGGGSDIQMTQSPASLSASVGETVTITCRASENIFSSLAWYQQRQGKSPQL LVYNAKTLAEGVPSRFSGSGSGTQFSLKINSLQPEDFGSYYCQHHYATPFTF GSGTKLEIKTTTPAPRPPTPAPTIASQPLSLRPEACRPAAGGAVHTRGLDFA CDIYIWAPLAGTCGVLLLSLVITLYCKRGRKKLLYIFKQPFMRPVQTTQEED GCSCRFPEEEEGGCELRVKFSRSADAPAYQQGQNQLYNELNLGRREEYDVLD KRRGRDPEMGGKPQRRKNPQEGLYNELQKDKMAEAYSEIGMKGERRRGKGHD GLYQGLSTATKDTYDALHMQALPPR (SEQ ID NO: 178) |
| **1.226.5** (VHS4VL) | GYCD84. 1-226 | MALPVTGLLLSLGLLLHAARPTGQVQLQQPGAELVKPGTSVKLSCKASGYNF TRYWINWVKLRPGQGLEWIGDIYPGSGSTNYNEKFKSKATLTVDTSSSTAYM |
| | | QLSRLASEDSALYYCARDTTIAYWGQGTLVTVSAGGGGSGGGGSGGGGSGGG GSDIVLTQSPTSLAVSLGQRATISCRASQSVTTSRYSYMHWYQQKPGQPPKL LIKFASNLESGVPARFSGSGSGTDFTLNIHPVEEEDTATYYCQHSWEIPYTF GGGTKLEIKTTTPAPRPPTPAPTIASQPLSLRPEACRPAAGGAVHTRGLDFA CDIYIWAPLAGTCGVLLLSLVITLYCKRGRKKLLYIFKQPFMRPVQTTQEED GCSCRFPEEEEGGCELRVKFSRSADAPAYQQGQNQLYNELNLGRREEYDVLD KRRGRDPEMGGKPQRRKNPQEGLYNELQKDKMAEAYSEIGMKGERRRGKGHD GLYQGLSTATKDTYDALHMQALPPR (SEQ ID NO: 179) |
| **3.89.5** (VHS4VL) | GYCD84. 3-89 | MALPVTGLLLSLGLLLHAARPTGEVKLLESGGGLVQPGGSLKLSCAASGFGF SRSWMSWVRQAPGKGLEWIGEINPDSSTINYTPSLKDKFIISRDNAKNTLFL QMSKVRSEDTALYYCATFYDGYSIYWYFDVWGAGTTVTVSSGGGGSGGGGSG GGGSGGGGSDVLMTQTPLSLPVSLGDQASISCRSSQSIVHSNGDTYLEWYLQ KPGQSPKLLIYKVSNRFSGVPDRFSGSGSGTDFTLKISRVEAEDLGVYYCFQ GSHVPRTFGGGTKLEIKTTTPAPRPPTPAPTIASQPLSLRPEACRPAAGGAV HTRGLDFACDIYIWAPLAGTCGVLLLSLVITLYCKRGRKKLLYIFKQPFMRP VQTTQEEDGCSCRFPEEEEGGCELRVKFSRSADAPAYQQGQNQLYNELNLGR REEYDVLDKRRGRDPEMGGKPQRRKNPQEGLYNELQKDKMAEAYSEIGMKGE RRRGKGHDGLYQGLSTATKDTYDALHMQALPPR (SEQ ID NO: 180) |

### CD84 CART production

Lentiviruses (LV) containing the different versions of the pCCL-EF1α-CD84 vectors were produced in HEK293-T cells and the number of transducing units was determined by the limiting dilution method. The lentiviruses were then used to transduce T cells isolated from whole blood. These CAR-T cells were then expanded for 6 to 8 days.

The following tables (Tables 9-11) summarize the number of T cells obtained after three different transductions for the different CARTs, as well as the percentage of these T cells that expressed the CD84 CAR on their surface.

**Table 9. Number and percentage of CAR-positive T cells obtained following three independent transductions with lentivirus expressing CD84 CARs based on the 152-1D5 antibody.**

| | **Transduction #1** | | **Transduction #2** | | **Transduction #3** | |
|---|---|---|---|---|---|---|
| | **Cell number** | **% CAR** | **Cell number** | **% CAR** | **Cell number** | **% CAR** |
| **UT** | 19.2 x10⁶ | 0% | 8.7 x10⁶ | 0.0% | 7.8 x10⁶ | 0.0% |
| **152.1** | 4.6 x10⁶ | 2.5% | 5.5 x10⁶ | 2.1% | 6.2 x10⁶ | 2.3% |
| **152.2** | 0.9 x10⁶ | 66.0% | 1.0 x10⁶ | 87.8% | 1.0 x10⁶ | 86.3% |
| **152.3** | 2.1 x10⁶ | 66.3% | 0.5 x10⁶ | 83.9% | 0.7 x10⁶ | 89.1% |
| **152.4** | 1.6 x10⁶ | 38.2% | 2.0 x10⁶ | 60.7% | 1.5 x10⁶ | 69.5% |
| **152.5** | 1.1 x10⁶ | 47.7% | 2.6 x10⁶ | 75.4% | 2.4 x10⁶ | 73.8% |

**Table 10. Number and percentage of CAR-positive T cells obtained following three independent transductions with lentivirus expressing CD84 CARs based on the 153-4D9 antibody.**

| | **Transduction #1** | | **Transduction #2** | | **Transduction #3** | |
|---|---|---|---|---|---|---|
| | **Cell number** | **% CAR** | **Cell number** | **% CAR** | **Cell number** | **% CAR** |
| **UT** | 5.7 x10⁶ | 0% | 18.4 x10⁶ | 0% | 12.3 x10⁶ | 0% |
| **153.1** | 0.2 x10⁶ | 0% | 13.9 x10⁶ | 1.4% | 11.6 x10⁶ | 0.5% |
| **153.2** | 5.2 x10⁶ | 1.3% | 13.0 x10⁶ | 4.5% | 8.3 x10⁶ | 0.5% |
| **153.3** | 6.5 x10⁶ | 19.4% | 11.3 x10⁶ | 43.4% | 7.3 x10⁶ | 60.5% |
| **153.4** | 5.7 x10⁶ | 39.2% | 7.6 x10⁶ | 39.8% | 5.5 x10⁶ | 34.7% |
| **153.5** | 4.3 x10⁶ | 52.1% | 12.6 x10⁶ | 51.7% | 4.5 x10⁶ | 58.7% |

**Table 11. Number and percentage of CAR-positive T cells obtained following three independent transductions with lentivirus expressing CD84 CARs based on the R3-B3, R3-G7 and R3-H3 scFvs.**

| | **Transduction #1** | | **Transduction #2** | | **Transduction #3** | |
|---|---|---|---|---|---|---|
| | **Cell number** | **% CAR** | **Cell number** | **% CAR** | **Cell number** | **% CAR** |
| **UT** | 41.8 x10⁶ | 0% | 79.4 x10⁶ | 0% | 27.3 x10⁶ | 0% |
| **B3.4** | 55.4 x10⁶ | 20.4% | 49.1 x10⁶ | 6.5% | 22.3 x10⁶ | 7.8% |
| **B3.5** | 47.5 x10⁶ | 11.6% | 38.9 x10⁶ | 3.8% | 16.6 x10⁶ | 5.3% |
| **G7.4** | 49.9 x10⁶ | 56.3% | 43.4 x10⁶ | 30.7% | 32.6 x10⁶ | 39.1% |
| **G7.5** | 42 x10⁶ | 64.3% | 48.8 x10⁶ | 34% | 10.3 x10⁶ | 47.4% |
| **H3.4** | 18.7 x10⁶ | 51.9% | 59.9 x10⁶ | 54% | 19.4 x10⁶ | 33.7% |
| **H3.5** | 10.3 x10⁶ | 55.6% | 52.5 x10⁶ | 62.7% | 34.6 x10⁶ | 40.2% |

The following CAR LVs did not lead to an efficient expansion of CAR-positive T cells: 152.1, 153.1, 153.2, B3.4 and B3.5. The 152.1 and 153.1 CARs were not used in further experiments.

### EXAMPLE 4: CD84 CART in vitro cytokine production

To assess the ability of each CART cell to release cytokines, the supernatant of the effector-target cell co-cultures were collected. The CD84^{high} cell line Ramos was used as the target cell at a 2:1 effector:target cell ratio. The levels of IFN-γ (Figure 7), IL-2 (Figure 8), granzyme-B (Figure 9) and TNF-α (Figure10) in the supernatants was determined by enzyme linked immunosorbent assays (ELISA) after 24 hours of co-culture. Untransduced T cells (UT) were used as negative control (in co-culture with the target cells). Four different independent experiments were performed.

These results showed that CART cells with the R3-B3 scFv CAR binding domain lack cytokine release activity and therefore were not used in subsequent experiments. On the other hand, CART cells with the R3-H3 scFv CAR binding domain released a high amount of cytokines, with these two CART cells (H3.4 and H3.5) being the ones with the largest proinflammatory profile (based on TNF-α secretion), followed by CART cells with the 152-1D5 antibody CAR binding domain, which also show a high cytokine release profile. The 152-1D5 CART cells showed a similar profile independently of the scFv design (VH-VL order and linker length). CART cells based on the 153-4D9 and R3-G7 antibodies have a similar profile, with lower cytokine release in comparison to R3-H3 and 152-1D5 CART cells, except for 153.3 CART cells, which barely secrete cytokines.

### EXAMPLE 5: Anti-CD84 CART in vitro cytotoxicity

To evaluate the efficacy of the CART84 cells *in vitro,* we performed cytotoxicity assays of each CART cell against several GFP-expressing target cell lines with different levels of CD84 expression and from both lymphoid (Ramos, NALM6 and MOLT-4 cell lines) and myeloid origin (K562 and MOLM-13 cell lines). CART cytotoxicity was assessed after a 24-hour co-culture by determining the percentage of live GFP-positive cells by cytometry. Effector:target cell ratios of 4:1, 2:1. 1:1 and 0.5:1 were tested.

First, we tested all the CARs against the CD84^{high} Ramos cell line (Figure 11). The results for the effector:target ratio of 2:1 are shown. R3-H3 and 152-1D5 CART cells showed the highest cytotoxic activity against this cell line, while 153-4D9 and R3-G7 CART cells showed a lower cytotoxic activity. 153.3 CART cells showed the lowest cytotoxic activity of all CARs based on 153-4D9 constructs.

Next, we tested all the CARs against K562, a myeloid cell line with a low expression of CD84 (Figure 12). The results for the effector:target ratio of 2:1 are shown. CARs based on the 152-1D5 and 153-4D9 antibodies showed a statistically significant cytotoxic activity in comparison with UT cells.

Based on the cytotoxicity against Ramos and K562, we selected the following CART cells for additional characterization: 152.3, 153.4, 153.5, G7.5 and H3.5. We assessed the cytotoxicity of these CART cells at four effector:target ratios (4:1, 2:1, 1:1 and 0.5:1) against several cell lines after 24 and 48 hours of co-culture. Ramos is an aggressive B-cell lymphoma cell line (Figure 13), K562 is an acute myeloid leukaemia cell line with low CD84 expression (Figure 14) whereas MOLM-13 is an acute myeloid leukaemia cell line with moderate CD84 expression (Figure 15). NALM-6 is a B-cell acute lymphoblastic leukaemia cell line (Figure 16) and MOLT-4 a T-cell acute lymphoblastic leukaemia cell line (Figure 17).

As shown for the 2:1 ratio (Figure 11), the selected CARs displayed a statistically significant cytotoxic activity against Ramos cells for each of the effector:target ratios in comparison with UT cells (Figure 13). In the case of K562, the same pattern shown in Figure 12 was observed, with CARs based on the 152-1D5 and 153-4D9 antibodies showing a statistically significant cytotoxic activity in comparison with UT cells (Figure 14).

All the selected CARs showed cytotoxic activity against MOLM-13, with CARs based on 152.1D5 and 153.4D9 with a higher cytotoxic effect than those based on R3-G7 and R3-H3 antibodies (Figure 15).

CARs based on 152.1D5 and 153.4D9 antibodies showed a higher cytotoxic activity against NALM-6 cell line than CARs based on R3-G7 and R3-H3 scFv (Figure 16). For the MOLT-4 cell line, the CAR based on the 152-1D5 antibody showed the highest statistically significant cytotoxic activity, compared to UT cells, followed by CARs based on 153-4D9 and R3-G7 antibodies (Figure 17).

### EXAMPLE 6: CD84 expression in peripheral blood mononuclear cells (PBMC)

We assessed CD84 expression in the different cell populations of PBMCs by flow cytometry, and obtained results similar to what has been previously described. Monocytes show high CD84 expression, similar to that observed in the Ramos cell line. B cells display moderate CD84 expression. Both CD4 and CD8 T cells have two distinct subpopulations with regards to CD84 expression (positive and negative), with moderate CD84 expression levels on the positive population (Figure 18).

### EXAMPLE 7: CD84 CART in vitro cytotoxicity against PBMCs

We assessed the cytotoxic activity of CD84 CART against PBMCs. For each experiment, both effector cells (i.e. the CART84 cells) and the target cells (PBMCs) were obtained from the same donor. The mean of three independent experiments of three different donors are shown. CART cells did not display statistically significant cytotoxic activity against their own PBMCs. However, these CART cells did show statistically significant cytotoxic activity against Ramos cells in a parallel experiment (Figure 19).

### EXAMPLE 8: Methods for Examples 1 to 20

### Generation of mouse monoclonal anti-CD84 antibodies

BALB/c mice were immunized three to four times, at 3-week intervals, with 300.19 cells stably transfected with the CD84 full-length DNA (de la Fuente MA et al. CD84 leukocyte antigen is a new member of the Ig superfamily. Blood. 1997; 15;90(6):2398-405). The first intraperitoneal (i.p.) injection consisted of 20x10⁶ cells in 300 µl PBS, the second consisted of 20x10⁶ cells in 300 µl of PBS and the final injection consisted of 30x10⁶ cells in 300 µl in PBS. Mice were euthanized on the third day after the final boost and the splenocytes were harvested to perform the cell fusion.

NS1 myeloma cells (European Collection of Cell Cultures, Salisbury, UK) were fused with spleen cells by incubating them at a ratio of 4:1 (splenocytes:NS1) at 37°C, centrifuging for 10 minutes at RT and adding 1 mL of warm PEG solution to the cell pellet while constantly swirling. Cells were slowly resuspended in RPMI culture medium, centrifuged and incubated at 37°C in 5% CO₂ in a humidified incubator.

Screening was carried out 10 days after fusion by analysing 50µl of hybridoma supernatant by flow cytometry with 300.19-CD84 cells, using untransfected 300.19 cells as a negative control. Hybridomas that tested positive to 300.19-CD84 and negative to 300.19 cells were transferred to 24-well plates, grown until confluent and then transferred to T75 flasks. Single hybridoma clones producing antibodies of interest were then isolated by limited dilution. For each hybridoma, 10 single clones were retested by flow cytometry with 300.19-CD84 cells. The cloning protocol was repeated with one of the positive clones.

For each antibody, isotype (class and subclass) was determined by ELISA using anti-IgG coated plates to which the antibodies were added and then incubating the plates with an anti-mouse HRP antibody. To determine which of the two CD84 extracellular domains is recognized by the antibody, flow cytometry analysis was carried out with COS cells expressing a chimeric CD84 extracellular domain in which the domain 1 (D1) and 2 (D2) sequence is either human or murine.

### Identification of fully human anti-CD84 scFv

Human CD84 produced by mammalian cells (checked by SDS-PAGE; Figure 20) was used as an antigen to carry out panning of a human naïve phage display LiAb SFMax library (Proteogenix, France) with high diversity of 5.37x10¹⁰ scFv variants.

For the biopanning rounds, tubes were coated with antigen, blocked, washed and incubated with the phage library. After washing, elution of phage binders was done with Glycine HCl followed by neutralization.

To determine the concentration of the eluted phages, these were added to *E. coli* TG1 cells which were then poured onto plates and cultured upside down. The calculation of PFU (plaque-forming units) was based on the number of plaques (i.e. dead TG1) on a plate.

To amplify the eluted phages, these were added to *E. coli* TG1 cells which were then infected by helper phage and culture. Phage precipitation was carried out with PEG/NaCl followed by resuspension and the amplified phages used in the next biopanning round of ELISA analysis.

This analysis showed significant enrichment over the rounds (Table 12) and since round 3 output already showed excellent enrichment, no additional biopanning rounds to prevent decrease in phage diversity.

**Table 12. Concentration of eluted phages**

| **Rounds** | **Antigen concentration** | **Number of washings** | **Phage quantity (pfu)** | |
|---|---|---|---|---|
| | | | **Input** | **Output** |
| 1 | 50 µg/mL | 5 | 2x10¹² | 2.5x10⁵ |
| 2 | 50 µg/mL | 5 | 2x10¹² | 4.5x10⁵ |
| 3 | 50 µg/mL | 5 | 2x10¹² | 9x10⁷ |

Phages from round 3 were selected for monoclonal ELISA analysis. Single *E. coli* TG1 clones were picked and cultured with helper phage. Following centrifugation, supernatants containing phages were collected.

Plates were coated with either antigen or buffer, washed, blocked and washed again. Phages were then added to plates and incubated. Plates were washed and then an anti-phage horseradish peroxidase (HRP) antibody was added, followed by washing and incubation of TMB followed by HCl. Plates were read at 450 nm.

After sequencing of positive clones, 3 different unique sequences were identified. The 3 unique clones identified were then re-tested by ELISA to ensure clone specificity. All phages were tested at the same concentration. Plates were either covered with antigen (Ag) or with buffer (NC). The results clearly confirmed that the 3 clones specifically bind to the CD84 antigen (Table 13).

**Table 13. ELISA of positive clones**

| **Clone ID** | **Ag¹** | **NC²** |
|---|---|---|
| R3-B3 | 0.5060 | 0.0240 |
| R3-G7 | 2.397 | 0.0220 |
| R3-H3 | 0.696 | 0.0170 |

### Donors, cell lines

Healthy donor blood buffy-coats were obtained from the reference blood bank Banc de Sang i Teixits, Barcelona (Spain).

Ramos, Raji, NALM6, K562, MOLM-13, Kasumi-6, THP-1, and U937 were purchased from the American Type Culture Collection (ATCC). Ramos, Raji, NALM6, K562, NS1 and THP-1 cell lines were cultured in RPMI medium (ThermoFisher) supplemented with 10% foetal bovine serum (FBS, Merck) and penicillin-streptomycin (Labclinics), 300.19 cells were supplemented also with 1% L-glutamine (Gibco) and 0.1% 2-Beta-Mercaptoethanol (Sigma). The HEK293T and the COS cell line was cultured in DMEM medium (Gibco) supplemented with 10% FBS (Merck) and penicillin-streptomycin (Labclinics) and with 1% L-glutamine (Gibco). All cell lines were grown at 37°C and 5% CO₂.

### Lentivirus production

HEK293-T cells were transfected with our transfer vector pCCL-EF1α-CD84 together with the packaging plasmids pRSV-Rev (Addgene, 12253), pMDLg/Prre (Addgene, 12251), and envelope plasmid pCMV-VSV-G (Addgene, 12259) using linear polyethylenimine (PEI, molecular weight 25000, Polysciences Inc 23966-1). Lentiviral supernatant was collected 48 hours later and concentrated with LentiX-Concentrator (Clontech, Takara) following the manufacturer's protocol. Concentrated lentivirus was stored at -80°C until use.

### Lentivirus titration

The number of transducing units (TU/mL) was determined by the limiting dilution method.

HEK293T cells were seeded 24 h before transduction and 1:10 dilutions of the viral supernatant were prepared and added in DMEM medium (Gibco) supplemented with polybrene (Sigma-Aldrich) at 8 mg/mL. Cells were trypsinyzed 48 h later and labelled with AffiniPure F(ab')2 fragment goat anti-mouse immunoglobulin G (IgG) allophycocyanin (APC)-conjugated (Jacksonlmmuno Research Laboratories, 115-136-072). A dilution corresponding to 2%-20% of positive cells was used to calculate viral titer.

### T-cell transduction and CART expansion

T cells were isolated from whole blood during density gradient centrifugation with Ficoll-Paque^{™} by RosetteSep^{™} (RosetteSep Human T cell Enrichment cocktail from StemCell). T cells were cultured in X-Vivo 15 Serum Free Cell Medium (Lonza) supplemented with 5% AB human serum (Sigma H4522), penicillin-streptomycin (ThermoFisher, 100 mg/mL), and IL-2 at 50 IU/mL (Miltenyi). Cells were then activated using beads conjugated with CD3 and CD28 mAbs (Dynabeads Human T-Activator CD3/CD28 Gibco, 11131D). Twenty-four hours later they were transduced with the lentivirus in the presence of polybrene (Sigma-Aldrich) at 8 ug/mL. An expansion of 6 to 8 days was required before conducting the experiments.

### Flow cytometry

CAR84 was detected with a recombinant CD84-His protein (R&D, 1855-CD) and a secondary His Tag APC-conjugated antibody (R&D, IC050A) and with a biotinSP-conjugated AffiniPure F(ab')2-fragment goat-anti-mouse IgG (Jackson ImmunoResearch Laboratories, 115-065-072) or with a biotin SP-conjugated AffiniPure F(ab')2-fragment goat-anti-human IgG (Jackson ImmunoResearch Laboratories, 109-065-006) with a BV421-conjugated streptavidin (BD Horizon, 563259). The following mAbs against human proteins were used: CD3-APC, CD4-PE-Cy7, CD4-Alexa Fluor-488, CD19-PE, CD8-APC-H7, PD-1- PE-Cy7, TIM-3- BB515, CD69-PerCP-Cy^{™}5.5, LAG-3-BV-605, CD62L-FITC, CCR7-PerCP-Cy^{™}5.5, CD84-PE, CD4-PE-Cy^{™}7, CD45RA-APC (Becton Dickinson), CD84-APC and CD84-PE (BioLegend). Samples were run through the flow cytometer BD FACSCanto II (BD Biosciences), LSR II Fortessa 4L with HTS (BD), and Attune NxT 4L cytometer (ThermoFisher). For ELISA the following mAbs were used: an anti-mouse IgG-HRP (anti-mouse IgG-peroxidase antibody produced in goat, Sigma, cat: A3673-1ML) and an anti-mouse IgG coating (anti-mouse IgG antibody produced in goat (sigma, cat: M2650-1ML) Data were analysed using the FlowJo Software 10.7.1.

### In vitro cytotoxicity assays

The cytotoxicity of CART cells was assessed using different effector:target ratios and at different time-points. Target cells used in these assays were modified with a lentiviral vector to over-express GFP-firefly luciferase (GFP-ffLuc) as previously described (Shah et al. Antigen Presenting Cell-Mediated Expansion of Human Umbilical Cord Blood Yields Log-Scale Expansion of Natural Killer Cells with Anti-Myeloma Activity. PLoS One. 2013; 8(10)).

The percentage of remaining alive GFP⁺ tumour cells was analysed by flow cytometry and calculated using the following formula: % of live cell = 100 x (number of GFP⁺ cells with T cells at time x / number of GFP⁺ cells alone at time x).

### In vitro proliferation assays

CART84 cell proliferation in response to CD84 antigen was measured using a CFSE assay (Castella, M. et al. (2019) Mol. Ther. - Methods Clin. Dev. 12: 134-144). CART cells were stained with CellTrace CFSE (Invitrogen, ThermoFisher, 15598431) before being co-cultured with or without stimuli for 96 hours. Proliferation was analyzed by flow cytometry and proliferating index (PI) was calculated (PI = sum of number of cells in different generations / number of cells).

### In vitro cytokine production

IFN-γ, TNF-α, IL-6, IL-1β cytokines were quantified by Enzyme-Linked ImmunoSorbent Assay (DuoSet ELISA, R&D systems) following manufacturer's protocol.

### EXAMPLE 9: CD84 CAR engineering

We engineered several anti-CD84 CAR constructs based on the antibodies GYCD84.1-7, GYCD84.1-226 and GYCD84.3-89. The complete CAR84 sequence was cloned into the third-generation lentiviral vector pCCL (Dull et al. A Third-Generation Lentivirus Vector with a Conditional Packaging System. J. Virol. 1998:72:8463-8471), including the signal peptide, the scFv specific for CD84, the CD8α hinge and transmembrane regions, the co-stimulatory domain 4-1BB and the signalling domain CD3-ζ, under the control of an EF1α promoter (Figure 6).

Two different scFv were designed based on the antibody GYCD84.1-7, differing in the order of VH and VL sequences. All these CARs contain a linker with four (S4) motifs (Gly-Gly-Gly-Gly-Ser). The name attributed to each CAR version reflects the design of the scFv. The sequences of the different scFv domains synthesized and cloned in the pCCL vector are listed in Table 14.

**Table 14. Sequence and structure of the scFv domains used in each CD84 CAR designed.**

| **CAR name** | **Antibody/ scFv** | **1st domain** | **Linker** | **2nd domain** |
|---|---|---|---|---|
| **1.7.3** (VLS4VH) | GYCD84. 1-7 | VL: DIQMTQSPASLSA SVGETVTITCRAS ENIFSSLAWYQQR QGKSPQLLVYNAK TLAEGVPSRFSGS GSGTQFSLKINSL QPEDFGSYYCQHH YATPFTFGSGTKL EIK (SEQ ID NO: 75) | GGGGSGGGG SGGGGSGGG GS (SEQ ID NO: 111) | VH: QVTLKESGPGILQPSQTLSLT CSFSGFSLSTSGMGVGWIRQP SGKGLEWLAHIWWDDVKRYNP ALKSRLTISKDTSSSQVFLKI ASVDTADTATYYCARMRTSYY FDYWGQGTTLTVSS (SEQ ID NO: 74) |
| **1.7.5** (VHS4VL) | GYCD84. 1-7 | VH: QVTLKESGPGILQ PSQTLSLTCSFSG FSLSTSGMGVGWI RQPSGKGLEWLAH IWWDDVKRYNPAL KSRLTISKDTSSS QVFLKIASVDTAD TATYYCARMRTSY YFDYWGQGTTLTV SS (SEQ ID NO: 74) | GGGGSGGGG SGGGGSGGG GS (SEQ ID NO: 111) | VL: DIQMTQSPASLSASVGETVTI TCRASENIFSSLAWYQQRQGK SPQLLVYNAKTLAEGVPSRFS GSGSGTQFSLKINSLQPEDFG SYYCQHHYATPFTFGSGTKLE IK (SEQ ID NO: 75) |
| **1.226.5** (VHS4VL) | GYCD84. 1-226 | VH: QVQLQQPGAELVK PGTSVKLSCKASG YNFTRYWINWVKL RPGQGLEWIGDIY PGSGSTNYNEKFK SKATLTVDTSSST AYMQLSRLASEDS ALYYCARDTTIAY WGQGTLVTVSA (SEQ ID NO: 82) | GGGGSGGGG SGGGGSGGG GS (SEQ ID NO: 111) | VL: DIVLTQSPTSLAVSLGQRATI SCRASQSVTTSRYSYMHWYQQ KPGQPPKLLIKFASNLESGVP ARFSGSGSGTDFTLNIHPVEE EDTATYYCQHSWEIPYTFGGG TKLEIK (SEQ ID NO: 83) |
| **3.89.5** (VHS4VL) | GYCD84. 3-89 | VH: EVKLLESGGGLVQ PGGSLKLSCAASG FGFSRSWMSWVRQ APGKGLEWIGEIN PDSSTINYTPSLK DKFIISRDNAKNT LFLQMSKVRSEDT ALYYCATFYDGYS IYWYFDVWGAGTT VTVSS (SEQ ID NO: 163) | GGGGSGGGG SGGGGSGGG GS (SEQ ID NO: 111) | VL: DVLMTQTPLSLPVSLGDQASI SCRSSQSIVHSNGDTYLEWYL QKPGQSPKLLIYKVSNRFSGV PDRFSGSGSGTDFTLKISRVE AEDLGVYYCFQGSHVPRTFGG GTKLEIK (SEQ ID NO: 164) |

### EXAMPLE 10: Expression of CD84 in haematological malignancies

We analysed the expression of CD84 on the surface of leukemic cells from patients diagnosed with AML or with T-ALL by flow cytometry. Table 15 and 16 summarize the qualitative assessment of CD84 expression on AML and T-ALL cells, respectively. Two representative examples of flow cytometry analysis of primary leukemic cells of AML samples are shown in Figure 5. Analysis of T-ALL samples (patient 01 and patient 02) are shown in Figure 21.

**Table 15. Expression of CD84 on leukemic cells of 16 patients with AML assessed by flow cytometry.**

| **Patient number** | **CD84 expression** |
|---|---|
| P11 | Moderate |
| P12 | High |
| P13 | Moderate |
| P14 | High |
| P15 | Moderate |
| P16 | Moderate |
| P17 | High |
| P18 | Moderate |
| P19 | Low |
| P20 | Moderate |
| P21 | Moderate |
| P22 | Low |
| P23 | High |
| P24 | High |
| P25 | High |
| P26 | High |

**Table 16. Expression of CD84 on leukemic cells of two patients with T-ALL assessed by flow cytometry.**

| **Patient number** | **CD84 expression** |
|---|---|
| P01 | High |
| P02 | High |

### EXAMPLE 11: CD84 CART production

Lentiviruses (LV) containing the CART84 1.7.3, 1.226.5 and 3.89.5 pCCL-EF1α-CD84 vectors were produced in HEK293-T cells and the number of transducing units per mL was determined by the limiting dilution method. The lentiviruses were used to transduce T cells isolated from whole blood and these CAR-T cells were then expanded for 6 to 8 days.

Table 17 summarizes the number of T cells obtained after three different transductions, as well as the percentage of T cells that expressed the CD84 CAR on their surface.

**Table 17. Number and percentage of CAR-positive T cells obtained 6-7 days after transduction with lentivirus expressing CD84 CARs. Data from three independent transductions are shown.**

| | **Transduction #1** | | **Transduction #2** | | **Transduction #3** | |
|---|---|---|---|---|---|---|
| | **Cell number** | **% CAR** | **Cell number** | **% CAR** | **Cell number** | **% CAR** |
| **UT** | 20.7 x10⁶ | 0% | 20.9 x10⁶ | 0% | 7.1 x10⁶ | 0% |
| **1.7.3** | 12.2 x10⁶ | 61.9% | 13.6 x10⁶ | 75.7% | 8.2 x10⁶ | 98.5% |
| **1.226.5** | 14.6 x10⁶ | 74.9% | 7.2 x10⁶ | 85.7% | 8.2 x10⁶ | 94.8% |
| **3.89.5** | 13.1 x10⁶ | 58% | 10.3 x10⁶ | 64.1% | 23.6 x10⁶ | 80.8% |

Expansion of CART84 1.7.3, 1.226.5 and 3.89.5, as well as further analysis of expansion of 152.3, 153.5 and H3.5, for 6-7 days after transduction is shown in Figure 22, represented as fold increase over the number of T cells at day 0.

### EXAMPLE 12: CD84 CART in vitro cytotoxicity

Based on the cytotoxicity results obtained against Ramos, K562, MOLM-13, NALM6 and MOLT-4 cells, CART84 152.3, 153.5 and H3.5 were selected for additional characterization and compared with 1.7.3 and 1.226.5 and 3.89.5. We assessed their cytotoxicity against Ramos, MOLM-13, U937 and MOLT-4 cells after 24-hour or 48-hour co-culture by determining the percentage of live GFP-positive cells by cytometry, at effector:target cell ratios of 4:1, 2:1, 1:1 and 0.5:1. CD84 expression on these cells is shown in Figure 3.

All CART84 exerted a high cytotoxic effect against Ramos cells (CD84^{high}), which was statistically significant in comparison with UT (Figure 23). The cytotoxic effect increased over time for all CART cells; however, this effect was lower for H3.5 and 1.226.5, especially at lower ratios (1:1 and 0.5:1).

Against MOLM-13 (CD84^{moderate}) and U937 (CD84^{low}) AML cells, CART84 152.3, 153.5, 1.7.3, 1.226.5 and 3.89.5 displayed a high cytotoxic effect, which was statistically significant in comparison with UT (Figure 24 and 25, respectively). CART84 H3.5 exerted less cytotoxic activity than the other CART cells against leukemic cells from both cell lines. The cytotoxic effect increased over time for all CARTs. All CART cells, except H3.5, displayed high cytotoxicity towards U937 cells despite their low CD84 expression.

Finally, we assessed the cytotoxicity of CART84 152.3, 153.5, 1.7.5, 1.226.5 and 3.89.5 towards MOLT-4 cells (CD84^{high}), observing the same pattern seen with U937 cells, i.e., all CART cells, except H3.5, exerted a high cytotoxic effect that increased over time, and which was statistically significant in comparison with UT (Figure 26).

### EXAMPLE 13: CD84 CART in vitro cytotoxicity against primary leukemic blasts from AML and T-ALL

We assessed the cytotoxicity of CART84 cells 152.3, 153.5, G7.5 and H.3.5 against primary AML cells from patient samples obtained from the Haematology Department of Hospital Clinic de Barcelona (HCB). CD84 expression was assessed on the primary blasts, which were then stained with CFSE and co-cultured with the effector cells (CART/UT) at effector:target cell ratios of 4:1, 2:1, 1:1 and 0.5:1 during 24 h and 48 h. After this period, cells were stained with LIVE/Dead^{™} Fixable Aqua to differentiate live/dead cells. CART cells 152.3 and 153.5 displayed the highest cytotoxic activity towards AML blasts, which was statistically significant in comparison with the effect of UT. CARTs H3.5 and G7.5 exerted a low cytotoxic effect. The cytotoxic effect induced by all CARTs increased over time (Figure 27).

We also assessed the cytotoxicity of CART84 cells 152.3, 153.5, 1.7.3, 1.226.5 and 3.89.5 against primary T-ALL patient samples obtained from the Haematology Department of Hospital Clinic de Barcelona (HCB). The experiment was carried out as described above for AML samples. Cytotoxicity of CARTs 152.3, 153.5 and 1.7.3 was higher than that of CARTs 1.226.5 and 3.89.5 (Figure 28).

### EXAMPLE 14: CD84 CART in vitro cytokine production

To assess the ability of each CART cell to release cytokines, the supernatant of the effector-target cell co-cultures were collected. Ramos, MOLM-13 and MOLT-4 were used as the target cell at a 2:1 effector:target cell ratio. The levels of IFN-γ (Figure 29), granzyme-B (Figure 30) and TNF-α (Figure 31) in the supernatants was determined by enzyme linked immunosorbent assays (ELISA) after 24 hours of co-culture. Untransduced T cells (UT) were used as negative control (in co-culture with the target cells). Four independent experiments were performed. IFN-γ and granzyme B are cytotoxic cytokines, whereas TNF-α is a proinflammatory one. CART84 H3.5 displayed the highest cytokine production of all CART84 tested when co-cultured with Ramos. The cytokine profile of CART84 1.7.3 was similar to that of H3.5 after being co-cultured with MOLM-13 and MOLT-4 cells. All other CART cells tested produced similar levels of cytokines. These results suggest that both H3.5 and 1.7.3 CART cells may have tonic signaling, i.e. constitutive or chronic activation in the absence of the ligand.

### EXAMPLE 15: CD84 CART in vitro proliferation

To assess the ability of each CART84 cell to specifically proliferate upon antigen CD84 binding, CFSE proliferation assays were performed. Briefly, on day 0 CART cells were stained with CellTrace^{™} CFSE. Four conditions were tested: Cells (UT or CART cells) at day 0; cells alone cultured with media at day 4; cells stimulated with an unspecific stimulus of IL-2 (50 UI/mL) at day 4; and cells stimulated with the specific antigen at day 4, that is, with MOLM-13 cells, at an effector: target ratio of 0.5:1. We were able to prove that CART84 152.3, 153.5 and 1.226.5 proliferated more when exposed to CD84 antigen than when incubated with medium only or with IL-2 (Figure 32). On the other hand, CARTs H3.5, 1.7.3 and 3.89.5 proliferated similarly when stimulated with MOLM-13 or with IL-2 stimulus, thus suggesting that these CART84 may display a tonic signaling.

### EXAMPLE 16: CD84 CART in vitro cytotoxicity against haematopoietic progenitor stem cells

CD84 is expressed on malignant cells from several haematological malignancies. Among these is AML, where CD84 expression is shared between myeloid leukemic blasts and, to a lower extent, haematopoietic progenitor stem cells (HPSC). The same occurs CD33 and CD123, both of which are targets for CART products in development for treatment of AML. Figure 33 shows the expression of CD84, CD33 and CD123 on HPSC from an apheresis product.

To assess the potential myelotoxicity of CART84, their cytotoxicity towards HPSC was examined on CD34⁺ HPSC obtained from two different sources: cord blood units (CBU, from BST) and the apheresis product from a healthy donor that had been mobilized with G-CSF for an allogeneic stem cell transplant (also from BST). CD34⁺ cells from the apheresis product represent the ideal model to study myelotoxicity; however, it is more common to use CD34⁺ cells from CBU to study this type of on-target/off-tumour toxicity

Purified CD34⁺ cells from CBU were co-cultured for 24 hours with CART84 152.3, 153.5, 1.7.3, 1.229 and 3.89.5 at an effector: target cell ratio of 4:1 and 2:1. CART84 152.3, 153.5, 1.7.3 and 1.226.5 displayed low to moderate cytotoxic activity against CD34⁺ cells from CBU, with that of CART84 3.89.5 being the highest. Figure 34 summarizes the results from 5 independent experiments.

For the cytotoxicity assays against CD34⁺ HPSC from the apheresis product, 3 different donor buffy coats were used to generate the CART84. Surprisingly, the cytotoxicity of CART84 against these cells was relatively low. Although CART cytotoxicity increased after 48 hours, so did that of UT, which is unspecific (Figure 35).

### EXAMPLE 17: CD84 CART in vitro T-cell toxicity

Since CD84 is expressed on T cells, we studied the potential CART84 toxicity towards them. T cells were isolated directly from donor whole blood; half of them were activated and transduced to generate CART cells, and the other half was cryopreserved until the CART cells were available (i.e., for 6-7 days). T cells were stained with CFSE and then, co-cultured with the CART84. Figure 36 displays the results of 5 independent experiments. CART84 152.3, 153.5, 1.7.3 and 3.89.5 exerted moderate cytotoxic effect against their own T cells. On the other hand, the cytotoxic activity induced by 1.226.5 was low and not statistically significant in comparison with UT.

To further understand which specific T-cell fraction was being targeted by CART84, the effect on the following T-cell subsets was analyzed: naïve, central-memory, effector-memory, and effector CD4/CD8 T cells. The different T-cell subpopulations were studied by flow cytometry on: 1) target T cells before the assay and 2) on surviving T cells after co-culture with the different CART84 as indicated in Figure 37. We observed that the central-memory T cell fraction was most affected by CART84, especially by 152.3, 153.5 and 1.7.3, as expected since CD84 is mostly expressed on memory T cells. Moreover, all T-cell subsets were still present after co-culture with CART84.

### EXAMPLE 18: In vivo efficacy of anti-CD84 CARTs in an AML model

The efficacy of several CART84 cells was tested in three different experiments in NOD scid gamma (NSG) immunodeficient mice using MOLM-13 AML cells, modified to express GFP-firefly Luciferase (GFPffLuc). The CART84 with the best *in vitro* efficacy and safety profile were selected for testing *in vivo:* 152.3, 153.5, H3.5, 1.7.3 and 1.226.5.

In the first and second experiments, 1 x 10⁶ or 0.25 x 10⁶ MOLM-13 GFP-ffLuc cells were intravenously (i.v.) injected into NSG mice at day 0, respectively (Figure 38 and 39). Six days later, 4 x 10⁶ to 5 x 10⁶ UT, CART84 cells 152.3, 153.5, H3.5 or 1.7.3 were i.v. injected into the mice. Mice treated with CARTs 152.3, 153.5 and 1.7.3 had a statistically significant increase in survival compared with mice treated with UT.

In the experiment shown in Figure 40, 1 x 10⁶ MOLM-13 GFP-ffLuc cells were i.v. injected at day 0, and UT, CARTs 152.3, 153.5 and 1.226.5 two and eleven days later, at a dose of 5 x 10⁶ and 3 x 10⁶ cells, respectively. CARTs 152.3 and 153.5 controlled tumour progression, monitored by bioluminescence, which was statistically significant in comparison with mice treated with UT. Moreover, mice treated with CARTs 152.3 and 153.5 had a statistically significant increased survival compared with mice treated with UT.

The bone marrow and the spleen of mice euthanized at experiment endpoint were collected and analysed by flow cytometry in order to quantify the presence of tumour cells (MOLM-13-GFPffLuc), human CD3⁺ T cells or CART cells. In both experiments, CART84-treated mice had a significantly lower number of tumour cells in both bone marrow and spleen when compared to UT-treated mice (Figures 39C and 40C). Human T cells proliferated both bone marrow and spleen of mice treated with UT, most probably due to xeno-graft *versus* host disease (xeno-GvHD) (Figures 39D and 40D). CART84 cells were found both bone marrow and spleen, most prominently in the case of CART84 152.3 (Figures 39E and 40E).

In summary, 152.3 and 153.5 were able to consistently control AML disease progression and increased survival of treated animals.

### EXAMPLE 19: In vivo efficacy of CART84 in a T-ALL model

The efficacy of CART84 cells was tested in two different experiments in NOD scid gamma (NSG) mice using MOLT-4 T-ALL cells modified to express GFP-ffLuc. The following CART84 were tested: 152.3, 153.5, 1.7.3 and 1.226.5.

In the experiment shown in Figure 41, 0.75 x 10⁶ MOLT-4 GFP-ffLuc cells were i.v. injected into NSG mice at day 0; five days later, 3 x 10⁶ CART or UT were i.v. injected. CART84 152.3 controlled tumour progression, monitored by bioluminescence, in a way that was statistically significant in comparison with mice treated with UT. Moreover, mice treated with CARTs 152.3 and 153.5 had a statistically significant increased survival compared with mice treated with UT.

In the experiment shown in Figure 42, 4 x 10⁵ MOLT-4 GFP-ffLuc cells were i.v. injected at day 0 and 5 x 10⁶ CART or UT 2 days later. Treatment with CART84 152.3 and 153.5 elicited statistically significant efficacy in terms of both control of tumour progression monitored by bioluminescence and increased survival in comparison with UT.

The bone marrow and spleen of mice euthanized at the experiment endpoint were analysed by flow cytometry to quantify the presence of tumour cells (MOLT-4), human CD3⁺ T cells and CART cells. In this case, there were no statistically significant differences in the number of tumour cells found in the bone marrow and spleen of animals from different groups (Figure 42C). Human T cells proliferated in mice treated with UT, most probably due to xeno-GvHD, in both bone marrow and spleen (Figure 42D). CART cells were found both bone marrow and spleen, especially in the case of 152.3 cells (Figure 42E).

CART84 152.3 and 153.5 were the most efficacious in terms of controlling T-ALL disease and prolonging survival in both T-ALL experiments.

### EXAMPLE 20: CD84 CART in vitro toxicity towards human primary cells

To assess potential on-target/off-tumor toxicity, human primary cells were studied for CD84 expression and afterwards, the potential cytotoxic effect induced by CART84. The following human cells were studied: human coronary artery endothelial cells (HCAEC), human small airway epithelial cells (HsaEpC), human cardiac myocytes (HCM), human renal epithelial cells (HREpC) and human uterine fibroblasts (HUF). Using flow cytometry, we were able to prove that CD84 is not expressed on the surface of these cells (Figure 43).

In XCELLigence assays, we found that CART84 152.3 and 153.5 were not cytotoxic towards HsaEpC, HCM, HREpC nor HUF. However, some cytotoxic effect was observed towards HCAEC, despite their lack of CD84 expression, suggesting that this effect is not antigen-specific. In order to confirm this, we compared the cytotoxic activity of CART84 with that of a CART that targets CD123, since this antigen is expressed on the surface of endothelial cells. Cytotoxicity induced by the CD123-directed CART cells was significantly higher than that induced by CART84 152.3 or 153.5 (Figure 44).

### EXAMPLE 21: Methods for Examples 9 to 20

### CD123 CAR engineering

We engineered an anti-CD123 CAR construct based on the IL3scfv-lgG4(L235E)-CD28gg-zeta (26292) CAR from US 2014/0271582. The CAR sequence was cloned into the third-generation lentiviral vector pCCL (Dull et al. A Third-Generation Lentivirus Vector with a Conditional Packaging System. J. Virol. 1998: 72:8463-8471), including the signal peptide (GMCSFR alpha), the 26292 scFv (VH-linker-VL), the IgG4-Fc hinge and the CD28-transmembrane region, the co-stimulatory domain CD28 and the signaling domain CD3- ζ, under the control of an EF1α promoter.

### Flow cytometry

The positive CAR fraction of T cells was detected with Biotin-SP (long spacer) AffiniPure Goat Anti-Mouse IgG, F(ab')₂ fragment goat-anti-mouse IgG (Jackson ImmunoResearch) or with a biotin SP-conjugated AffiniPure F(ab')2-fragment goat-anti-human IgG (Jackson ImmunoResearch), washed and then incubated with a BV421/PE-conjugated streptavidin (eBioscience) and with the antibodies required to study each panel of proteins as described below.

The following mAbs against human proteins were used to study CAR T-cell phenotype: CD197-BV510 (CCR7), CD62L-FITC, CD4-Pecy7, CD8-APCH7, CD45RA-APC, (Becton Dickinson).

The following mAbs against human proteins were used to study the different population cells present in AML/T-ALL samples: CD11-APC, CD19-Fitc, HLA-DR-A450, CD45-PerCPCy5.5, CD34-PerCPCy5.5, CD3-APC, CD3-APCH7, CD33-Fitc, CD14-Fitc, CD14-APCH7, CD13-PE, CD13-BV421 (Becton Dickinson), CD84-APC, CD84-PE (BioLegend), CD45-A750, CD117-PeCy7 (Beckman Coulter), CD123-PECy7 (eBiosciences).

The following mAbs against human proteins were used to study the different population of mononuclear cells present in peripheral blood: CD3-BV421, CD14-APCH7, CD19-FITC, CD33-APC, CD34-PerCPCy5.5, CD38-APC, CD84-PE (BioLegend), CD123-PECy7 (eBiosciences) and HLA-DR-BV450 (Becton Dickinson)

The following mAbs against proteins were used in the *in vivo* experiments carried out in the MOLM-13 model: anti-human CD3-APCH7, anti-human CD33-APC, anti-human CD45-PerCP-Cy5.5, anti-mouse CD45-PECy7, Streptavidin-BV421 (Becton Dickinson). The following mAbs against proteins were used in the *in vivo* experiments carried out in the MOLT-4 model: anti-human CD3-APC, anti-human CD45-PerCP-Cy5.5, anti-mouse CD45 Pe-Cy7, Streptavidin-BV421 (Becton Dickinson).

All samples were run through a BD FACSCanto^{™} II, LSRFortessa^{™} 4L (DB Biosciences) or Attune^{™} NxT (Invitrogen) flow cytometer and analyzed with FlowJo v10.8.0 Software (BD Biosciences).

### In vitro cytotoxicity assays against primary leukemic blasts from AML and T-ALL patients

Primary blasts were stained with CFSE (ThermoFisher) and co-cultured with effector cells (CART/ UT) at effector:target cell ratios 4:1, 2:1, 1:1 and 0.5:1 for 24 h and 48 h. After this period, co-cultured cells were stained with LIVE/Dead^{™} Fixable Aqua (ThermoFisher) to differentiate live/dead cells. The percentage of remaining live blasts was determined by flow cytometry and calculated using the following formula: % of live cell = 100 x (number of negative LIVE/Dead-positive CFSE cells with CART cells at time x / number of negative LIVE/Dead-positive CFSE cells alone at time x).

### Isolation of CD34⁺ cells

Haematopoietic progenitor stem cells (CD34⁺) were obtained from two sources: cord blood units (CBU, from the blood bank Banc de Sang i Teixits de Barcelona, BST) and from an apheresis product from a healthy donor that had been mobilized with G-CSF for an allogeneic stem cell transplant (also obtained from BST).

CD34⁺ cells were isolated from CBU by first, Ficoll-Paque density-gradient centrifugation and second, purifying the CD34⁺ haematopoietic progenitor stem cells by magnetic separation with a human CD34-MicroBead kit (Miltenyi Biotec, positive selection). To isolate CD34⁺ cells from the apheresis product, cells were directly purified with the CD34-magnetic beads.

### In vitro cytotoxicity assays against CD34⁺ cells

To assess CD84 CART cytotoxicity towards CD34⁺ cells, CD34⁺ cells were co-cultured with effector cells (CART/ UT) at effector:target cell ratios 4:1, 2:1 for 24 h and 48 h. After this period, co-cultured cells were stained with APC-conjugated CD3 and PE-conjugated CD34 antibodies to differentiate effector from target cells and with LIVE/Dead Fixable Aqua to differentiate live/dead cells.

### In vitro cytotoxicity assays against purified T cells

T cells were isolated during Ficoll-Paque density-gradient centrifugation from donor buffy coats using an immune-cell isolation reagent from RosetteSep^{™}. Half of the T cells obtained in the process were activated and transduced to generate CART cells, and the other half was cryopreserved to be used as target cells until the CART cells were available (i.e. for 8 days). To perform the cytotoxicity assay, T cells were thawed and stained with CFSE before being co-cultured with the CART cells at different effector:target ratios. After 24 hours, both effector and target cells were fixed and stained with LIV/Dead Fixable Aqua cell stain kit.

The percentage of remaining live T cells was determined by flow cytometry and calculated using the following formula: % of live cell = 100 x (number of negative LIVE/Dead-positive CFSE cells with CART cells at time x / number of negative LIVE/Dead-positive CFSE cells alone at time x).

To study the remaining live T-cell subsets, these were also stained with human mAbs from the phenotype panel, as explained in the flow cytometry section.

### In vivo efficacy assays

Eight- to 12-week-old non obese diabetic-Cg-Prkdcscid II2rgtm1Wjl/SzJ (NSG) mice (Charles River) were bred and housed under pathogen-free conditions in the animal facility of the Faculty of Medicine and Health Sciences of University of Barcelona. Between 0.1 and 1 × 10⁶ GFPffLuc-expressing MOLM-13 or GFPffLuc-MOLT-4 cells were resuspended in saline and infused intravenously (IV) into each NSG mouse at day 0. Between 3 and 5 × 10⁶ UT or CD84 CARTs were intravenously (i.v.) infused 2 to 9 days after tumour-cells infusion. Tumour progression was monitored by bioluminescence using the Xenogen IVIS 50 Imaging System (PerkinElmer). To measure bioluminescence, 100uL of XenoLight D-Luciferin (Perkin Elmer Ref. 122799) were administered intraperitoneally into each mouse, and tumour burden was monitored weekly. Living Image software (PerkinElmer) was used to visualize and calculate total luminescence flux. Mice were euthanized when presenting signs of humane endpoint criteria. The bone marrow and the spleen were extracted and analysed by flow cytometry to quantify the presence of tumour cells, T cells and CART cells.

All procedures were performed in compliance with the institutional animal care committee of the Faculty of Medicine and Health Sciences of University of Barcelona.

### Primary human cells

The following primary human cells were acquired from PromoCell: human coronary artery endothelial cells (HCAEC), human small airway epithelial cells (HSAEpC), human uterine fibroblasts (HUF), human cardiac myocytes (HCM) and human renal epithelial cells (HREpC). Cells were cultured at 37°C and 5% CO₂ with the specific medium and supplements (also from PromoCells) as indicated in their data sheet.

### In vitro cytotoxicity assays with adherent cells

To assess CART cytotoxicity towards adherent cells, an xCELLigence instrument was used that allows real-time measurements of live cell proliferation by impedance. Target cells were seeded in RTCA E-Plates for xCELLigence (16 wells) and cell index was monitored during 24 h using the xCELLigence RTCA multiple plate monitoring system. At this point, 100 mL of either growth medium or T cells (untransduced T cells or CART cells at a 4:1 effector:target ratio) were added to each well. Data acquired from each well (cell index) was normalized to 1 and cell growth monitored during 72 h. Data were analysed using the xCELLigence RTCA Software Lite 2.0.0.1301.

### EXAMPLE 22: In vivo efficacy of CART84 in a B-cell lymphoma model

The efficacy of several CART84 cells was tested in an *in vivo* experiment in NOD scid gamma (NSG) mice using Ramos (Burkitt lymphoma) cells modified to express GFP-ffLuc. The following CART84 were tested: 152.3, 153.5 and H3.5.

In the experiment shown in Figure 45, 4 x 10⁵ Ramos GFP-ffLuc cells were i.v. injected into NSG mice at day 0. Two days later, 5 x 10⁶ CART or UT cells were i.v. injected. Mice treated with CARTs 152.3, 153.5 and H3.5 showed a trend of slower growth (Figure 45A). The bone marrow of mice euthanized at experiment endpoint were collected and analysed by flow cytometry in order to quantify the presence of tumour cells. CART84-treated mice had a statistically significant lower number of Ramos GFP-ffLuc cells in the bone marrow when compared to UT-treated mice (Figure 45B).

## Claims

1. A chimeric antigen receptor (CAR) that binds to CD84, wherein the CAR comprises an antigen-binding domain comprising:
a) a heavy chain variable region (VH) having complementarity determining regions (CDRs) with the sequences: CDR1 - NYWIN (SEQ ID NO: 1); CDR2 - DIYPVSGTTNYNEKFKR (SEQ ID NO: 2); and CDR3 - GTGRFAY (SEQ ID NO: 3); and
a light chain variable region (VL) having CDRs with the sequences: CDR1 - RASQSVSTSSYSYMH (SEQ ID NO: 4); CDR2 - FASNLES (SEQ ID NO: 5); and CDR3 - QHSWEIPYT (SEQ ID NO: 6);
b) a heavy chain variable region (VH) having complementarity determining regions (CDRs) with the sequences: CDR1 - NYWLG (SEQ ID NO: 7); CDR2 - DIYPGGGYTNYIEKFKG (SEQ ID NO: 8); and CDR3 - YEGGYYGNYDAMDY (SEQ ID NO: 9); and
a light chain variable region (VL) having CDRs with the sequences: CDR1 - RASESVDNYGISFMN (SEQ ID NO: 10); CDR2 - AASNQGS (SEQ ID NO: 11); and CDR3 - QQSKAVPRT (SEQ ID NO: 12);
c) a heavy chain variable region (VH) having complementarity determining regions (CDRs) with the sequences: CDR1 - GFTFSSYP (SEQ ID NO: 19); CDR2 - ISYHGRNK (SEQ ID NO: 20); and CDR3 - ARDRDATPGGTGVGNHGMAV (SEQ ID NO: 21); and
a light chain variable region (VL) having CDRs with the sequences: CDR1 - QSLLHSSGYNY (SEQ ID NO: 22); CDR2 - MGS (SEQ ID NO: 23); and CDR3 - MQGLQTPPT (SEQ ID NO: 24); or
d) a heavy chain variable region (VH) having complementarity determining regions (CDRs) with the sequences: CDR1 - GFTFSDNA (SEQ ID NO: 25); CDR2 - ISGTGRTT (SEQ ID NO: 26); and CDR3 - AKWDCSDGRCYWAY (SEQ ID NO: 27); and
a light chain variable region (VL) having CDRs with the sequences: CDR1 - QSLVYSDGDTY (SEQ ID NO: 28); CDR2 - KVS (SEQ ID NO: 29); and CDR3 - MQGTHWPPNT (SEQ ID NO: 30).

2. The CAR of claim 1, wherein the antigen-binding domain comprises:
a) a VH domain having the sequence of SEQ ID NO: 61; and a VL domain having the sequence of SEQ ID NO: 62 or 108;
b) a VH domain having the sequence of SEQ ID NO: 63; and a VL domain having the sequence of SEQ ID NO: 64 or 109;
c) a VH domain having the sequence of SEQ ID NO: 68; and a VL domain having the sequence of SEQ ID NO: 69; or
d) a VH domain having the sequence of SEQ ID NO: 71; and a VL domain having the sequence of SEQ ID NO: 72;
or variants thereof each having at least 90% sequence identity thereto.

3. The CAR of claim 1 or 2, wherein:
a) the CAR is an scFv CAR;
b) the CAR comprises a CD8a transmembrane domain;
c) the CAR comprises a 4-1BB or a CD28 co-stimulatory domain; and/or
d) the CAR comprises a CD3-zeta signalling domain.

4. A polynucleotide comprising one or more nucleotide sequences encoding the CAR of any preceding claim.

5. A vector comprising the polynucleotide of claim 4.

6. A cell comprising the polynucleotide of claim 4 or the vector of claim 5.

7. A cell comprising the CAR of any one of claims 1-3.

8. A cell comprising a first CAR and a second CAR, wherein the first CAR is the CAR of any one of claims 1-3, optionally wherein the second CAR is an anti-CD19 CAR, an anti-CD20 CAR, an anti-CD22 CAR, an anti-CD33 CAR, an anti-CD123 CAR; or an anti-CD7 CAR.

9. The cell of any one of claims 6 to 8, wherein the cell is a T cell or NK cell, optionally wherein the cell is an autologous or allogeneic cell.

10. A pharmaceutical composition comprising the CAR of any one of claims 1-3, the polynucleotide of claim 4, the vector of claim 5, or the cell of any one of claims 6 to 9.

11. The CAR of any one of claims 1-3, the polynucleotide of claim 4, the vector of claim 5, the cell of any one of claims 6 to 9, or the pharmaceutical composition of claim 10 for use in therapy.

12. The CAR, polynucleotide, vector, cell, or pharmaceutical composition for use according to claim 11, wherein the therapy is treatment of cancer

13. The CAR, polynucleotide, vector, cell, or pharmaceutical composition for use according to claim 12, wherein the cancer is a haematological malignancy, optionally a CD84-expressing haematological malignancy, and/or wherein the cancer is selected from the group consisting of chronic lymphocytic leukaemia (CLL), B-cell lymphoma, diffuse large B-cell lymphoma (DLBCL), Burkitt's lymphoma, follicular lymphoma, mantle cell lymphoma, B-cell acute lymphoblastic leukaemia (B-ALL), acute myeloid leukaemia (AML), myelodysplastic syndrome, T-cell acute lymphoblastic leukaemia/lymphoma (T-ALL), chronic myeloproliferative syndrome, chronic myeloid leukaemia (CML), chronic myelomonocytic leukaemia, dendritic-cell neoplasm and histiocytic sarcoma.

## Patentansprüche

1. Chimärer Antigenrezeptor (CAR), der an CD84 bindet, wobei der CAR eine antigenbindende Domäne umfasst, die Folgendes umfasst:
a) eine variable Region einer Schwerkette (VH), die komplementaritätsbestimmende Regionen (CDR) mit den folgenden Sequenzen aufweist: CDR1 - NYWIN (SEQ ID NO: 1); CDR2 - DIYPVSGTTNYNEKFKR (SEQ ID NO: 2); und CDR3 - GTGRFAY (SEQ ID NO: 3); und
eine variable Region einer Leichtkette (VL), die CDR mit den folgenden Sequenzen aufweist: CDR1 - RASQSVSTSSYSYMH (SEQ ID NO: 4); CDR2 - FASNLES (SEQ ID NO: 5); und CDR3 - QHSWEIPYT (SEQ ID NO: 6);
b) eine variable Region einer Schwerkette (VH), die komplementaritätsbestimmende Regionen (CDR) mit den folgenden Sequenzen aufweist: CDR1 - NYWLG (SEQ ID NO: 7); CDR2 - DIYPGGYTNYIEKFKG (SEQ ID NO: 8); und CDR3 - YEGGYYGNYDAMDY (SEQ ID NO: 9); und
eine variable Region einer Leichtkette (VL), die CDR mit den folgenden Sequenzen aufweist: CDR1 - RASESVDNYGISFMN (SEQ ID NO: 10); CDR2 - AASNQGS (SEQ ID NO: 11); und CDR3 - QQSKAVPRT (SEQ ID NO: 12);
c) eine variable Region einer Schwerkette (VH), die komplementaritätsbestimmende Regionen (CDR) mit den folgenden Sequenzen aufweist: CDR1 - GFTFSSYP (SEQ ID NO: 19); CDR2 - ISYHGRNK (SEQ ID NO: 20); und CDR3 - ARDRDATPGGTGVGNHGMAV (SEQ ID NO: 21); und
eine variable Region einer Leichtkette (VL), die CDR mit den folgenden Sequenzen aufweist: CDR1 - QSLLHSSGYNY (SEQ ID NO: 22); CDR2 - MGS (SEQ ID NO: 23); und CDR3 - MQGLQTPPT (SEQ ID NO: 24); oder
d) eine variable Region einer Schwerkette (VH), die komplementaritätsbestimmende Regionen (CDR) mit den folgenden Sequenzen aufweist: CDR1 - GFTFSDNA (SEQ ID NO: 25); CDR2 - ISGTGRTT (SEQ ID NO: 26); und CDR3 - AKWDCSDGRCYWAY (SEQ ID NO: 27); und
eine variable Region einer Leichtkette (VL), die CDR mit den folgenden Sequenzen aufweist: CDR1 - QSLVYSDGDTY (SEQ ID NO: 28); CDR2 - KVS (SEQ ID NO: 29); und CDR3 - MQGTHWPNT (SEQ ID NO: 30).

2. CAR nach Anspruch 1, wobei die antigenbindende Domäne Folgendes umfasst:
a) eine VH-Domäne, die die Sequenz unter SEQ ID NO: 61 aufweist; und eine VL-Domäne, die die Sequenz unter SEQ ID NO: 62 oder 108 aufweist;
b) eine VH-Domäne, die die Sequenz unter SEQ ID NO: 63 aufweist; und eine VL-Domäne, die die Sequenz unter SEQ ID NO: 64 oder 109 aufweist;
c) eine VH-Domäne, die die Sequenz unter SEQ ID NO: 68 aufweist; und eine VL-Domäne, die die Sequenz unter SEQ ID NO: 69 aufweist; oder
d) eine VH-Domäne, die die Sequenz unter SEQ ID NO: 71 aufweist; und eine VL-Domäne, die die Sequenz unter SEQ ID NO: 72 aufweist;
oder Varianten davon, die jeweils mindestens 90 % Sequenzidentität dazu aufweisen.

3. CAR nach Anspruch 1 oder 2, wobei:
a) es sich bei dem CAR um einen scFv-CAR handelt;
b) der CAR eine CD8a-Transmembrandomäne umfasst;
c) der CAR eine co-stimulatorische 4-1BB- oder eine CD28-Domäne umfasst; und/oder
d) der CAR eine CD3-Zeta-Signalgebungsdomäne umfasst.

4. Polynukleotid, umfassend eine oder mehrere Nukleotidsequenzen, die den CAR nach einem vorhergehenden Anspruch codieren.

5. Vektor, umfassend das Polynukleotid nach Anspruch 4.

6. Zelle, umfassend das Polynukleotid nach Anspruch 4 oder den Vektor nach Anspruch 5.

7. Zelle, umfassend den CAR nach einem der Ansprüche 1-3.

8. Zelle, umfassend einen ersten CAR und einen zweiten CAR, wobei es sich bei dem ersten CAR um den CAR nach einem der Ansprüche 1-3 handelt, gegebenenfalls wobei es sich bei dem zweiten CAR um einen Anti-CD19-CAR, einen Anti-CD20-CAR, einen Anti-CD22-CAR, einen Anti-CD33-CAR, einen Anti-CD123-CAR oder einen Anti-CD7-CAR handelt.

9. Zelle nach einem der Ansprüche 6 bis 8, wobei es sich bei der Zelle um eine T-Zelle oder NK-Zelle handelt, gegebenenfalls wobei es sich bei der Zelle um eine autologe oder allogene Zelle handelt.

10. Pharmazeutische Zusammensetzung, umfassend den CAR nach einem der Ansprüche 1-3, das Polynukleotid nach Anspruch 4, den Vektor nach Anspruch 5 oder die Zelle nach einem der Ansprüche 6 bis 9.

11. CAR nach einem der Ansprüche 1-3, Polynukleotid nach Anspruch 4, Vektor nach Anspruch 5, Zelle nach einem der Ansprüche 6 bis 9 oder pharmazeutische Zusammensetzung nach Anspruch 10 zur Verwendung in der Therapie.

12. CAR, Polynukleotid, Vektor, Zelle oder pharmazeutische Zusammensetzung zur Verwendung nach Anspruch 11, wobei es sich bei der Therapie um Behandlung von Krebs handelt.

13. CAR, Polynukleotid, Vektor, Zelle oder pharmazeutische Zusammensetzung zur Verwendung nach Anspruch 12, wobei es sich bei dem Krebs um ein hämatologisches Malignom, gegebenenfalls ein CD84-exprimierendes hämatologisches Malignom handelt und/oder wobei der Krebs aus der Gruppe bestehend aus chronischer lymphatischer Leukämie (CLL), B-Zell-Lymphom, diffusem großzelligem B-Zell-Lymphom (DLBCL), Burkitt-Lymphom, follikulärem Lymphom, Mantelzelllymphom, akuter lymphatischer B-Zell-Leukämie (B-ALL), akuter myeloischer Leukämie (AML), myelodysplastischem Syndrom, akuter/m lymphoblastischer/m T-Zell-Leukämie/Lymphom (T-ALL), chronischem myeloproliferativem Syndrom, chronischer myeloischer Leukämie (CML), chronischer myelomonozytärer Leukämie, dendritischem Zell-Neoplasma und histiozytischem Sarkom ausgewählt ist.

## Revendications

1. Récepteur d'antigène chimérique (CAR) qui se lie à CD84, le CAR comprenant un domaine de liaison d'antigène comprenant :
a) une région variable de chaîne lourde (VH) comportant des régions déterminant la complémentarité (CDR) ayant les séquences : CDR1 - NYWIN (SEQ ID NO : 1) ; CDR2 - DIYPVSGTTNYNEKFKR (SEQ ID NO : 2) ; et CDR3 - GTGRFAY (SEQ ID NO : 3) ; et
une région variable de chaîne légère (VL) comportant des CDR ayant les séquences : CDR1 - RASQSVSTSSYSYMH (SEQ ID NO : 4) ; CDR2 - FASNLES (SEQ ID NO : 5) ; et CDR3 - QHSWEIPYT (SEQ ID NO : 6) ;
b) une région variable de chaîne lourde (VH) comportant des régions déterminant la complémentarité (CDR) ayant les séquences : CDR1 - NYWLG (SEQ ID NO : 7) ; CDR2 - DIYPGGGYTNYIEKFKG (SEQ ID NO : 8) ; et CDR3 - YEGGYYGNYDAMDY (SEQ ID NO : 9) ; et
une région variable de chaîne légère (VL) comportant des CDR ayant les séquences : CDR1 - RASESVDNYGISFMN (SEQ ID NO : 10) ; CDR2 - AASNQGS (SEQ ID NO : 11) ; et CDR3 - QQSKAVPRT (SEQ ID NO : 12) ;
c) une région variable de chaîne lourde (VH) comportant des régions déterminant la complémentarité (CDR) ayant les séquences : CDR1 - GFTFSSYP (SEQ ID NO : 19) ; CDR2 - ISYHGRNK (SEQ ID NO : 20) ; et CDR3 - ARDRDATPGGTGVGNHGMAV (SEQ ID NO : 21) ; et
une région variable de chaîne légère (VL) comportant des CDR ayant les séquences : CDR1 - QSLLHSSGYNY (SEQ ID NO : 22) ; CDR2 - MGS (SEQ ID NO : 23) ; et CDR3 - MQGLQTPPT (SEQ ID NO : 24) ; ou
d) une région variable de chaîne lourde (VH) comportant des régions déterminant la complémentarité (CDR) ayant les séquences : CDR1 - GFTFSDNA (SEQ ID NO : 25) ; CDR2 - ISGTGRTT (SEQ ID NO : 26) ; et CDR3 - AKWDCSDGRCYWAY (SEQ ID NO : 27) ; et
une région variable de chaîne légère (VL) comportant des CDR ayant les séquences : CDR1 - QSLVYSDGDTY (SEQ ID NO : 28) ; CDR2 - KVS (SEQ ID NO : 29) ; et CDR3 - MQGTHWPPNT (SEQ ID NO : 30).

2. CAR selon la revendication 1, le domaine de liaison d'antigène comprenant :
a) un domaine VH ayant la séquence de SEQ ID NO : 61 ; et un domaine VL ayant la séquence de SEQ ID NO : 62 ou 108 ;
b) un domaine VH ayant la séquence de SEQ ID NO : 63 ; et un domaine VL ayant la séquence de SEQ ID NO : 64 ou 109 ;
c) un domaine VH ayant la séquence de SEQ ID NO : 68 ; et un domaine VL ayant la séquence de SEQ ID NO : 69 ; ou
d) un domaine VH ayant la séquence de SEQ ID NO : 71 ; et un domaine VL ayant la séquence de SEQ ID NO : 72 ;
ou des variants de ceux-ci ayant chacun au moins 90 % d'identité de séquence à ceux-ci.

3. CAR selon la revendication 1 ou 2, **caractérisé en ce que** :
a) le CAR est un CAR scFv ;
b) le CAR comprend un domaine transmembranaire CD8a ;
c) le CAR comprend un domaine de costimulation 4-1BB ou CD28 ; et/ou
d) le CAR comprend un domaine de signalisation CD3-zêta.

4. Polynucléotide comprenant une ou plusieurs séquences nucléotidiques codant pour le CAR selon l'une quelconque des revendications précédentes.

5. Vecteur comprenant le polynucléotide selon la revendication 4.

6. Cellule comprenant le polynucléotide selon la revendication 4 ou le vecteur selon la revendication 5.

7. Cellule comprenant le CAR selon l'une quelconque des revendications 1 à 3.

8. Cellule comprenant un premier CAR et un deuxième CAR, le premier CAR étant le CAR selon l'une quelconque des revendications 1 à 3, facultativement, le deuxième CAR étant un CAR anti-CD19, un CAR anti-CD20, un CAR anti-CD22, un CAR anti-CD33, un CAR anti-CD123 ; ou un CAR anti-CD7.

9. Cellule selon l'une quelconque des revendications 6 à 8, la cellule étant un lymphocyte T ou une cellule NK, facultativement, la cellule étant une cellule autologue ou allogénique.

10. Composition pharmaceutique comprenant le CAR selon l'une quelconque des revendications 1 à 3, le polynucléotide selon la revendication 4, le vecteur selon la revendication 5 ou la cellule selon l'une quelconque des revendications 6 à 9.

11. CAR selon l'une quelconque des revendications 1 à 3, polynucléotide selon la revendication 4, vecteur selon la revendication 5, cellule selon l'une quelconque des revendications 6 à 9 ou composition pharmaceutique selon la revendication 10 pour une utilisation en thérapie.

12. CAR, polynucléotide, vecteur, cellule ou composition pharmaceutique selon la revendication 11, la thérapie étant le traitement du cancer.

13. CAR, polynucléotide, vecteur, cellule ou composition pharmaceutique selon la revendication 12, le cancer étant une hémopathie maligne, facultativement une hémopathie maligne exprimant CD84, et/ou un cancer choisi dans le groupe constitué par : la leucémie lymphoïde chronique (LLC), le lymphome à cellules B, le lymphome diffus à grandes cellules B (LDGCB), le lymphome de Burkitt, le lymphome folliculaire, le lymphome à cellules du manteau, la leucémie lymphoblastique aiguë à cellules B (LLA-B), la leucémie myéloïde aiguë (LMA), le syndrome myélodysplasique, la leucémie/le lymphome lymphoblastique aigu à cellules T (LLA-T), le syndrome myéloprolifératif chronique, la leucémie myéloïde chronique (LMC), la leucémie myélomonocytaire chronique, une néoplasie à cellules dendritiques et le sarcome histiocytaire.
